# EUROPEAN PATENT APPLICATION

(11) **EP 4 249 489 A1**
(43) Date of publication of application: **27.09.2023**
(21) Application number: 21898638.8
(22) Date of filing: 25.11.2021
(51) Int. Cl.: C07D 471/04, A61K 31/4985, A61P 35/00

(54) **HETEROCYCLIC COMPOUND AS DIACYLGLYCEROL KINASE INHIBITOR AND USE THEREOF**

(30) Priority: 26.11.2020 KR 20200161547
(71) Applicant: Lg Chem, Ltd., Seoul 07336 (KR)
(72) Inventor: KIM, Byung Gyu, Seoul 07796 (KR); YOON, Su Young, Seoul 07796 (KR); KWAK, Young Shin, Seoul 07796 (KR); JANG, Chang Young, Seoul 07796 (KR)
(74) Representative: Hoffmann Eitle
(86) International application number: PCT/KR2021/017518
(87) International publication number: WO 2022/114812

(57) **Abstract**

The present invention relates to a heterocyclic compound, represented by chemical formula 1, exhibiting a diacylglycerol kinase inhibitor activity, a pharmaceutical composition comprising same as an active ingredient, and a use thereof.

## Description

### TECHNICAL FIELD

The present invention relates to a heterocyclic compound represented by Formula 1 showing inhibitory activity against diacylglycerol kinases, a pharmaceutical composition comprising the same as an active ingredient, and use thereof.

### BACKGROUND ART

Recently, anticancer immunotherapy, specifically T cell therapy, has been attracting attention as it shows great effects on some tumors such as malignant melanoma. However, as a major obstacle to be overcome by anticancer T cell therapy, tumor-induced T cell immune suppression (T cell tolerance, T cell anergy) exists. That is, even if a large number of anti-cancer T cells approach the tumor, there is a mechanism in which the tumor disables the T cells, and so the effect of the T cells can be greatly reduced. Therefore, understanding the mechanism of T cell inactivation caused by tumors and preparing countermeasures to prevent this inactivation may greatly improve the therapeutic efficiency of anticancer T cell therapy. As an immune anticancer target to overcome this, diacylglycerol kinases (DGKs) are attracting great interest. DGKs are overexpressed when T cells fall into anergy and play a role in inactivating T cell activity. Specifically, DGKs act as an intracellular checkpoint through a reaction that converts diacylglycerol (DAG)-which is an important factor in signal transduction-into phosphatidic acid (PA), and it has been known that when such DGKs are inhibited, the accumulated DAG reactivates T cells that are in anergy by enhancing the TCR signaling pathway of T cells. As such, when DGKs inhibition alone or in combination with cancer immunotherapy such as PD-(L)1 is used, synergy may be expected. In addition, DGKs are overexpressed in various cancer cells and have been known to cause cancer cell survival, migration and drug resistance. Therefore, if a substance that inhibits DGKs is developed, an excellent anticancer efficacy can be expected through the dual pharmacological effect of exhibiting apoptotic effect and cancer immunotherapy roles such as T cell reactivation simultaneously. Furthermore, since DGKs are known to be involved in NK cell anergy as well as T cell anergy, an additional advantage of eliminating cancer cells by NK cells may be obtained when developing an inhibitor.

### DISCLOSURE OF INVENTION

### TECHNICAL PROBLEM

An object of the present invention is to provide a novel heterocyclic compound represented by Formula 1 showing inhibitory activity against diacylglycerol kinases.

Another object of the present invention is to provide a pharmaceutical composition for the prevention or treatment of diseases associated with diacylglycerol kinases such as cancer comprising the heterocyclic compound as an active ingredient.

Still another object of the present invention is to provide a method for preventing or treating diseases associated with diacylglycerol kinases such as cancer in a subject by using the heterocyclic compound as an active ingredient.

### SOLUTION TO PROBLEM

In order to achieve the above object, the present invention provides a compound of the following Formula 1, or a pharmaceutically acceptable salt or stereoisomer thereof: 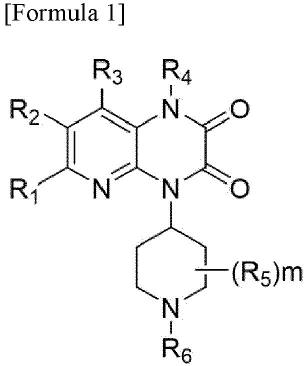 wherein
m represents an integer of 0, 1 or 2;
R₁ represents hydrogen, halo, cyano (-CN), alkyl, alkoxy, alkylcarbonyl or aryl;
R₂ represents hydrogen, halo, cyano, carboxy (-COOH), alkyl, alkylcarbonyl, alkoxycarbonyl, aminocarbonyl, alkylaminocarbonyl, dialkylaminocarbonyl, dialkylaminoalkylaminocarbonyl, cycloalkyl, aryl or heteroaryl;
R₃ represents hydrogen or alkyl;
R₄ represents alkyl;
R₅ represents alkyl, or may combine with each other to form a ring when m is 2;
R₆ represents 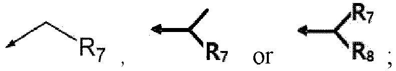 wherein R₇ and R₈ independently of one another represent carbocyclyl or heterocyclyl;
wherein the heteroaryl and heterocyclyl have one or more heteroatoms selected from nitrogen (N), oxygen (O) and sulfur (S); and
the aryl, heteroaryl, carbocyclyl and heterocyclyl may be optionally substituted with one or more substituents selected from the group consisting of halo, alkyl, alkoxy, haloalkyl, haloalkoxy, aryl and aryloxy.

The compound of Formula 1 according to the present invention may form a pharmaceutically acceptable salt. A pharmaceutically acceptable salt may include an acid-addition salt which is formed from an inorganic acid such as hydrochloric acid, sulfuric acid, nitric acid, phosphoric acid, hydrobromic acid and hydroiodic acid; an organic acid such as tartaric acid, formic acid, citric acid, acetic acid, trichloroacetic acid, trifluoroacetic acid, gluconic acid, benzoic acid, lactic acid, fumaric acid, maleic acid and salicylic acid; or sulfonic acid such as methanesulfonic acid, ethanesulfonic acid, benzenesulfonic acid and p-toluenesulfonic acid, which form non-toxic acid-addition salt including pharmaceutically acceptable anion. In addition, a pharmaceutically acceptable carboxylic acid salt includes the salt with alkali metal or alkali earth metal such as lithium, sodium, potassium, calcium and magnesium; salts with amino acid such as lysine, arginine and guanidine; an organic salt such as dicyclohexylamine, N-methyl-D-glucamine, tris(hydroxymethyl)methylamine, diethanolamine, choline and triethylamine. The compound of Formula 1 according to the present invention may be converted into their salts by conventional methods.

Meanwhile, since the compound of Formula 1 according to the present invention can have an asymmetric carbon center and asymmetric axis or plane, they can exist as E- or Z-isomer, R- or S-isomer, racemic mixtures or diastereoisomer mixtures and each diastereoisomer, all of which are within the scope of the present invention.

Herein, unless indicated otherwise, the term "the compound of Formula 1" is used to mean all the compounds of Formula 1, including the pharmaceutically acceptable salts and stereoisomers thereof.

Herein, the following concepts defined to the substituents are used to define the compound of Formula 1.

Unless indicated otherwise, the term "halo" used herein, either alone or in combination with additional terms (for example, haloalkyl or haloalkoxy), means a radical of fluoride (F), chlorine (Cl), bromine (Br) or iodine (I).

Unless indicated otherwise, the term "alkyl" used herein, either alone or in combination with additional terms (for example, haloalkyl), means a radical of a saturated or unsaturated aliphatic hydrocarbon group having-for example 1 to 7 carbon atoms of a linear or branched chain. For example, the alkyl may include methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1-ethylpropyl, 1,2-dimethylpropyl and the like, but is not limited thereto.

Unless indicated otherwise, the term "alkoxy" used herein means alkyloxy having-for example 1 to 7 carbon atoms.

Unless indicated otherwise, the term "cycloalkyl" used herein means a saturated ring aliphatic hydrocarbon having-for example 3 to 7 carbon atoms. For example, the cycloalkyl may include cyclopropyl, cyclobutyl, cyclopentyl, cyclohexyl and the like, but is not limited thereto.

Unless indicated otherwise, the term "aryl" used herein means aromatic hydrocarbons having-for example 6 to 10 carbon atoms. For example, the aryl may include phenyl, naphthyl and the like, but is not limited thereto.

Unless indicated otherwise, the term "heteroaryl" used herein means aromatic hydrocarbons including one or more heteroatoms selected from N, O and S as a ring member, and for example means 5- to 10-membered aromatic hydrocarbons. Examples of heteroaryl include, but are not limited to, pyridinyl, pyrimidinyl, pyridazinyl, pyrazinyl, oxadiazolyl, isoxadiazolyl, tetrazolyl, triazolyl, indolyl, indazolyl, isoxazolyl, oxazolyl, thiazolyl, isothiazolyl, furanyl, benzofuranyl, imidazolyl, thiophenyl, benzthiazole, benzimidazole, quinolinyl, indolinyl, 1,2,3,4-tetrahydroisoquinolyl, 3,4-dihydroisoquinolinyl, thiazolopyridyl, 2,3-dihydrobenzofuran, 2,3-dihydrothiophene, 2,3-dihydroindole, benzo[1,3]dioxin, chroman, thiochroman, 1,2,3,4-tetrahydroquinoline, 4H-benzo[1,3]dioxin, 2,3-dihydrobenzo[1,4]-dioxin, 6,7-dihydro-5H-cyclopenta[d]pyrimidine and the like.

Unless indicated otherwise, the term "carbocyclyl" used herein means a radical of a hydrocarbon that is unsaturated or partially or fully saturated, forming a single or fused cyclic ring having-for example 5 to 10 carbon atoms. The unsaturated carbocycle may include an aromatic hydrocarbon such as aryl.

Unless indicated otherwise, the term "heterocyclyl" used herein means unsaturated or partially or fully saturated, forming a single or fused cyclic ring, and having one or more heteroatoms-for example 1 to 3 heteroatoms selected from the group consisting of N, O and S. Specifically, the heterocyclyl may be a 5- to 12-membered hydrocarbon having 1 to 3 heteroatoms. The unsaturated heterocyclyl may include an aromatic hydrocarbon such as heteroaryl.

According to one embodiment of the present invention, in the above Formula 1 m represents an integer of 0, 1 or 2;
R₁ represents hydrogen, halo, cyano, C₁-C₇ alkyl, C₁-C₇ alkoxy, C₁-C₇ alkylcarbonyl or C₆-C₁₀ aryl;
R₂ represents hydrogen, halo, cyano, carboxy, C₁-C₇ alkyl, C₁-C₇ alkylcarbonyl, C₁-C₇ alkoxycarbonyl, aminocarbonyl, C₁-C₇ alkylaminocarbonyl, di(C₁-C₇ alkyl)aminocarbonyl, di(C₁-C₇ alkyl)amino-C₁-C₇ alkylaminocarbonyl, C₃-C₇ cycloalkyl, C₆-C₁₀ aryl, or 5- to 10-membered heteroaryl having 1 to 3 heteroatoms selected from N and O;
R₃ represents hydrogen or C₁-C₇ alkyl;
R₄ represents C₁-C₇ alkyl;
R₅ represents C₁-C₇ alkyl, or may combine with each other to form a C₂-C₄ ring when m is 2;
R₆ represents 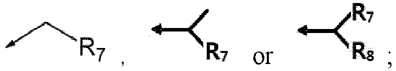 wherein R₇ and R₈ independently of one another represent C₅-C₁₀ carbocyclyl, or 5- to 12-membered heterocyclyl having 1 to 3 heteroatoms selected from N, O and S;
wherein the aryl, heteroaryl, carbocyclyl and heterocyclyl may be unsubstituted or substituted with 1 to 3 substituents selected from the group consisting of halo, C₁-C₇ alkyl, C₁-C₇ alkoxy, halo-C₁-C₇ alkyl, halo-C₁-C₇ alkoxy, C₆-C₁₀ aryl and C₆-C₁₀ aryloxy.

According to another embodiment of the present invention, in the above Formula 1
m represents an integer of 0, 1 or 2;
R₁ represents hydrogen, halo, cyano, C₁-C₅ alkyl, C₁-C₅ alkoxy, C₁-C₅ alkylcarbonyl or C₆ aryl;
R₂ represents hydrogen, halo, cyano, carboxy, C₁-C₅ alkyl, C₁-C₅ alkylcarbonyl, C₁-C₅ alkoxycarbonyl, aminocarbonyl, C₁-C₅ alkylaminocarbonyl, di(C₁-C₅ alkyl)aminocarbonyl, di(C₁-C₅ alkyl)amino-C₁-C₅ alkylaminocarbonyl, C₃-C₆ cycloalkyl, C₆ aryl, or 5- or 6-membered heteroaryl having 1 to 3 heteroatoms selected from N and O;
R₃ represents hydrogen or C₁-C₅ alkyl;
R₄ represents C₁-C₅ alkyl;
R₅ represents C₁-C₅ alkyl, or may combine with each other to form a C₂ ring when m is 2;
R₆ represents 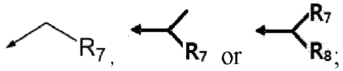 wherein R₇ represents C₆-C₁₀ aryl, 6- to 10-membered heteroaryl having 1 to 3 heteroatoms selected from N and O, partially saturated C₉-C₁₀ carbocyclyl, or partially saturated 9- or 10-membered heterocyclyl having 1 to 3 heteroatoms selected from N and O; and R₈ represents C₆-C₁₀ aryl;
wherein the aryl, heteroaryl, carbocyclyl and heterocyclyl may be unsubstituted or substituted with 1 to 3 substituents selected from the group consisting of halo, C₁-C₅ alkyl, C₁-C₅ alkoxy, halo-C₁-C₅ alkyl, halo-C₁-C₅ alkoxy, C₆-C₁₀ aryl and C₆-C₁₀ aryloxy.

Representative compounds of Formula 1 according to the present invention include, but are not limited to, the following compounds:
methyl-4-(1-(4-(trifluoromethoxy)benzyl)piperidin-4-yl)-1,4-dihydropyrido[2,3-b]pyrazine-2,3-dione;
1,6-dimethyl-4-(1-(4-(trifluoromethoxy)benzyl)piperidin-4-yl)-1,4-dihydropyrido[2,3-b]pyrazine-2,3-dione;
ethyl-4-(1-(4-(trifluoromethoxy)benzyl)piperidin-4-yl)-1,4-dihydropyrido[2,3-b]pyrazine-2,3-dione;
isobutyl-4-(1 -(4-(trifluoromethoxy)benzyl)piperidin-4-yl)-1,4-dihydropyrido[2,3-b]pyrazine-2,3-dione;
1,6-dimethyl-4-(1-(naphthalen-1-ylmethyl)piperidin-4-yl)-1,4-dihydropyrido[2,3-b]pyrazine-2,3-dione;
4-(1-((2-chloroquinolin-3-yl)methyl)piperidin-4-yl)-1,6-dimethyl-1,4-dihydropyrido[2,3-b]pyrazine -2,3-dione;
4-(1-((1,2-dimethyl-1H-indol-3-yl)methyl)piperidin-4-yl)-1,6-dimethyl-1,4-dihydropyrido[2,3-b]pyrazine-2,3-dione;
1,6-dimethyl-4-(1-((1-methyl-1H-indol-5-yl)methyl)piperidin-4-yl)-1,4-dihydropyrido[2,3-b]pyrazine-2,3-dione;
1,6-dimethyl-4-(1-(quinolin-4-ylmethyl)piperidin-4-yl)-1,4-dihydropyrido[2,3-b]pyrazine-2,3-dione;
4-(1-([1,1'-biphenyl]-2-ylmethyl)piperidin-4-yl)-1,6-dimethyl-1,4-dihydropyrido[2,3-b]pyrazine-2,3-dione;
1-methyl-2,3-dioxo-4-(1-(4-(trifluoromethoxy)benzyl)piperidin-4-yl)-1,2,3,4-tetrahydropyrido[2,3-b]pyrazine-6-carbonitrile;
4-(1-(2-chlorobenzyl)piperidin-4-yl)-1,6-dimethyl-1,4-dihydropyrido[2,3-b]pyrazine-2,3-dione;
4-(1-(3-chlorobenzyl)piperidin-4-yl)-1,6-dimethyl-1,4-dihydropyrido[2,3-b]pyrazine-2,3-dione;
4-(1-(4-chlorobenzyl)piperidin-4-yl)-1,6-dimethyl-1,4-dihydropyrido[2,3-b]pyrazine-2,3-dione;
4-(1-((2-methoxypyridin-3-yl)methyl)piperidin-4-yl)-1,6-dimethyl-1,4-dihydropyrido[2,3-b]pyrazine-2,3-dione;
4-(1-((6-fluoropyridin-2-yl)methyl)piperidin-4-yl)-1,6-dimethyl-1,4-dihydropyrido[2,3-b]pyrazine-2,3-dione;
4-(1-((6-fluoropyridin-3-yl)methyl)piperidin-4-yl)-1,6-dimethyl-1,4-dihydropyrido[2,3-b]pyrazine-2,3-dione;
1,6-dimethyl-4-(1-(4-phenoxybenzyl)piperidin-4-yl)-1,4-dihydropyrido[2,3-b]pyrazine-2,3 -dione;
1,6-dimethyl-4-(1-(naphthalen-2-ylmethyl)piperidin-4-yl)-1,4-dihydropyrido[2,3-b]pyrazine-2,3-dione;
4-(1-(isoquinolin-5-ylmethyl)piperidin-4-yl)-1,6-dimethyl-1,4-dihydropyrido[2,3-b]pyrazine-2,3-dione;
1,6-dimethyl-4-(1-(3-phenoxybenzyl)piperidin-4-yl)-1,4-dihydropyrido[2,3-b]pyrazine-2,3 -dione;
4-(1-(3-chlorobenzyl)piperidin-4-yl)-1-methyl-1,4-dihydropyrido[2,3-b]pyrazine-2,3 -dione;
4-(1-benzylpiperidin-4-yl)-1, 6-dimethyl-1,4-dihydropyri do[2,3-b]pyrazine-2,3-dione;
1,6-dimethyl-4-(1-(1-(quinoxalin-6-yl)ethyl)piperidin-4-yl)-1,4-dihydropyrido[2,3-b]pyrazine-2,3-dione;
4-(1-(3-chlorobenzyl)piperidin-4-yl)-1-methyl-2,3-dioxo-1,2,3,4-tetrahydropyrido[2,3-b]pyrazine-6-carbonitrile;
1-methyl-4-(1-(naphthalen-1-ylmethyl)piperidin-4-yl)-2,3-dioxo-1,2,3,4-tetrahydropyrido[2,3-b]pyrazine-6-carbonitrile;
7-chloro-4-(1-(3-chlorobenzyl)piperidin-4-yl)-1-methyl-1,4-dihydropyrido[2,3-b]pyrazine-2,3 -dione;
7-chloro-1-methyl-4-(1-(4-(trifluoromethoxy)benzyl)piperidin-4-yl)-1,4-dihydropyrido[2,3-b]pyrazine-2,3-dione;
7-chloro-1-methyl-4-(1-(naphthalen-1-ylmethyl)piperidin-4-yl)-1,4-dihydropyrido[2,3-b]pyrazine-2,3-dione;
7-chloro-1-methyl-4-(1-(3-(trifluoromethyl)benzyl)piperidin-4-yl)-1,4-dihydropyrido[2,3-b]pyrazine-2,3-dione;
7-chloro-4-(3,3-dimethyl-1-(4-(trifluoromethoxy)benzyl)piperidin-4-yl)-1-methyl-1,4-dihydropyrido[2,3-b]pyrazine-2,3-dione;
7-chloro-1-methyl-4-((1R,3s,5S)-8-(4-(trifluoromethoxy)benzyl)-8-azabicyclo[3.2.1]octan-3-yl)-1,4-dihydropyrido[2,3-b]pyrazine-2,3-dione;
7-chloro-1-methyl-4-(1-(quinoxalin-5-ylmethyl)piperidin-4-yl)-1,4-dihydropyrido[2,3-b]pyrazine-2,3-dione;
7-bromo-1-methyl-4-(1-(4-(trifluoromethoxy)benzyl)piperidin-4-yl)-1,4-dihydropyrido[2,3-b]pyrazine -2,3-dione;
7-chloro-4-(1-((5-chloronaphthalen-1-yl)methyl)piperidin-4-yl)-1-methyl-1,4-dihydropyrido[2,3-b]pyrazine-2,3-dione;
1-methyl-7-(1-methyl-1H-pyrazol-4-yl)-4-(1-(4-(trifluoromethoxy)benzyl)piperidin-4-yl)-1,4-dihydropyrido[2,3-b]pyrazine-2,3-dione;
7-chloro-1-methyl-4-(1-((5,6,7,8-tetrahydronaphthalen-1-yl)methyl)piperidin-4-yl)-1,4-dihydropyrido[2,3-b]pyrazine-2,3-dione;
7-bromo-1-methyl-2,3-dioxo-4-(1-(4-(trifluoromethoxy)benzyl)piperidin-4-yl)-1,2,3,4-tetrahydropyrido[2,3-b]pyrazine-6-carbonitrile;
7-(2-fluorophenyl)-1-methyl-4-(1-(4-(trifluoromethoxy)benzyl)piperidin-4-yl)-1,4-dihydropyrido[2,3-b]pyrazine-2,3-dione;
7-cyclopropyl-1-methyl-4-(1-(4-(trifluoromethoxy)benzyl)piperi din-4-yl)-1,4-dihydropyrido[2,3-b]pyrazine-2,3-dione;
7-chloro-6-(2-fluorophenyl)-1-methyl-4-(1-(4-(trifluoromethoxy)benzyl)piperidin-4-yl)-1,4-dihydropyrido[2,3-b]pyrazine-2,3-dione;
7-chloro-4-(1-(3-chloro-4-fluorobenzyl)piperidin-4-yl)-1-methyl-1,4-dihydropyrido[2,3-b]pyrazine-2,3-dione;
7-chloro-4-(1-(3-chloro-4-(trifluoromethoxy)benzyl)piperidin-4-yl)-1-methyl-1,4-dihydropyrido[2,3-b]pyrazine-2,3-dione;
7-cyclopropyl-1-methyl-2,3-dioxo-4-(1-(4-(trifluoromethoxy)benzyl)piperidin-4-yl)-1,2,3,4-tetrahydropyrido[2,3-b]pyrazine-6-carbonitrile;
4-(1-(bis(3-chlorophenyl)methyl)piperidin-4-yl)-7-chloro-1-methyl-1,4-dihydropyrido[2,3-b]pyrazine-2,3-dione;
7-chloro-4-(1-((2,3-dihydrobenzo[b][1,4]dioxin-6-yl)methyl)piperidin-4-yl)-1-methyl-1,4-dihydropyrido[2,3-b]pyrazine-2,3-dione;
1-methyl-2,3-dioxo-4-(1-(4-(trifluoromethoxy)benzyl)piperidin-4-yl)-1,2,3,4-tetrahydropyrido[2 ,3-b]pyrazine-7-carbonitrile;
7-acetyl-1-methyl-4-(1-(4-(trifluoromethoxy)benzyl)piperidin-4-yl)-1,4-dihydropyrido[2,3-b]pyrazine-2,3-dione;
7-chloro-4-((2R,5S)-2,5-dimethyl-1-(4-trifluoromethoxy)benzyl)piperidin-4-yl)-1-methyl-1,4-dihydropyrido[2,3-b]pyrazine-2,3-dione;
7-chloro-1,6-dimethyl-4-(1-(4-(trifluoromethoxy)benzyl)piperidin-4-yl)-1,4-dihydropyrido[2,3-b]pyrazine-2,3-dione;
7-bromo-1,6-dimethyl-4-(1-(4-(trifluoromethoxy)benzyl)piperidin-4-yl)-1,4-dihydropyrido[2,3- b]pyrazine-2,3-dione;
7-chloro-4-(1-((1-isopropyl-1H-pyrazol-4-yl)methyl)piperidin-4-yl)-1-methyl-1,4-dihydropyrido[2 ,3-b]pyrazine-2,3-dione;
6-acetyl-1-methyl-4-(1-(4-(trifluoromethoxy)benzyl)piperidin-4-yl)-1,4-dihydropyrido[2,3-b]pyrazine-2,3-dione;
6-chloro-1-methyl-4-(1-(4-(trifluoromethoxy)benzyl)piperidin-4-yl)-1,4-dihydropyrido[2,3-b]pyrazine-2,3-dione;
6-methoxy-1-methyl-4-(1-(4-(trifluoromethoxy)benzyl)piperidin-4-yl)-1,4-dihydropyrido[2,3-b]pyrazine-2,3-dione;
1,8-dimethyl-4-(1-(4-(trifluoromethoxy)benzyl)piperidin-4-yl)-1,4-dihydropyrido[2,3-b]pyrazine-2,3-dione;
7-chloro-1-methyl-4-((1R,3r,5S)-8-(4-(trifluoromethoxy)benzyl)-8-azabicyclo[3.2.1]octan-3-yl)-1,4-dihydropyrido[2,3-b]pyrazine-2,3-dione;
6-isopropoxy-1-methyl-4-(1-(4-(trifluoromethoxy)benzyl)piperidin-4-yl)-1,4-dihydropyrido[2,3-b]pyrazine-2,3-dione;
7-fluoro-1-methyl-4-(1-(4-(trifluoromethoxy)benzyl)piperidin-4-yl)-1,4-dihydropyrido[2,3-b]pyrazine -2,3-dione;
methyl 1-methyl-2,3-dioxo-4-(1-(4-(trifluoromethoxy)benzyl)piperidin-4-yl)-1,2,3,4-tetrahydropyrido[2,3-b]pyrazine-7-carboxylate;
1,7-dimethyl-4-(1-(4-(trifluoromethoxy)benzyl)piperidin-4-yl)-1,4-dihydropyrido[2,3-b]pyrazine-2,3-dione;
1-methyl-2,3-dioxo-4-(1-(4-(trifluoromethoxy)benzyl)piperidin-4-yl)-1,2,3,4-tetrahydropyrido[2,3-b]pyrazine-7-carboxylic acid;
N-(2-(dimethylamino)ethyl)-1-methyl-2,3-dioxo-4-(1-(4-(trifluoromethoxy)benzyl)piperidin-4-yl)-1,2,3,4-tetrahydropyrido[2,3-b]pyrazine-7-carboxamide;
N,N,1-trimethyl-2,3-dioxo-4-(1-(4-(trifluoromethoxy)benzyl)piperidin-4-yl)-1,2,3,4-tetrahydropyrido[2,3-b]pyrazine-7-carboxamide;
N,1-dimethyl-2,3-dioxo-4-(1-(4-(trifluoromethoxy)benzyl)piperidin-4-yl)-1,2,3,4-tetrahydropyrido[2,3-b]pyrazine-7-carboxamide;
1-methyl-2,3-dioxo-4-(1-(4-(trifluoromethoxy)benzyl)piperidin-4-yl)-1,2,3,4-tetrahydropyrido[2,3-b]pyrazine-7-carboxamide; and
4-(1-(3-chlorobenzyl)piperidin-4-yl)-1,7-dimethyl-1,4-dihydropyrido[2,3-b]pyrazine-2,3-dione.

The terms and abbreviations used herein retain their original meanings unless indicated otherwise.

Hereinafter, the method for preparing the compound of Formula 1 is explained based on exemplary reactions in order to illustrate the present invention. However, a person skilled in the art could prepare the compound of Formula 1 by various methods based on the structure of Formula 1, and such methods should be interpreted as being within the scope of the present invention. That is, the compound of Formula 1 may be prepared by the methods described herein or by combining various methods disclosed in the prior art, which should be interpreted as being within the scope of the present invention. Accordingly, a method for preparing the compound of Formula 1 is not limited to the following methods.

For example, the compound of Formula 1 may be prepared according to the following Reaction Scheme 1, 2 or 3.

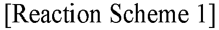

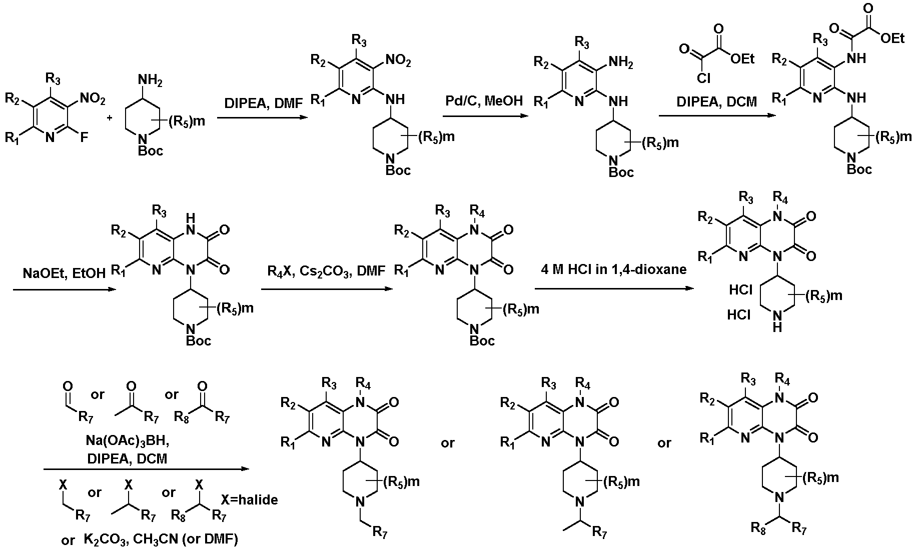

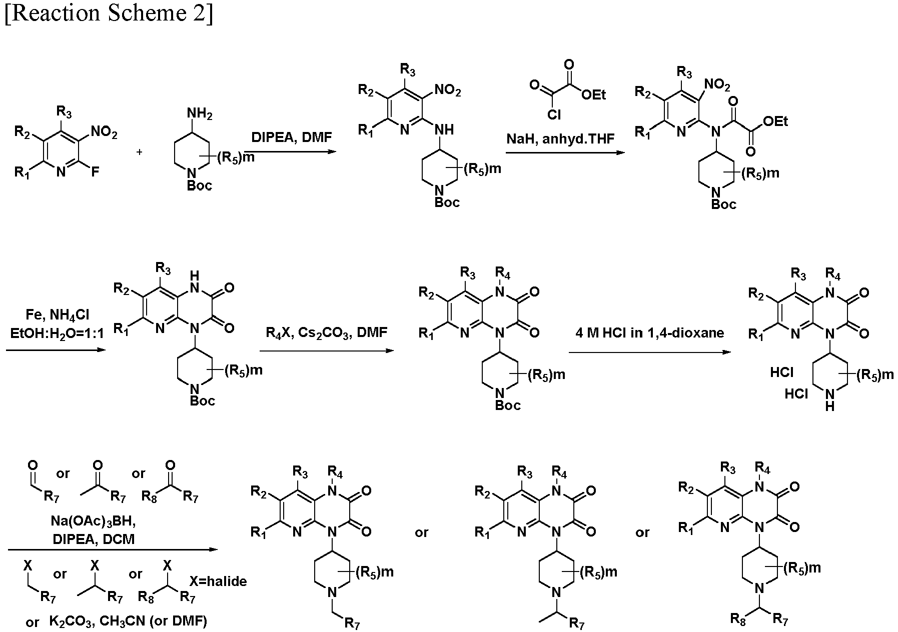

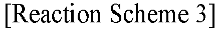

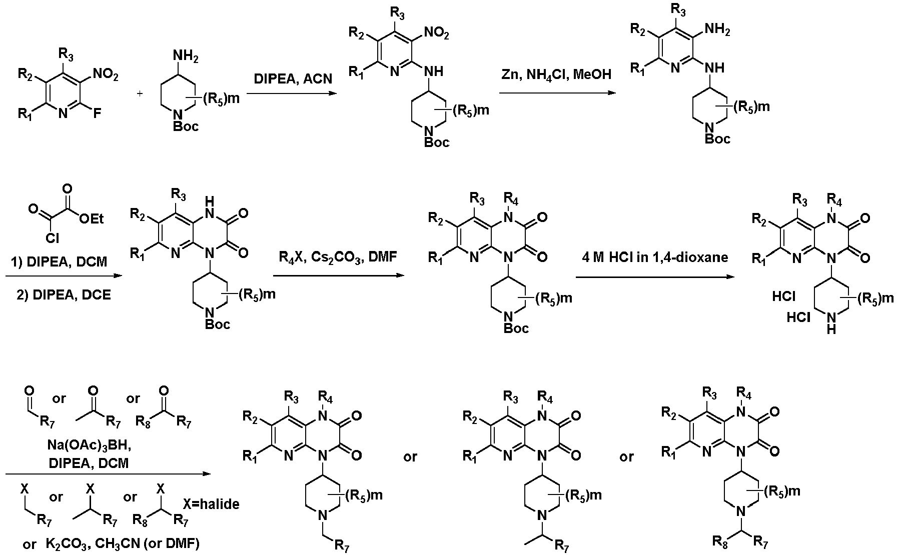

The compound of Formula 1 according to the present invention exhibits inhibitory activity against diacylglycerol kinases (DGKs). Accordingly, the present invention provides a pharmaceutical composition for the prevention or treatment of diseases associated with diacylglycerol kinases comprising the compound of Formula 1, or a pharmaceutically acceptable salt or stereoisomer thereof, together with a pharmaceutically acceptable carrier.

In one embodiment according to the present invention, the disease associated with diacylglycerol kinases is cancer. Exemplary cancers which can be treated by the pharmaceutical composition according to the present invention include, but are not limited to, gastrointestinal cancer, pancreatic cancer, breast cancer, colon cancer, retinoblastoma, liver cancer, lung cancer, ovarian cancer, cervical cancer, endometrial cancer, brain tumor, testicular cancer, laryngeal cancer, prostate cancer, neuroblastoma, kidney cancer, thyroid cancer, esophageal cancer, skin cancer, osteosarcoma and bladder cancer.

In the present invention, a "pharmaceutical composition" may include other components such as carriers, diluents, excipients, etc., in addition to the active ingredient of the present invention. Accordingly, the pharmaceutical composition may include pharmaceutically acceptable carriers, diluents, excipients or combinations thereof, if necessary. The pharmaceutical composition facilitates the administration of compounds into the body. Various methods for administering the compounds include, but are not limited to, oral, injection, aerosol, parenteral and local administration.

Herein, a "carrier" means a compound that facilitates the addition of compounds into the cell or tissue. For example, dimethylsulfoxide (DMSO) is a conventional carrier facilitating the administration of many organic compounds into living cells or tissues.

Herein, a "diluent" means a compound that not only stabilizes a biologically active form but is diluted in solvent dissolving the compounds. A dissolved salt in buffer is used as a diluent in this field. A conventionally used buffer is a phosphate buffer saline mimicking salt form in body fluid. Since a buffer solution can control the pH of the solution at low concentration, a buffer diluent hardly modifies the biological activity of compounds.

Herein, "pharmaceutically acceptable" means such property that does not impair the biological activity and physical property of compounds.

The compounds according to the present invention can be formulated as various pharmaceutically administered dosage forms. In the preparation of the pharmaceutical composition of the present invention, an active component-specifically, the compound of Formula 1 or a pharmaceutically acceptable salt or stereoisomer thereof-is mixed with selected pharmaceutically acceptable carriers considering the dosage form to be prepared. For example, the pharmaceutical composition of the present invention can be formulated as injections, oral preparations and the like, as needed.

The compound of the present invention can be formulated by conventional methods using known pharmaceutical carriers and excipients, and inserted into a unit or multi-unit containers. The formulations may be solution, suspension or emulsion in oil or aqueous solvent and include conventional dispersing agents, suspending agents or stabilizing agents. In addition, the compound may be, for example, dry powder form which is dissolved in sterilized pyrogen-free water before use. The compound of the present invention can be formulated into suppositories by using a conventional suppository base such as cocoa butter or other glycerides. Solid forms for oral administration include capsules, tablets, pills, powders and granules. Capsules and tablets are preferred. Tablets and pills are preferably enteric-coated. Solid forms are manufactured by mixing the compounds of the present invention with at least one carrier selected from inert diluents such as sucrose, lactose or starch, lubricants such as magnesium stearate, disintegrating agents, binders and the like.

The compound or a pharmaceutical composition comprising the same according to the present invention can be administered in combination with other drugs-for example, other cancer immunotherapy-as required.

The dose of the compound of Formula 1 according to the present invention is determined by a physician's prescription considering the patient's body weight, age and disease condition. A typical dose for adults is in the range of about 0.3 to 500 mg per day according to the frequency and intensity of administration. A typical daily dose of intramuscular or intravenous administration for adults is in the range of about 1 to 300 mg per day which can be administered in divided unit dosages. Some patients need a higher daily dose.

Herein, the term "treatment" is used to mean deterring, delaying or ameliorating the progress of diseases in a subject exhibiting symptoms of diseases.

### EFFECTS OF THE INVENTION

The heterocyclic compound represented by Formula 1 according to the present invention can be usefully used in the prevention or treatment of diseases associated with diacylglycerol kinases (DGKs) such as cancer by inhibiting diacylglycerol kinases.

### MODE FOR THE INVENTION

Hereinafter, the present invention is explained in more detail with the following examples. However, it must be understood that the protection scope of the present invention is not limited to the examples.

The descriptions of abbreviations and terms used in the Preparation Examples and Examples are as follows:
CH₃CN: acetonitrile
DCM: dichloromethane
DIPEA: N,N-diisopropylethylamine
DME: dimethoxyethane
DMF: N,N-dimethylformamide
EtOAc: ethyl acetate
EtOH: ethanol
HATU: hexafluorophosphate azabenzotriazole tetramethyl uronium
HCl: hydrochloric acid
MeOH: methanol
MPLC: medium pressure liquid chromatography
NaH: sodium hydride
NBS: N-bromosuccinimide
NCS: N-chlorosuccinimide
Pd(OAc)₂: palladium(II) acetate
(PPh₄)₄Pd: tetrakis(triphenylphosphine)palladium(0)
THF: tetrahydrofuran
TLC: thin layer chromatography
Zn(CN)₂: zinc cyanide

### Preparation Example 1: Preparation of 1-methyl-4-(piperidin-4-yl)-1,4-dihydropyrido[2,3-b]pyrazine-2,3-dione hydrochloride

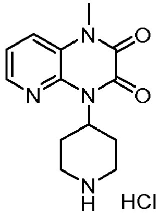

### Step A: Preparation of tert-butyl 4-((3-nitropyridin-2-yl)amino)piperidine-1-carboxylate

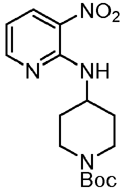

Tert-butyl 4-amidopiperidine-1-carboxylate (1.00 g, 5.00 mmol) and 2-fluoro-3-nitropyridine (0.71 g, 5.00 mmol) were dissolved in DMF (15 mL), and isopropylamine (1.75 mL, 10.00 mmol) was added thereto, followed by stirring at 60°C for 4 hours. The reaction mixture was cooled to room temperature, diluted with EtOAc, washed twice with brine, dried over anhydrous magnesium sulfate and concentrated under reduced pressure. The residue was purified by MPLC to obtain the title compound (1.53 g).
¹H-NMR (500 MHz, CHLOROFORM-D) δ 8.46~8.41 (m, 2H), 8.19 (dd, 1H), 6.68 (dd, 1H), 4.41~4.38 (m, 1H), 4.09 (br s, 2H), 3.03 (br s, 2H), 2.11~2.07 (m, 2H), 1.55~1.53 (m, 2H), 1.50 (s, 9H)
LC/MS: 267 (M+H-t-Bu)

### Step B: Preparation of tert-butyl 4-((3-aminopyridin-2-yl)amino)piperidine-1-carboxylate

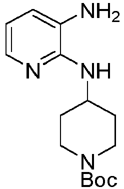

To tert-butyl 4-((3-nitropyridin-2-yl)amino)piperidine-1-carboxylate (0.50 g, 1.55 mmol) and 10% palladium on carbon (49.5 mg, 0.047 mmol), methanol (40 mL) was added and stirred at room temperature for 4 hours under a hydrogen balloon. The reaction mixture was filtered and concentrated under reduced pressure to obtain the title compound (440 mg), which was used directly in the next reaction without purification.
LC/MS: 293 (M+H)

### Step C: Preparation of tert-butyl 4-((3-(2-ethoxy-2-oxoacetamido)pyridin-2-yl)amino)piperidine-1 -carboxylate

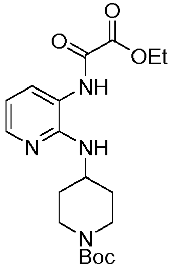

Tert-butyl 4-((3-aminopyridin-2-yl)amino)piperidine-1-carboxylate (130 mg, 0.18 mmol) and diisopropylamine (0.23 mL, 1.33 mmol) were dissolved in dichloromethane (10 mL), and ethyl oxalyl chloride (0.075 mL, 0.67 mmol) was added thereto at 0°C, followed by stirring at room temperature for 2 hours. The reaction mixture was diluted with water and extracted with EtOAc. The organic layer was dried over anhydrous magnesium sulfate and then concentrated under reduced pressure to obtain the title compound (146 mg), which was used directly in the next reaction without purification.
LC/MS: 393 (M+H)

### Step D: Preparation of tert-butyl 4-(2,3-dioxo-2,3-dihydropyrido[2,3-b]pyrazin-4(1H)-yl)piperidine-1-carboxylate

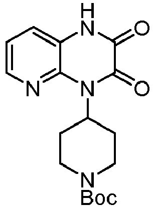

Tert-butyl 4-((3-(2-ethoxy-2-oxoacetamido)pyridin-2-yl)amino)piperidine-1-carboxylate (70 mg, 0.18 mmol) was dissolved in EtOH (6 mL), and sodium ethoxide (0.35 mL, 0.89 mmol, 20 wt% in EtOH) was added thereto, followed by stirring at 70°C for 0.5 hour. The reaction mixture was cooled to room temperature, diluted with water, acidified to about pH 5 with 1 N HCl, and extracted twice with EtOAc. The organic layer was dried over anhydrous magnesium sulfate and then concentrated under reduced pressure to obtain the title compound (42 mg), which was used directly in the next reaction without purification.
LC/MS: 347 (M+H), 369 (M+Na)

### Step E: Preparation of tert-butyl 4-(1-methyl-2,3-dioxo-2,3-dihydropyrido[2,3-b]pyrazin-4(1H)-yl)piperidine-1-carboxylate

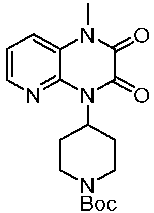

Tert-butyl 4-(2,3-dioxo-2,3-dihydropyrido[2,3-b]pyrazin-4(1H)-yl)piperidine-1-carboxylate (42 mg, 0.12 mmol) was dissolved in DMF (5 mL), and cesium carbonate (158 mg, 0.49 mmol) and idomethane (0.011 mL, 0.18 mmol) were added thereto, followed by stirring at room temperature for 0.5 hour. The reaction mixture was diluted with water and extracted 3 times with EtOAc. The organic layer was dried over anhydrous magnesium sulfate, concentrated under reduced pressure, and the residue was purified by preparatory TLC to obtain the title compound (19 mg).
¹H-NMR (500 MHz, CHLOROFORM-D) δ 8.27~8.26 (m, 1H), 7.53~7.51 (m, 1H), 7.25~7.23 (m, 1H), 5.58 (s, 1H), 4.29 (br s, 2H), 3.64 (s, 3H), 2.91~2.87 (m, 4H), 1.65 (br s, 2H), 1.51 (s, 9H)
LC/MS: 383 (M+Na)

### Step F: Preparation of 1-methyl-4-(piperidin-4-yl)-1,4-dihydropyrido[2,3-b]pyrazine-2,3-dione hydrochloride

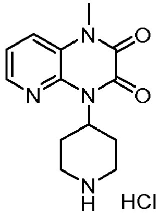

Tert-butyl 4-(1-methyl-2,3-dioxo-2,3-dihydropyrido[2,3-b]pyrazin-4(1H)-yl)piperidine-1-carboxylate (19 mg, 0.053 mmol) was dissolved in dichloromethane (4 mL), and HCl (0.26 mL, 1.05 mmol, 4 M in 1,4-dioxane) was added thereto, followed by stirring at room temperature for 3 hours. The reaction mixture was concentrated under reduced pressure to obtain the title compound (18 mg), which was used directly in the next reaction without purification.
LC/MS: 261 (M+H)

### Preparation Example 2: Preparation of 7-chloro-1-methyl-4-(piperidin-4-yl)-1,4-dihydropyrido[2,3-b]pyrazine-2,3-dione dihydrochloride

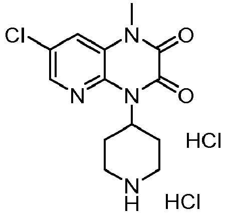

### Step A: Preparation of tert-butyl 4-((5-chloro-3-nitropyridin-2-yl)amino)piperidine-1 -carboxylate

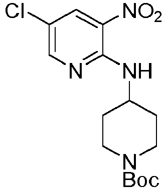

Tert-butyl 4-amidopiperidine-1-carboxylate (5.96 g, 29.7 mmol) and 5-chloro-2-fluoro-3-nitropyridine (5.00 g, 28.3 mmol) were used in the same manner as in Step A of Preparation Example 1 to obtain the title compound (10.0 g).
¹H-NMR (400 MHz, CHLOROFORM-D) δ 8.40 (d, J = 2.3 Hz, 1H), 8.34 (d, J = 2.7 Hz, 1H), 8.09 (d, J = 7.3 Hz, 1H), 4.30 (tt, J= 10.7, 3.5 Hz, 1H), 4.18-3.95 (m, 2H), 2.98 (t, J = 12.1 Hz, 2H), 2.05 (d, J = 10.5 Hz, 2H), 1.53-1.48 (2H), 1.46 (s, 9H)
LC/MS: 357 (M+H), 379 (M+Na)

### Step B: Preparation of tert-butyl 4-(N-(5-chloro-3-aminopyridin-2-yl)-2-ethoxy-2-oxoacetoamido)piperidine-1-carboxylate

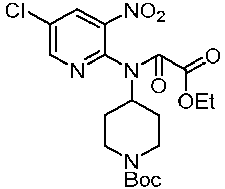

Tert-butyl 4-((5-chloro-3-nitropyridin-2-yl)amino)piperidine-1-carboxylate (3.20 g, 8.97 mmol) was dissolved in THF (100 mL), and NaH (538 mg, 13.45 mmol, 60% in dispersion oil) was slowly added thereto at 0°C, followed by stirring for 30 minutes. Ethyl oxalyl chloride (1.99 mL, 17.94 mmol) was added to the reaction mixture at 0°C, followed by stirring at room temperature for 1 hour. Water was slowly added to the reaction mixture to terminate the reaction. The reaction mixture was diluted with EtOAc, washed with 0.5 N aqueous NaOH solution, sodium bicarbonate aqueous solution and brine, and the organic layer was dried over anhydrous sodium sulfate and concentrated under reduced pressure. The residue was purified by MPLC to obtain the title compound (1.32 g).
LC/MS: 479 (M+Na)

### Step C: Preparation of tert-butyl 4-(7-chloro-2,3-dioxo-2,3-dihydropyrido[2,3-b]pyrazin-4(1H)-yl)piperidin-1-carboxylate

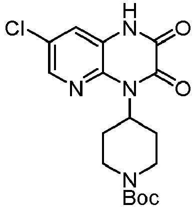

Tert-butyl 4-(N-(5-chloro-3-aminopyridin-2-yl)-2-ethoxy-2-oxoacetoamido)piperidine-1-carboxylate (1.90 g, 4.16 mmol) was dissolved in EtOH (30 mL), and then water (30 mL), iron (2.32 g, 41.6 mmol) and ammonium chloride (4.05 g, 76 mmol) were added thereto at room temperature and stirred at 70°C for 4 hours. The reaction mixure was cooled to room temperature, filtered under reduced pressure with Celite and extracted with EtOAc. The organic layer was dried over anhydrous sodium sulfate and concentrated under reduced pressure to obtain the title compound (1.28 g), which was used directly in the next reaction without purification.
LC/MS: 325 (M+H-t-Bu)

### Step D: Preparation of tert-butyl 4-(7-chloro-1-methyl-2,3-dioxo-2,3-dihydropyrido[2,3-b]pyrazin-4(1H)-yl)piperidin-1-carboxylate

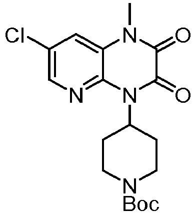

The residue—which was obtained by using tert-butyl 4-(7-chloro-2,3-dioxo-2,3-dihydropyrido[2,3-b]pyrazin-4(1H)-yl)piperidine-1-carboxylate (1.28 g, 3.36 mmol) in the same method as in Step E of Preparation Example 1-was purified by MPLC to obtain the title compound (1.08 g).
LC/MS: 417 (M+Na)

### Step E: Preparation of 7-chloro-1-methyl-4-(piperidin-4-yl)-1,4-dihydropyrido[2,3-b]pyrazine-2,3-dione dihydrochloride

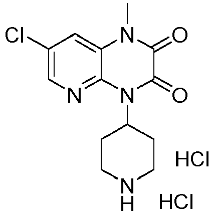

Tert-butyl 4-(7-chloro-1-methyl-2,3-dioxo-2,3-dihydropyrido[2,3-b]pyrazin-4(1H)-yl)piperidine-1-carboxylate (1.08 g, 2.74 mmol) was used in the same manner as in Step F of Preparation Example 1 to obtain the title compound (1,000 mg), which was used directly in the next reaction without purification.
¹H-NMR (400 MHz, METHANOL-D4) δ 8.23 (d, J = 1.8 Hz, 1H), 7.90 (d, J = 1.8 Hz, 1H), 5.72 (t, J = 11.7 Hz, 1H), 3.59 (s, 4H), 3.54 (d, J = 12.3 Hz, 2H), 3.22-3.02 (m, 3H), 1.97 (d, J = 15.6 Hz, 2H)
LC/MS: 295 (M+H)

### Preparation Example 3: Preparation of 1,6-dimethyl-4-(piperidin-4-yl)-1,4-dihydropyrido[2,3-b]pyrazine-2,3-dione dihydrochloride

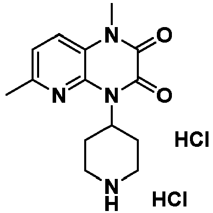

### Step A: Preparation of tert-butyl 4-((6-methyl-3-nitropyridin-2-yl)amino)piperidine-1 -carboxylate

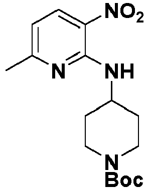

2-Fluoro-6-methyl-3-nitropyridine (0.781 g, 5.00 mmol) was used in the same manner as in Step A of Preparation Example 1 to obtain the title compound (1.61 g).
¹H-NMR (500 MHz, CHLOROFORM-D) δ 8.31 (d, J = 8.5 Hz, 1H), 8.24 (d, J = 7.0 Hz, 1H), 6.51 (d, J= 8.5 Hz, 1H), 4.45-4.40 (m, 1H), 4.07 (s, 2H), 3.07-3.02 (m, 2H), 2.48 (s, 3H), 2.08 (d, J = 10.4 Hz, 2H), 1.58-1.52 (m, 2H), 1.50 (s, 9H)
LC/MS: 337 (M+H)

### Step B: Preparation of 6-tert-butyl 4-((3-amino-6-methylpyridin-2-yl)amino)piperidine-1-carboxylate

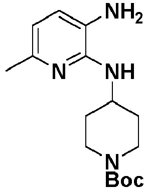

Tert-butyl 4-((6-methyl-3-nitropyridin-2-yl)amino)piperidine-1-carboxylate (800 mg, 2.38 mmol) was used in the same manner as in Step B of Preparation Example 1 to obtain the title compound (720 mg).
LC/MS: 307 (M+H), 329 (M+Na)

### Step C: Preparation of tert-butyl 4-((3-(2-ethoxy-2-oxoacetamido)-6-methylpyridin-2-yl)amino)piperidine-1-carboxylate

6-Tert-butyl 4-((3-amino-6-methylpyridin-2-yl)amino)piperidine-1-carboxylate (720 mg, 2.35 mmol) was used in the same manner as in Step C of Preparation Example 1 to obtain the title compound (898 mg).
¹H-NMR (500 MHz, CHLOROFORM-D) δ 8.43 (s, 1H), 7.41 (d, J = 7.6 Hz, 1H), 7.28 (s, 2H), 6.53 (d, J = 7.9 Hz, 1H), 4.46 (q, J = 7.2 Hz, 2H), 4.28 (d, J = 7.0 Hz, 1H), 4.18-4.12 (m, 2H), 4.06 (s, 1H), 2.98 (t, J = 11.3 Hz, 2H), 2.41 (s, 4H), 2.09-2.05 (m, 4H), 1.49 (s, 9H)
LC/MS: 408 (M+H).

### Step D: Preparation of tert-butyl 4-(6-methyl-2,3-dioxo-2,3-dihydropyrido[2,3-b]pyrazin-4(1H)-yl)piperidine-1-carboxylate

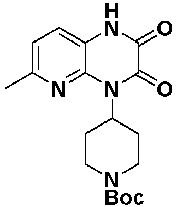

Tert-butyl 4-((3-(2-ethoxy-2-oxoacetamido)-6-methylpyridin-2-yl)amino)piperidine-1-carboxylate (898 mg, 2.21 mmol) was used in the same manner as in Step D of Preparation Example 1 to obtain the title compound (781 mg).
LC/MS: 383 (M+Na), 743 (2M+Na)

### Step E: Preparation of tert-butyl 4-(1,6-dimethyl-2,3-dioxo-2,3-dihydropyrido[2,3-b]pyrazin-4(1H)-yl)piperidine-1-carboxylate

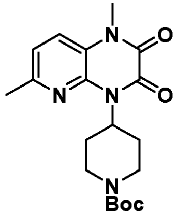

Tert-butyl 4-(6-methyl-2,3-dioxo-2,3-dihydropyrido[2,3-b]pyrazin-4(1H)-yl)piperidine-1-carboxylate (660 mg, 1.83 mmol) was used in the same manner as in Step E of Preparation Example 1 to obtain the title compound (322 mg).
¹H-NMR (500 MHz, DMSO-D6) δ 7.71 (d, J = 8.2 Hz, 1H), 7.18 (d, J= 8.2 Hz, 1H), 5.47 (s, 1H), 4.09 (s, 2H), 3.50-3.43 (m, 3H), 2.85 (s, 1H), 2.66-2.60 (m, 2H), 2.48-2.35 (m, 4H), 1.60 (d, J = 9.5 Hz, 2H), 1.50-1.24 (m, 9H)
LC/MS: 375 (M+H)

### Step F: Preparation of 1,6-dimethyl-4-(piperidin-4-yl)-1,4-dihydropyrido[2,3-b]pyrazine-2,3-dione hydrochloride

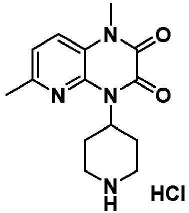

Tert-butyl 4-(1,6-dimethyl-2,3-dioxo-2,3-dihydropyrido[2,3-b]pyrazin-4(1H)-yl)piperidine-1-carboxylate (300 mg, 0.83 mmol) was used in the same manner as in Step F of Preparation Example 1 to obtain the title compound (256 mg).
LC/MS: 275 (M+H)

### Preparation Example 4: Preparation of 1-methyl-2,3-dioxo-4-(piperidin-4-yl)-1,2,3,4-tetrahydropyrido[2,3-b]pyrazine-6-carbonitrile dihydrochloride

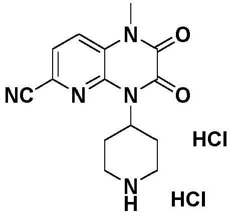

### Step A: Preparation of tert-butyl 4-((6-chloro-3-nitropyridin-2-yl)amino)piperidine-1 -carboxylate

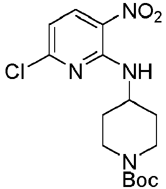

Tert-butyl 4-amidopiperidine-1-carboxylate (4.21 g, 21.0 mmol) and 2,6-dichloro-3-nitropyridine (4.20 g, 20.0 mmol) were used in the same manner as in Step A of Preparation Example 1 to obtain the title compound (6.21 g).
¹H-NMR (500 MHz, CHLOROFORM-D) δ 8.37 (d, J = 8.5 Hz, 1H), 8.30 (d, J = 7.5 Hz, 1H), 6.65 (d, J = 9.0 Hz, 1H), 4.37~4.33 (m, 1H), 4.09 (br s, 2H), 3.04 (br s, 2H), 2.08 (d, J = 10.0 Hz, 2H), 1.57-1.52 (m, 2H), 1.50 (s, 9H)
LC/MS: 379 (M+Na)

### Step B: Preparation of tert-butyl 4-((6-cyano-3-nitropyridin-2-yl)amino)piperidine-1-carboxylate

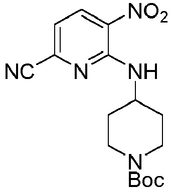

Tert-butyl 4-((6-chloro-3-nitropyridin-2-yl)amino)piperidine-1-carboxylate (2.0 g, 5.61 mmol), Zn(CN)₂ (0.987 g, 8.41) mmol) and (PPh₄)₄Pd (0.648 g, 0.561 mmol) were dissolved in DMF (50 mL) and stirred at 100°C for 1 hour. The reaction mixture was cooled to room temperature, diluted with EtOAc, and washed with an aqueous sodium bicarbonate solution and brine. The organic layer was dried over anhydrous sodium sulfate and concentrated under reduced pressure, and the residue was purified by MPLC to obtain the title compound (1.84 g).
¹H-NMR (500 MHz, CHLOROFORM-D) δ 8.55 (d, J = 8.5 Hz, 1H), 8.14 (d, J = 7.5 Hz, 1H), 7.03 (d, J= 8.5 Hz, 1H), 4.39~4.33 (m, 1H), 4.17~4.12 (m, 2H), 3.06~3.01 (m, 2H), 2.10~2.08 (m, 2H), 1.57-1.52 (m, 2H), 1.51 (s, 9H)
LC/MS: 370 (M+Na)

### Step C: Preparation of tert-butyl 4-(N-(6-cyano-3-nitropyridin-2-yl)-2-ethoxy-2-oxoacetamido)piperidine-1 -carboxylate

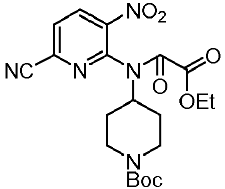

Tert-butyl 4-((6-cyano-3-nitropyridin-2-yl)amino)piperidine-1-carboxylate (1.04 g, 2.99 mmol) was used in the same manner as in Step B of Preparation Example 2 to obtain the title compound (1.02 g).
LC/MS: 470 (M+Na)

### Step D: Preparation of tert-butyl 4-(6-cyano-2,3-dioxo-2,3-dihydropyrido[2,3-b]pyrazin-4(1H)-yl)piperidine-1-carboxylate

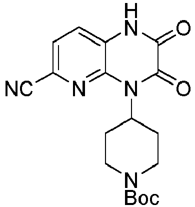

Tert-butyl 4-(N-(6-cyano-3-nitropyridin-2-yl)-2-ethoxy-2-oxoacetamido)piperidine-1-carboxylate (0.51 g, 1.14 mmol) was used in the same manner as in Step C of Preparation Example 2 to obtain the title compound (0.388 g), which was used directly in the next reaction without purification.
LC/MS: 394 (M+Na)

### Step E: Preparation of tert-butyl 6-cyano-1-methyl-2,3-dioxo-2,3-dihydropyrido[2,3-b]pyrazin-4(1H)-yl)piperidine-1-carboxylate

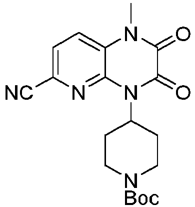

The residue—which was obtained by using tert-butyl 4-(6-cyano-2,3-dioxo-2,3-dihydropyrido[2,3-b]pyrazin-4(1H)-yl)piperidine-1-carboxylate (0.388 g, 1.045 mmol) in the same manner as in Step E of Preparation Example 1-was purified by MPLC to obtain the title compound (0.368 g).
¹H-NMR (500 MHz, CHLOROFORM-D) δ 7.64 (d, J = 8.5 Hz, 1H), 7.57 (d, J = 8.5 Hz, 1H), 5.50~5.47 (m, 1H), 4.37~4.29 (m, 2H), 3.67 (s, 3H), 2.91 (m, 2H), 2.84~2.80 (m, 2H), 1.68-1.65 (m, 2H), 1.53 (s, 9H)
LC/MS: 408 (M+Na)

### Step F: Preparation of 1-methyl-2,3-dioxo-4-(piperidin-4-yl)-1,2,3,4-tetrahydropyrido[2,3-b]pyrazine-6-carbonitrile hydrochloride

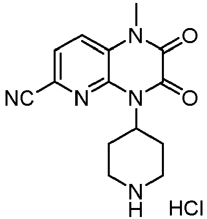

Tert-butyl 4-(6-cyano-1-methyl-2,3-dioxo-2,3-dihydropyrido[2,3-b]pyrazin-4(1H)-yl)piperidine-1-carboxylate (0.300 g, 0.778 mmol) was used in the same manner as in Step F of Preparation Example 1 to obtain the title compound (250 mg), which was used directly in the next reaction without purification.
LC/MS: 286 (M+H), 308 (M+Na)

### Preparation Example 5: Preparation of 7-chloro-4-(3,3-dimethylpiperidin-4-yl)-1-methyl-1,4-dihydropyrido[2,3-b]pyrazine-2,3-dione dihydrochloride

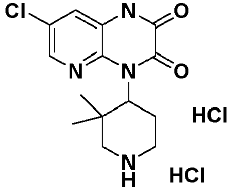

### Step A: Preparation of tert-butyl 4-((5-chloro-3-nitropyridin-2-yl)amino)-3,3-dimethylpiperidine-1 -carboxylate

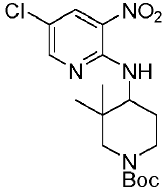

Tert-butyl 4-amido-3,3-dimethylpiperidine-1-carboxylate (0.951 g, 4.16 mmol) and 5-chloro-2-fluoro-3-nitropyridine (0.700 g, 3.97) mmol) were used in the same manner as in Step A of Preparation Example 1 to obtain the title compound (1.36 g).
¹H-NMR (500 MHz, CHLOROFORM-D) δ 8.44 (d, J = 2.5 Hz, 1H), 8.35 (d, J = 2.5 Hz, 1H), 8.28 (d, J= 8.5 Hz, 1H), 4.36~4.31 (m, 1H), 4.19~4.06 (m, 1H), 3.88~3.76 (m, 1H), 2.95 (br s, 1H), 2.78~2.74 (m, 1H), 1.84 (br s, 1H), 1.68-1.61 (m, 1H), 1.50 (s, 9H), 1.04 (s, 3H), 0.96 (s, 3H)
LC/MS: 407 (M+Na)

### Step B: Preparation of tert-butyl 4-(N-(5-chloro-3-nitropyridin-2-yl)-2-ethoxy-2-oxoacetamido)-3,3-dimethylpiperidine-1-carboxylate

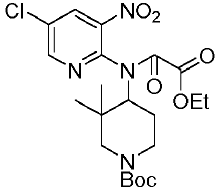

Tert-butyl 4-((5-chloro-3-nitropyridin-2-yl)amino)-3,3-dimethylpiperidine-1-carboxylate (1.01 g, 2.62 mmol) was used in the same manner as in Step B of Preparation Example 2 to obtain the title compound (0.120 g).
LC/MS: 507 (M+Na)

### Step C: Preparation of tert-butyl 4-(7-chloro-2,3-dioxo-2,3-dihydropyrido[2,3-b]pyrazin-4(1H)-yl)-3,3-dimethylpiperidine-1-carboxylate

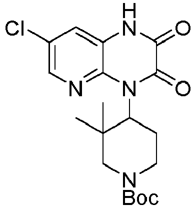

Tert-butyl 4-(N-(5-chloro-3-nitropyridin-2-yl)-2-ethoxy-2-oxoacetamido)-3,3-dimethylpiperidine-1-carboxylate (0.23 g, 0.474 mmol) was used in the same manner as in Step C of Preparation Example 2 to obtain the title compound (0.161 g), which was used directly in the next reaction without purification.
LC/MS: 431 (M+Na)

### Step D: Preparation of tert-butyl 4-(7-chloro-1-methyl-2,3-dioxo-2,3-dihydropyrido[2,3-b]pyrazin-4(1H)-yl)-3,3-dimethylpiperidine-1-carboxylate

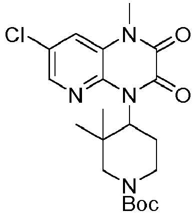

The residue—which was obtained by using tert-butyl 4-(7-chloro-2,3-dioxo-2,3-dihydropyrido[2,3-b]pyrazin-4(1H)-yl)-3,3-dimethylpiperidine-1-carboxylate (0.161 g, 0.394 mmol) in the same manner as in Step E of Preparation Example 1-was purified by MPLC to obtain the title compound (0.100 g).
¹H-NMR (500 MHz, CHLOROFORM-D) δ 8.21 (d, J = 2.0 Hz, 1H), 7.48 (dd, J = 7.5, 2.0 Hz, 1H), 5.68~5.32 (2 dd, 1H), 4.42~4.29 (m, 1H), 3.96~3.81 (m, 1H), 3.64 (s, 3H), 3.49~3.37 (m, 1H), 2.84~2.74 (m, 2H), 1.58~1.52 (m, 1H), 1.50 (s, 9H), 1.12~1.05 (2 s, 3H), 0.94~0.83 (2 s, 3H)
LC/MS: 445 (M+Na)

### Step E: Preparation of 7-chloro-4-(3,3-dimethylpiperidin-4-yl)-1-methyl-1,4-dihydropyrido[2,3-b]pyrazine-2,3-dione dihydrochloride

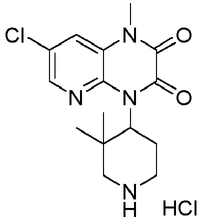

Tert-butyl 4-(7-chloro-1-methyl-2,3-dioxo-2,3-dihydropyrido[2,3-b]pyrazin-4(1H)-yl)-3,3-dimethylpiperidine-1-carboxylate (0.100 g, 0.236 mmol) was used in the same manner as in Step F of Preparation Example 1 to obtain the title compound (0.094 g), which was used directly in the next reaction without purification.
LC/MS: 323 (M+H)

### Preparation Example 6: Preparation of 4-(8-azabicyclo[3.2.1]octan-3-yl)-7-chloro-1-methyl-1,4-dihydropyrido[2,3-b]pyrazine-2,3-dione dihydrochloride

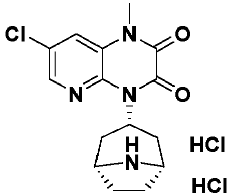

### Step A: Preparation of tert-butyl (1R,3s,5S)-3-((5-chloro-3-nitropyridin-2-yl)amino)-8-azabicyclo[3.2.1]octane-8-carboxylate

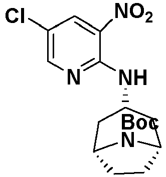

5-Chloro-2-fluoro-3-nitropyridine (5.00 g, 28.3 mmol) and tert-butyl (1R,3s,5S)-3-amino-8-azabicyclo[3.2.1]octane-8-carboxylate (6.41 g, 28.30 mmol) were used in the same manner as in Step A of Preparation Example 2 to obtain the title compound (10.71 g).
¹H-NMR (500 MHz, DMSO-D6) δ 8.67 (d, J = 6.7 Hz, 1H), 8.56 (d, J = 2.4 Hz, 1H), 8.52 (d, J = 2.4 Hz, 1H), 4.44 (q, J = 6.4 Hz, 1H), 4.13 (s, 2H), 2.15 (d, J = 12.2 Hz, 2H), 1.98 (s, 4H), 1.82 (d, J = 13.7 Hz, 2H), 1.43 (s, 9H)
LC/MS: 405 (M+Na)

### Step B: Preparation of tert-butyl (1R,3s,5S)-3-(N-(5-chloro-3-nitropyridin-2-yl)-2-ethoxy-2-oxoacetamido)-8-azabicyclol3.2.11octane-8-carboxylate

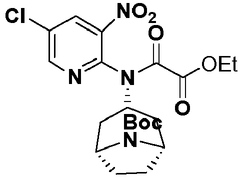

Tert-butyl (1R,3s,5S)-3-((5-chloro-3-nitropyridin-2-yl)amino)-8-azabicyclo[3.2.1]octane-8-carboxylate (3.20 g, 8.36 mmol) was used in the same manner as in Step B of Preparation Example 2 to obtain the title compound (1.17 g).
¹H-NMR (500 MHz, CHLOROFORM-D) δ 8.68 (s, 1H), 8.36 (d, J = 2.4 Hz, 1H), 4.29 (d, J = 29.6 Hz, 2H), 4.16-4.12 (m, 2H), 3.94 (t, J = 8.5 Hz, 1H), 2.56 (d, J = 27.2 Hz, 1H), 1.98 (s, 2H), 1.72-1.65 (m, 3H), 1.52-1.49 (m, 1H), 1.46 (s, 9H), 1.33-1.24 (m, 4H)
LC/MS: 483 (M+H), 505 (M+Na)

### Step C: Preparation of tert-butyl 1R,3s,5S)-3-(7-chloro-2,3-dioxo-2,3-dihydropyrido[2,3-b]pyrazine-4(1H)-yl)-8-azabicyclo[3.2.1]octane-8-carboxylate

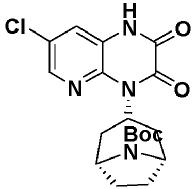

Tert-butyl (1R,3s,5S)-3-(N-(5-chloro-3-nitropyridin-2-yl)-2-ethoxy-2-oxoacetamido)-8-azabicyclo[3.2.1]octane-8-carboxylate (1.17 g, 2.42 mmol) was used in the same manner as in Step C of Preparation Example 2 to obtain the title compound (0.77 g).
¹H-NMR (500 MHz, DMSO-D6) δ 12.14 (br s, 1H), 8.14 (d, J = 2.1 Hz, 1H), 7.50 (d, J = 2.1 Hz, 1H), 5.27 (q, J = 9.6 Hz, 1H), 4.22 (d, J = 34.2 Hz, 2H), 2.26-2.18 (m, 4H), 1.95 (d, J = 39.7 Hz, 2H), 1.85 (s, 2H), 1.47 (s, 9H)
LC/MS: 407 (M+H)

### Step D: Preparation of tert-butyl (1R,3s,5S)-3-(7-chloro-1-methyl-2,3-dioxo-2,3-dihydropyrido[2,3-b]pyrazin-4(1H)-yl)-8-azabicyclo[3.2.1]octane-8-carboxylate

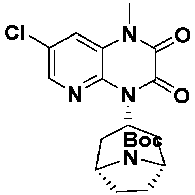

Tert-butyl (1R,3s,5S)-3-(7-chloro-2,3-dioxo-2,3-dihydropyrido[2,3-b]pyrazin-4(1H)-yl)-8-azabicyclo[3.2.1]octane-8-carboxylate (0.72 g, 1.78 mmol) was used in the same manner as in Step D of Preparation Example 2 to obtain the title compound (0.75 g).
¹H-NMR (500 MHz, CHLOROFORM-D) δ 8.17 (d, J = 2.1 Hz, 1H), 7.49 (d, J = 2.1 Hz, 1H), 5.59-5.51 (m, 1H), 4.36 (d, J = 58.0 Hz, 1H), 3.64 (s, 3H), 2.37 (m, 3H), 2.27-2.19 (m, 2H), 2.12-1.98 (m, 4H), 1.56 (s, 9H)
LC/MS: 443 (M+Na)

### Step E: Preparation of 4-(8-azabicyclo[3.2.1]octan-3-yl)-7-chloro-1-methyl-1,4-dihydropyridol2,3-b]pyrazine-2,3-dione dihydrochloride

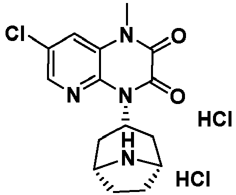

Tert-butyl (1R,3s,5S)-3-(7-chloro-1-methyl-2,3-dioxo-2,3-dihydropyrido[2,3-b]pyrazin-4(1H)-yl)-8-azabicyclo[3.2.1]octane-8-carboxylate (590 mg, 1.40 mmol) was used in the same manner as in Step E of Preparation 2 to obtain the title compound (440 mg).
LC/MS: 321 (M+H)

### Preparation Example 7: Preparation of 7-bromo-1-methyl-4-(piperidin-4-yl)-1,4-dihydropyrido[2,3-b]pyrazine-2,3-dione dihydrochloride

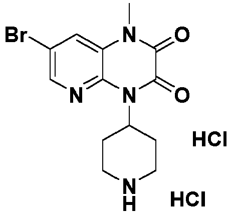

### Step A: Preparation of tert-butyl 4-((5-bromo-3-nitropyridin-2-yl)amino)piperidine-1 -carboxylate

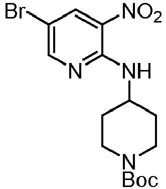

To a solution in which tert-butyl 4-((3-nitropyridin-2-yl)amino)amidopiperidine-1-carboxylate (1.00 g, 3.10 mmol) was dissolved in CH₃CN (20 mL), NBS (0.552 g, 3.10 mmol) was added and stirred at room temperature for 18 hours. After the reaction solution was concentrated under reduced pressure, the residue was diluted with EtOAc and washed with an aqueous sodium bicarbonate solution, and the organic layer was dried over anhydrous sodium sulfate and concentrated under reduced pressure. The residue was purified by MPLC to obtain the title compound (1.23 g).
¹H-NMR (400 MHz, CHLOROFORM-D) δ 8.53 (d, J = 2.4 Hz, 1H), 8.40 (d, J = 2.4 Hz, 1H), 8.10 (d, J = 7.2 Hz, 1H), 4.31~4.28 (m, 1H), 4.10-4.04 (m, 2H), 3.01~2.95 (m, 2H), 2.05~2.03 (m, 2H), 1.52-1.48 (2H), 1.46 (s, 9H)
LC/MS: 433, 435 (M+Na)

### Step B: Preparation of tert-butyl 4-(N-(5-bromo-3-nitropyridin-2-yl)-2-ethoxy-2-oxoacetamido)piperidine-1 -carboxylate

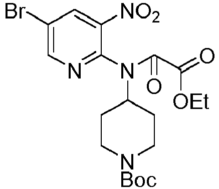

Tert-butyl 4-((5-bromo-3-nitropyridin-2-yl)amino)piperidine-1-carboxylate (1.23 g, 3.07 mmol) was used in the same manner as in Step B of Preparation Example 2 to obtain the title compound (0.880 g).
LC/MS: 523, 525 (M+Na)

### Step C: Preparation of tert-butyl 4-(7-bromo-2,3-dioxo-2,3-dihydropyrido[2,3-b]pyrazin-4(1H)-yl)piperidine-1-carboxylate

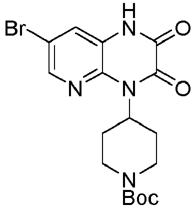

Tert-butyl 4-(N-(5-bromo-3-nitropyridin-2-yl)-2-ethoxy-2-oxoacetamido)piperidine-1-carboxylate (0.880 g, 1.755 mmol) was used in the same manner as in Step C of Preparation Example 2 to obtain the title compound (0.480 g), which was used directly in the next reaction without purification.
LC/MS: 447, 449 (M+Na)

### Step D: Preparation of tert-butyl 4-(7-bromo-1-methyl-2,3-dioxo-2,3-dihydropyrido[2,3-b]pyrazin-4(1H)-yl)-3,3-dimethylpiperidine-1-carboxylate

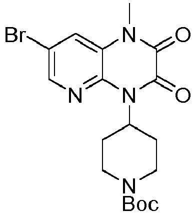

The residue—which was obtained by using tert-butyl 4-(7-bromo-2,3-dioxo-2,3-dihydropyrido[2,3-b]pyrazin-4(1H)-yl)piperidine-1-carboxylate (0.480 g, 1.129 mmol) in the same manner as in Step E of Preparation Example 1-was purified by MPLC to obtain the title compound (0.335 g).
¹H-NMR (400 MHz, CHLOROFORM-D) δ 8.29 (d, J = 1.2 Hz, 1H), 7.63 (d, J = 1.6 Hz, 1H), 5.51~5.49 (m, 1H), 4.31~4.27 (m, 2H), 3.63 (s, 3H), 2.87~2.83 (m, 4H), 1.65 (br s, 2H), 1.52 (s, 9H)
LC/MS: 461, 463 (M+Na)

### Step E: Preparation of 7-bromo-4-(piperidin-4-yl)-1-methyl-1,4-dihydropyrido[2,3-b]pyrazine-2,3-dione dihydrochloride

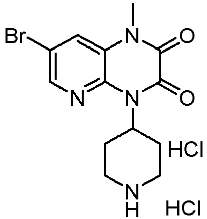

Tert-butyl 4-(7-bromo-1-methyl-2,3-dioxo-2,3-dihydropyrido[2,3-b]pyrazin-4(1H)-yl)piperidine-1-carboxylate (0.335 g, 0.763 mmol) was used in the same manner as in Step F of Preparation Example 1 to obtain the title compound (0.314 g), which was used directly in the next reaction without purification.
LC/MS: 338, 340 (M+H)

### Preparation Example 8: Preparation of 1-methyl-2,3-dioxo-4-(piperidin-4-yl)-1,2,3,4-tetrahydropyrido[2,3-b]pyrazine-7-carbonitrile dihydrochloride

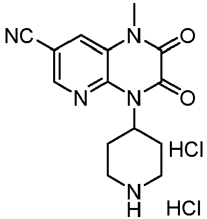

### Step A: Preparation of tert-butyl 4-(7-cyano-1-methyl-2,3-dioxo-2,3-dihydropyrido[2,3-b]pyrazin-4(1H)-yl)piperidine-1-carboxylate

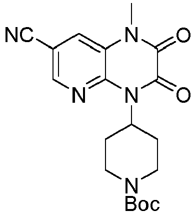

7-Bromo-1-methyl-4-(piperidin-4-yl)-1,4-dihydropyrido[2,3-b]pyrazine-2,3-dione dihydrochloride (0.120 g, 0.273 mmol) obtained in Preparation Example 7 was used in the same manner as in Step B of Preparation Example 4 to obtain the title compound (0.098 g).
¹H-NMR (500 MHz, CHLOROFORM-D) δ 8.52 (d, J = 1.5 Hz, 1H), 7.73 (d, J = 1.5 Hz, 1H), 5.55~5.50 (m, 1H), 4.31~4.30 (m, 2H), 3.67 (s, 3H), 2.84~2.77 (m, 4H), 1.67-1.65 (m, 2H), 1.51 (s, 9H)
LC/MS: 408 (M+Na)

### Step B: Preparation of 1-methyl-2,3-dioxo-4-(piperidin-4-yl)-1,2,3,4-tetrahydropyrido[2,3-b]pyrazine-7-carbonitrile dihydrochloride

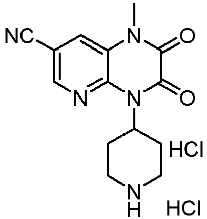

Tert-butyl 4-(7-cyano-1-methyl-2,3-dioxo-2,3-dihydropyrido[2,3-b]pyrazin-4(1H)-yl)piperidine-1-carboxylate (0.098 g, 0.254 mmol) was used in the same manner as in Step F of Preparation Example 1 to obtain the title compound (0.091 g), which was used directly in the next reaction without purification.
LC/MS: 286 (M+H)

### Preparation Example 9: Preparation of 7-acetyl-1-methyl-4-(piperidin-4-yl)-1,4-dihydropyrido[2,3-b]pyrazine-2,3-dione dihydrochloride

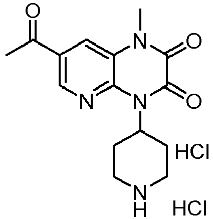

### Step A: Preparation of tert-butyl 4-(7-(1-ethoxyvinyl)-1-methyl-23-dioxo-23-dihydropyrido[2,3-b]pyrazin-4(1H)-yl)piperidine-1-carboxylate

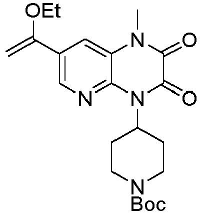

To 7-bromo-1-methyl-4-(piperidin-4-yl)-1,4-dihydropyrido[2,3-b]pyrazine-2,3-dione dihydrochloride (0.150 g, 0.341 mmol) obtained in Preparation Example 7, tributyl(1-ethoxyvinyl)stannane (148 mg, 0.410 mmol) and tetrakis(triphenylphosphine) palladium (40 mg, 0.034 mmol), 1,4-dioxane (10 mL) was added and stirred at 100°C for 3 hours. The reaction mixture was cooled to room temperature, filtered through Celite, and the filtrate was concentrated under reduced pressure. The residue was purified by MPLC to obtain the title compound (0.134 g).
¹H-NMR (500 MHz, CHLOROFORM-D) δ 8.50 (d, J = 2.0 Hz, 1H), 7.70 (d, J = 2.0 Hz, 1H), 5.64~5.60 (m, 1H), 4.73 (d, J= 3.0 Hz, 1H), 4.36 (d, J= 3.0 Hz, 1H), 4.30~4.28 (m, 2H), 3.99 (q, J = 7.0 Hz, 2H), 3.67 (s, 3H), 2.91~2.89 (m, 4H), 1.67-1.65 (m, 2H), 1.52 (s, 9H), 1.47 (t, J = 7.0 Hz, 3H)
LC/MS: 453 (M+Na)

### Step B: Preparation of 7-acetyl-1-methyl-4-(piperidin-4-yl)-1,4-dihydropyrido[2,3-b]pyrazine-2,3-dione dihydrochloride

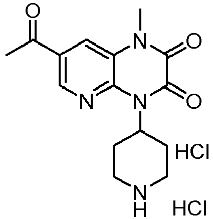

Tert-butyl 4-(7-(1-ethoxyvinyl)-1-methyl-2,3-dioxo-2,3-dihydropyrido[2,3-b]pyrazin-4(1H)-yl)piperidine-1-carboxylate (0.134 g, 0.311 mmol) was used in the same manner as in Step F of Preparation Example 1 to obtain the title compound (0.111 g), which was used directly in the next reaction without purification.
LC/MS: 303 (M+H)

### Preparation Example 10: Preparation of 7-bromo-1-methyl-2,3-dioxo-4-(piperidin-4-yl)-1,2,3,4-tetrahydropyrido[2,3-b]pyrazine-6-carbonitrile dihydrochloride

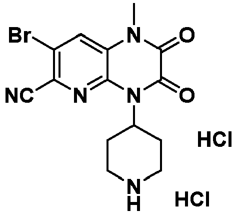

### Step A: Preparation of tert-butyl 4-((5-bromo-6-chloro-3-nitropyridin-2-yl)amino)piperidine-1 -carboxylate

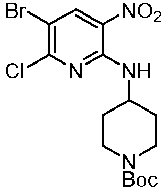

Tert-butyl 4-((6-chloro-3-nitropyridin-2-yl)amino)piperidine-1-carboxylate (1.00 g, 2.80 mmol) obtained in Step A of Preparation Example 3 was used in the same manner as in Step A of Preparation Example 5 to obtain the title compound (1.08 g).
¹H-NMR (400 MHz, CHLOROFORM-D) δ 8.62 (s, 1H), 8.16 (d, J = 5.6 Hz, 1H), 4.33~4.27 (m, 1H), 4.15-4.09 (m, 2H), 3.06~3.01 (m, 2H), 2.09~2.07 (m, 2H), 1.55-1.52 (m, 2H), 1.50 (s, 9H)
LC/MS: 457, 459 (M+Na)

### Step B: Preparation of tert-butyl 4-((5-bromo-6-cyano-3-nitropyridin-2-yl)amino)piperidine-1-carboxylate

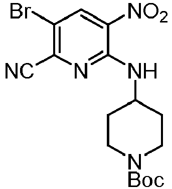

Tert-butyl 4-((5-bromo-6-chloro-3-nitropyridin-2-yl)amino)piperidine-1-carboxylate (0.500 g, 1.148 mmol) was used in the same manner as in Step B of Preparation Example 4 to obtain the title compound (0.201 g).
¹H-NMR (400 MHz, CHLOROFORM-D) δ 8.69 (s, 1H), 8.05 (d, J = 7.5 Hz, 1H), 4.33~4.28 (m, 1H), 4.17-4.12 (m, 2H), 3.05~3.00 (m, 2H), 2.09~2.07 (m, 2H), 1.55-1.52 (m, 2H), 1.50 (s, 9H)
LC/MS: 523, 525 (M+Na)

### Step C: Preparation of tert-butyl 4-(N-(5-bromo-6-cyano-3-nitropyridin-2-yl)-2-ethoxy-2-oxoacetamido)piperidine-1-carboxylate

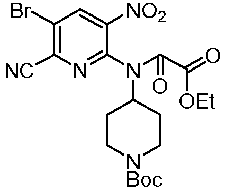

Tert-butyl 4-((5-bromo-6-cyano-3-nitropyridin-2-yl)amino)piperidine-1-carboxylate (0.370 g, 0.868 mmol) was used in the same manner as in Step B of Preparation Example 2 to obtain the title compound (0.251 g).
LC/MS: 548, 550 (M+Na)

### Step D: Preparation of tert-butyl 4-(7-bromo-6-cyano-2,3-dioxo-2,3-dihydropyrido[2,3-b]pyrazin-4(1H)-yl)piperidine-1-carboxylate

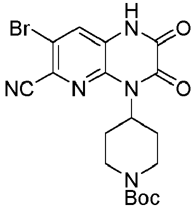

Tert-butyl 4-(N-(5-bromo-6-cyano-3-nitropyridin-2-yl)-2-ethoxy-2-oxoacetamido)piperidine-1-carboxylate (0.251 g, 0.477 mmol) was used in the same manner as in Step C of Preparation Example 2 to obtain the title compound (0.215 g), which was used directly in the next reaction without purification.
LC/MS: 472, 474 (M+Na)

### Step E: Preparation of tert-butyl 4-(7-bromo-6-cyano-1-methyl-2,3-dioxo-2,3-dihydropyrido[2,3-b]pyrazine-4(1H)-yl)piperidine-1-carboxylate

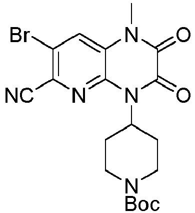

The residue—which was obtained by using tert-butyl 4-(7-bromo-6-cyano-2,3-dioxo-2,3-dihydropyrido[2,3-b]pyrazin-4(1H)-yl)piperidine-1-carboxylate (0.215 g, 0.477 mmol) in the same manner as in Step E of Preparation Example 1-was purified by MPLC to obtain the title compound (0.114 g).
¹H-NMR (500 MHz, CHLOROFORM-D) δ 8.29 (d, J = 1.2 Hz, 1H), 7.63 (d, J = 1.6 Hz, 1H), 5.51~5.49 (m, 1H), 4.31~4.27 (m, 2H), 3.63 (s, 3H), 2.87~2.83 (m, 4H), 1.65 (br s, 2H), 1.52 (s, 9H)
LC/MS: 486, 488 (M+Na)

### Step F: Preparation of 7-bromo-1-methyl-2,3-dioxo-4-(piperidin-4-yl)-1,2,3,4-tetrahydropyrido[2,3-b]pyrazine-6-carbonitrile dihydrochloride

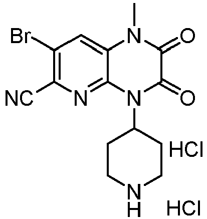

Tert-butyl 4-(7-bromo-6-cyano-1-methyl-2,3-dioxo-2,3-dihydropyrido[2,3-b]pyrazin-4(1H)-yl)piperidine-1-carboxylate (0.114 g, 0.246 mmol) was used in the same manner as in Step F of Preparation Example 1 to obtain the title compound (0.107 g), which was used directly in the next reaction without purification.
LC/MS: 364, 366 (M+H)

### Preparation Example 11: Preparation of 7-chloro-6-(2-fluorophenyl)-1-methyl-4-(piperidin-4-yl)-1,4-dihydropyrido[2,3-b]pyrazine-2,3-dione hydrochloride

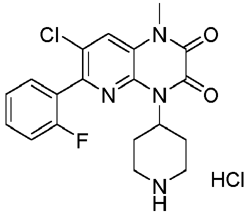

### Step A: Preparation of tert-butyl 4-((5,6-dichloro-3-nitropyridin-2-yl)amino)piperidine-1-carboxylate

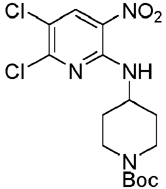

Tert-butyl 4-((6-chloro-3-nitropyridin-2-yl)amino)piperidine-1-carboxylate (1.5 g, 4.20 mmol) obtained in Step A of Preparation Example 4 was dissolved in CH₃CN (50 mL), and NCS (0.842 g, 6.31 mmol) was added thereto, followed by stirring at 50°C for 5 hours. The reaction mixture was concentrated under reduced pressure, diluted with EtOAc and washed with an aqueous sodium bicarbonate solution and brine. The organic layer was dried over anhydrous sodium sulfate and concentrated under reduced pressure, and the residue was purified by MPLC to obtain the title compound (1.57 g).
LC/MS: 413, 415 (M+Na)

### Step B: Preparation of tert-butyl 4-(N-(5,6-dichloro-3-nitropyridin-2-yl)-2-ethoxy-2-oxoacetamido)piperidine-1-carboxylate

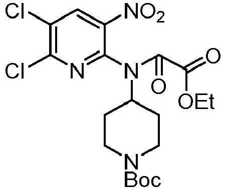

Tert-butyl 4-((5,6-dichloro-3-nitropyridin-2-yl)amino)piperidine-1 -carboxylate (0.920 g, 2.351 mmol) was used in the same manner as in Step B of Preparation Example 2 to obtain the title compound (1.05 g).
LC/MS: 513, 515 (M+Na)

### Step C: Preparation of tert-butyl 4-(6,7-dichloro-2,3-dioxo-2,3-dihydropyrido[2,3-b]pyrazin-4(1H)-yl)piperidine-1-carboxylate

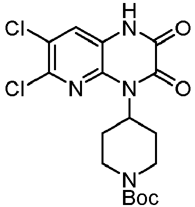

Tert-butyl 4-(N-(5,6-dichloro-3-nitropyridin-2-yl)-2-ethoxy-2-oxoacetamido)piperidine-1-carboxylate (220 mg, 0.448 mol) was used in the same manner as in Step C of Preparation Example 2 to obtain the title compound (0.186 g), which was used directly in the next reaction without purification.
LC/MS: 437, 439 (M+Na)

### Step D: Preparation of tert-butyl 4-(6,7-dichloro-1-methyl-23-dioxo-23-dihydropyrido[2,3-b]pyrazin-4(1H)-yl)piperidine-1-carboxylate

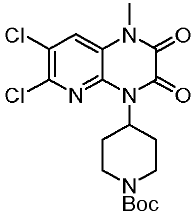

The residue-which was obtained by using tert-butyl 4-(6,7-dichloro-1-methyl-2,3-dioxo-2,3-dihydropyrido[2,3-b]pyrazin-4(1H)-yl)piperidine-1-carboxylate (0.186 g, 0.448 mmol) in the same manner as in Step E of Preparation Example 1-was purified by MPLC to obtain the title compound (0.121 g).
LC/MS: 451. 453 (M+Na)

### Step E: Preparation of tert-butyl 4-(7-chloro-6-(2-fluorophenyl)-1-methyl-2,3-dioxo-2,3-dihydropyrido[2,3-b]pyrazin-4(1H)-yl)piperidine-1-carboxylate

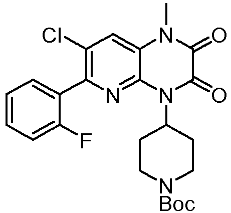

Tert-butyl 4-(6,7-dichloro-1-methyl-2,3-dioxo-2,3-dihydropyrido[2,3-b]pyrazin-4(1H)-yl)piperidine-1-carboxylate (0.090 g, 0.210 mmol), (2-fluorophenyl)boronic acid (44 mg, 0.314), 2 M aqueous sodium carbonate solution (0.314 mL) and tetrakistriphenylphosphinepalladium (24 mg, 0.02 mmol) were dissolved in 1,4-dioxane (5 mL) and stirred at 90°C for 3 hours. The reaction mixture was cooled to room temperature and filtered through Celite, and the filtrate was concentrated under reduced pressure. The residue was purified by MPLC to obtain the title compound (89 mg).
¹H-NMR (500 MHz, CHLOROFORM-D) δ 7.62 (s, 1H), 7.49∼7.46 (m, 2H), 7.32-7.28(m, 1H), 7.24∼7.20 (m, 2H), 5.48∼5.45 (m, 1H), 4.30∼4.20 (m, 2H), 3.67 (s, 3H), 2.81 (br s, 4H), 1.67 (br s, 2H), 1.44 (s, 9H)
LC/MS: 511 (M+Na)

### Step F: Preparation of 7-chloro-6-(2-fluorophenyl)-1-methyl-4-(piperi din-4-yl)-1,4-dihydropyrido[2,3-b]pyrazine-2,3-dione hydrochloride

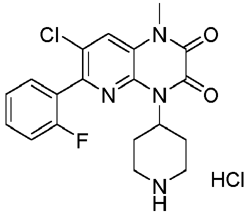

Tert-butyl 4-(7-chloro-6-(2-fluorophenyl)-1-methyl-2,3-dioxo-2,3-dihydropyrido[2,3-b]pyrazin-4(1H)-yl)piperidine-1-carboxylate (0.089 g, 0.182 mmol) was used in the same manner as in Step F of Preparation Example 1 to obtain the title compound (80 mg), which was used directly in the next reaction without purification.
LC/MS: 389 (M+H)

### Preparation Example 12: Preparation of 1-ethyl-4-(piperidin-4-yl)-1,4-dihydropyrido[2,3-b]pyrazine-2,3-dione dihydrochloride

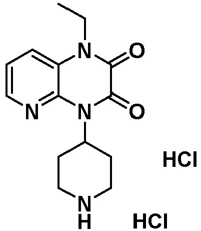

### Step A: Preparation of tert-butyl 4-(1-ethyl-2,3-dioxo-2,3-dihydropyrido[2,3-b]pyrazin-4(1H)-yl)piperidin-1-carboxylates

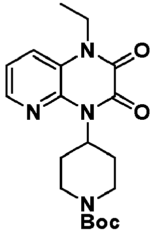

Tert-butyl 4-(2,3-dioxo-2,3-dihydropyrido[2,3-b]pyrazin-4(1H)-yl)piperidine-1-carboxylate (50 mg, 0.144 mmol) obtained in Step D of Preparation Example 1 and iodoethane (17.50 µl, 0.217 mmol) were used in the same manner as in Step E of Preparation Example 1 to obtain the title compound (50 mg).
LC/MS: 375 (M+H)

### Step B: Preparation of 1-ethyl-4-(piperidin-4-yl)-1,4-dihydropyrido[2,3-b]pyrazine-2,3-dione dihydrochloride

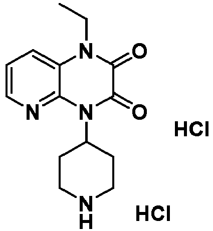

Tert-butyl 4-(1-ethyl-2,3-dioxo-2,3-dihydropyrido[2,3-b]pyrazin-4(1H)-yl)piperidine-1-carboxylate (50 mg, 0.134 mmol) was used in a similar manner to Step F of Preparation Example 1 to obtain the title compound (36 mg), and was used directly in the next reaction without purification.
LC/MS: 275 (M+H)

### Preparation Example 13: Preparation of 1-isobutyl-4-(piperidin-4-yl)-1,4-dihydropyrido[2,3-b]pyrazine-2,3-dione dihydrochloride

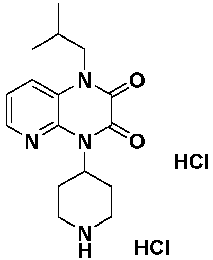

### Step A: Preparation of tert-butyl 4-(1-isobutyl-2,3-dioxo-2,3-dihydropyrido[2,3-b lpyrazin-4(lH)-yl)piperidine-1-carboxylate

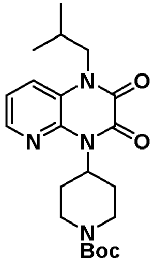

Tert-butyl 4-(2,3-dioxo-2,3-dihydropyrido[2,3-b]pyrazin-4(1H)-yl)piperidin-1-carboxylate (50 mg, 0.144 mmol) obtained in Step D of Preparation Example 1 and 1-bromo-2-methylpropane (29.7 mg, 0.217 mmol) were used in the same manner as in Step E of Preparation Example 1 to obtain the title compound (26 mg).
LC/MS: 403 (M+H)

### Step B: Preparation of 1-isobutyl-4-(piperidin-4-yl)-1,4-dihydropyrido[2,3-b]pyrazine-2,3-dione dihydrochloride

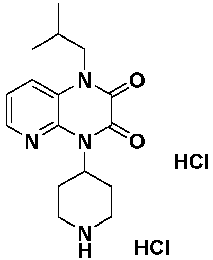

Tert-butyl 4-(1-isobutyl-2,3-dioxo-2,3-dihydropyrido[2,3-b]pyrazin-4(1H)-yl)piperidine-1-carboxylate (26 mg, 0.065 mmol) was used in a similar manner to Step F of Preparation Example 1 to obtain the title compound (19 mg).
LC/MS: 303 (M+H)

### Preparation Example 14: Preparation of 1,2-dimethyl-1H-indole-3-carbaldehvde

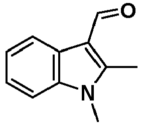

2-Methyl-1H-indole-3-carbaldehyde (100 mg, 0.628 mmol) was used in the same manner as in Step E of Preparation Example 1 to obtain the title compound (100 mg).
LC/MS: 174 (M+H)

### Preparation Example 15: Preparation of 1-methyl-1H-indole-5-carbaldehyde

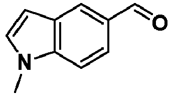

1H-indole-5-carbaldehyde (100 mg, 0.689 mmol) was used in the same manner as in Step E of Preparation Example 1 to obtain the title compound (109 mg).
LC/MS: 160 (M+H)

### Preparation Example 16: Preparation of 6-(1-chloroethyl)quinoxaline

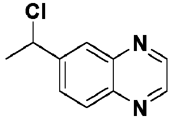

### Step A: Preparation of 1-(quinoxalin-6-yl)ethan-1-ol

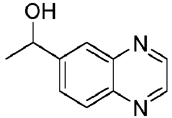

1-(Quinoxalin-6-yl)ethan-1-one (500 mg, 2.90 mmol) was dissolved in MeOH (5,808 µl), and sodium borohydride (165 mg, 4.36 mmol) was added thereto at 0°C, followed by stirring for hours. After the reaction was terminated by adding water to the reaction mixture, the reaction mixture was extracted 3 times with EtOAc. The organic layer was dried over anhydrous magnesium sulfate and concentrated under reduced pressure, and the residue was purified by MPLC to obtain the title compound (312 mg).
¹H-NMR (400 MHz, CHLOROFORM-D) δ 8.81 (dd, J= 3.9, 1.1 Hz, 2H), 8.16-7.99 (m, 2H), 7.82 (dd, J = 8.7, 1.8 Hz, 1H), 5.15 (q, J = 6.4 Hz, 1H), 1.60 (d, J = 6.9 Hz, 3H)
LC/MS: 175 (M+H)

### Step B: Preparation of 6-(1-chloroethyl)quinoxaline

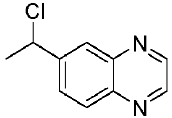

1-(Quinoxalin-6-yl)ethan-1-ol (312 mg, 1.791 mmol) was dissolved in DCM, and SOCl₂ (196 µl, 2.69 mmol) was added thereto at 0°C, followed by stirring for 1 hour. After the reaction was terminated by adding water to the reaction mixture, the reaction mixture was extracted 3 times with EtOAc. The organic layer was dried over anhydrous magnesium sulfate and concentrated under reduced pressure, and the residue was purified by MPLC to obtain the title compound (300 mg).
LC/MS: 193 (M+H)

### Preparation Example 17: Preparation of 7-chloro-4-((2R,SS)-2,5-dimethylpiperidin-4-yl)-1-methyl-1,4-dihydropyrido[2,3-b]pyrazine-2,3-dione dihydrochloride

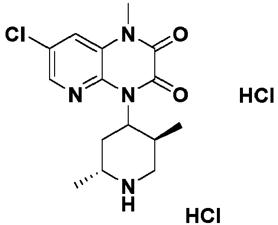

### Step A: Preparation of tert-butyl (2R,5S)-4-((5-chloro-3-nitropyridin-2-yl)amino)-2,5-dimethylpiperidine-1-carboxylate

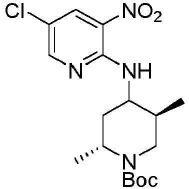

5-Chloro-2-fluoro-3-nitropyridine (306 mg, 1.73 mmol) and tert-butyl (2R,5S)-4-amino-2,5-dimethylpiperidine-1-carboxylate acetate (500 mg, 1.73 mmol) were dissolved in DMF (20 mL), and DIPEA (0.606 mL, 3.47 mmol) was added thereto, followed by stirring for 3 hours. After the reaction was terminated by adding water to the reaction mixture, the reaction mixture was extracted 3 times with EtOAc. The organic layer was dried over anhydrous magnesium sulfate and concentrated under reduced pressure, and the residue was purified by MPLC to obtain the title compound (500 mg, 75%).
¹H-NMR (500 MHz, CHLOROFORM-D) δ 8.53 (d, J = 6.4 Hz, 1H), 8.44 (d, J = 2.0 Hz, 1H), 8.38 (d, J= 1.6 Hz, 1H), 4.59∼4.55 (m, 1H), 4.39∼4.33 (m, 1H), 3.98∼3.95 (m, 1H), 2.87∼2.81 (m, 1H), 2.13∼2.08 (m, 1H), 1.99∼1.96 (m, 2H), 1.50 (s, 9H), 1.22 (d, J = 6.0 Hz, 3H), 0.96 (d, J = 6.4 Hz, 3H)
LC/MS: 329 (M+H-t-Bu)

### Step B: Preparation of tert-butyl (2K5S)-4-((3-amino-5-chloropyridin-2-yl)amino)-2,5-dimethylpiperidine-1-carboxylate

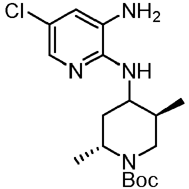

Tert-butyl (2R,5 S)-4-((5-chloro-3 -nitropyridin-2-yl)amino)-2,5-dimethylpiperidine-1-carboxylate (330 mg, 0.857 mmol) was dissolved in MeOH (10 mL), and zinc (1.12 g, 17.2 mmol) and ammonium chloride (0.460 g, 8.57 mmol) were added thereto, followed by stirring at 70°C for 3 hours. After filtering with a Celite pad, the reaction mixture was concentrated under reduced pressure to obtain the title compound (304 mg), which was used directly in the next reaction.
LC/MS: 355 (M+H)

### Step C: Preparation of tert-butyl 2R5S)-4-(7-chloro-1-methyl-2,3-dioxo-2,3-dihydropyrido[2,3-b]pyrazin-4(1H)-yl)-2,5-dimethylpiperidine-1-carboxylate

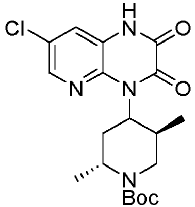

Tert-butyl (2R,5 S)-4-((3-amino-5-chloropyridin-2-yl)amino)-2, 5-dimethylpiperidine-1-carboxylate (304 mg, 0.857 mmol) was dissolved in DCE (10 mL), and ethyl 2-chloro-2-oxoacetate (0.144 mL, 1.29 mmol) and DIPEA (0.449 mL, 2.57 mmol) were added thereto, followed by stirring at room temperature for 12 hours and stirring at 70°C over the weekend. After terminating the reaction by adding water to the reaction mixture, it was extracted 3 times with DCM. The organic layer was dried over anhydrous magnesium sulfate and concentrated under reduced pressure to obtain the title compound (209 mg), which was used directly in the next reaction.
LC/MS: 309 (M+H-t-Bu)

### Step D: Preparation of tert-butyl (2R,5S)-4-(7-chloro-2,3-dioxo-2,3-dihydropyrido[2,3-b]pyrazin-4(1H)-yl)-2,5-dimethylpiperidine-1-carboxylate

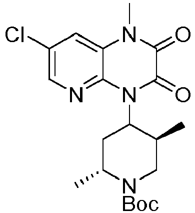

Tert-butyl (2R,5S)-4-(7-chloro-1-methyl-2,3-dioxo-2,3-dihydropyrido[2,3-b]pyrazin-4(1H)-yl)-2,5-dimethylpiperidine-1-carboxylate (209 mg, 0.511 mmol) was used in a similar manner to Step E of Preparation Example 1 to obtain the title compound (81 mg, 38%).
¹H-NMR (500 MHz, CHLOROFORM-D) δ 8.21 (d, J = 2.0 Hz, 1H), 7.49 (d, J = 2.0 Hz, 1H), 5.36∼5.31 (m, 1H), 4.04∼3.93 (m, 2H), 3.64 (s, 3H), 3.07∼2.95 (m, 2H), 2.76∼2.69 (m, 1H), 1.85∼1.80 (m, 1H), 1.52 (s, 9H), 1.25 (d, J = 6.0 Hz, 3H), 0.78 (d, J = 6.4 Hz, 3H)
LC/MS: 367 (M+H-t-Bu), 445 (M+Na)

### Step E: Preparation of 7-chloro-4-((2R,5S)-2,5-dimethylpiperidin-4-yl)-1-methyl-1,4-dihydropyrido[2,3-b]pyrazine-2,3-dione dihydrochloride

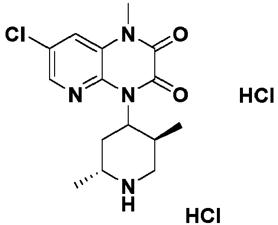

Tert-butyl (2R,5S)-4-(7-chloro-2,3-dioxo-2,3-dihydropyrido[2,3-b]pyrazin-4(1H)-yl)-2,5-dimethylpiperidine-1-carboxylate (81 mg, 0.192 mmol) was used in a manner similar to Step F of Preparation Example 1 to obtain the title compound (68 mg, 99%).
LC/MS: 323 (M+H)

### Preparation Example 18: Preparation of 7-chloro-1,6-dimethyl-4-(piperidin-4-yl)-1,4-dihydropyrido[2,3-b]pyrazine-2,3-dione dihydrochloride

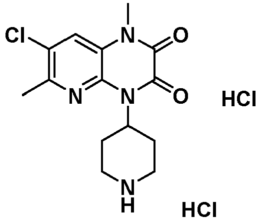

### Step A: Preparation of tert-butyl 4-((5-chloro-6-methyl-3-nitropyridin-2-yl)amino)piperidine-1-carboxylate

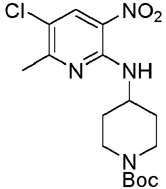

Tert-butyl 4-((6-methyl-3-nitropyridin-2-yl)amino)piperidine-1 -carboxylate (1.15 g, 3.42 mmol) obtained in Step A of Preparation Example 3 was dissolved in CH₃CN (30 mL), and NCS was added thereto, followed by stirring at 60°C for 72 hours. After the reaction mixture was cooled to room temperature, it was concentrated under reduced pressure. The residue was diluted with EtOAc and an aqueous sodium bicarbonate solution, and the organic layer was dried over anhydrous sodium sulfate and concentrated under reduced pressure. The residue was purified by MPLC to obtain the title compound (770 mg, 61%).
¹H-NMR (500 MHz, CHLOROFORM-D) δ 8.38 (s, 1H), 8.14 (d, J = 7.0 Hz, 1H), 4.40∼4.35 (m, 1H), 4.08 (br s, 2H), 3.03 (d, J= 10.5 Hz, 2H), 2.57 (s, 3H), 2.08∼2.06 (m, 2H), 1.55∼1.51 (m, 2H), 1.50 (s, 9H)
LC/MS: 393 (M+H)

### Step B: Preparation of tert-butyl 4-((3-amino-5-chloro-6-methylpyridin-2-yl)amino)piperidine-1-carboxylate

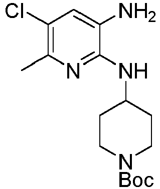

Tert-butyl 4-((5-chloro-6-methyl-3-nitropyridin-2-yl)amino)piperidine-1-carboxylate (310 mg, 0.836 mmol) was used in a similar manner to Step B of Preparation Example 17 to obtain the title compound (271 mg, 95%).
LC/MS: 341 (M+H)

### Step C: Preparation of tert-butyl 4-(7-chloro-6-methyl-2,3-dioxo-2,3-dihydropyrido[2,3-b]pyrazin-4(1H)-yl)piperidine-1-carboxylate

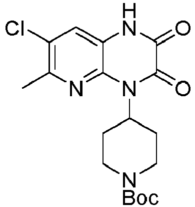

Tert-butyl 4-((3-amino-5-chloro-6-methylpyridin-2-yl)amino)piperidine-1-carboxylate (271 mg, 0.795 mmol) was used in a similar manner to Step C of Preparation Example 17 to obtain the title compound (314 mg, 99%).
LC/MS: 339 (M+H-t-Bu)

### Step D: Preparation of tert-butyl 4-(7-chloro-1,6-dimethyl-2,3-dioxo-2,3-dihydropyrido[2,3-b]pyrazin-4(1H)-yl)piperidine-1-carboxylate

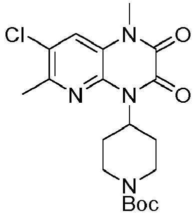

Tert-butyl 4-(7-chloro-6-methyl-2,3-dioxo-2,3-dihydropyrido[2,3-b]pyrazin-4(1H)-yl)piperidine-1-carboxylate (314 mg, 0.795 mmol) was used in a similar manner to Step E of Preparation Example 1 to obtain the title compound (271 mg, 83%).
¹H-NMR (500 MHz, CHLOROFORM-D) δ 7.46 (s, 1H), 5.50 (s, 1H), 4.35∼4.24 (m, 2H), 3.61 (s, 3H), 2.87∼2.85 (m, 4H), 2.61 (s, 3H), 1.65 (br s, 2H), 1.52 (s, 9H)
LC/MS: 431 (M+Na), 353 (M+H-t-Bu)

### Step E: Preparation of 7-chloro-1,6-dimethyl-4-(piperidin-4-yl)-L4-dihydropyrido[2,3-b]pyrazine-2,3-dione dihydrochloride

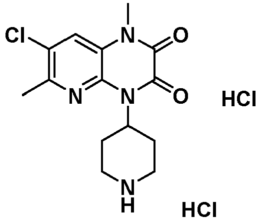

Tert-butyl 4-(7-chloro-1,6-dimethyl-2,3-dioxo-2,3-dihydropyrido[2,3-b]pyrazin-4(1H)-yl)piperidine-1-carboxylate (271 mg, 0.663 mmol) was used in a similar manner to Step F of Preparation Example 1 to obtain the title compound (221 mg, 97%).
LC/MS: 309 (M+H)

### Preparation Example 19: Preparation of 7-bromo-1,6-dimethyl-4-(piperidin-4-yl)-1,4-dihydropyrido[2,3-b]pyrazine-2,3-dione dihydrochloride

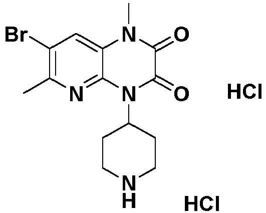

### Step A: Preparation of tert-butyl 4-((5-bromo-6-methyl-3-nitropyridin-2-yl)amino)piperidine-1-carboxylate

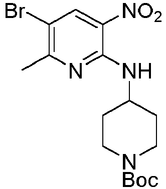

Tert-butyl 4-((6-methyl-3-nitropyridin-2-yl)amino)piperidine-1-carboxylate (4.90 g, 14.57 mmol) obtained in Step A of Preparation Example 3 was dissolved in CH₃CN (146 mL), and NBS was added thereto, followed by stirring at room temperature for 18 hours. After the reaction mixture was cooled to room temperature, it was concentrated under reduced pressure. The residue was diluted with EtOAc and an aqueous sodium bicarbonate solution, and the organic layer was dried over anhydrous sodium sulfate and concentrated under reduced pressure. The residue was purified by MPLC to obtain the title compound (6.05 g, 92%).
¹H-NMR (500 MHz, CHLOROFORM-D) δ 8.52 (s, 1H), 8.12 (d, J = 7.3 Hz, 1H), 4.37 (m, 1H), 4.08 (br s, 2H), 3.03 (t, J = 11.9 Hz, 2H), 2.61 (s, 3H), 2.08-2.06 (m, 2H), 1.58 (s, 3H), 1.53 (dd, J = 12.8, 4.0 Hz, 2H), 1.50 (s, 9H)
LC/MS: 437 (M+Na)

### Step B: Preparation of tert-butyl 4-((3-amino-5-bromo-6-methylpyridin-2-yl)amino)piperidine-1-carboxylate

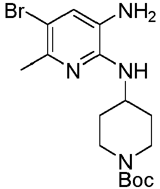

Tert-butyl 4-((5-bromo-6-methyl-3-nitropyridin-2-yl)amino)piperidine-1-carboxylate (1,000 mg, 2.41 mmol) was used in a similar manner to Step B of Preparation Example 17 to obtain the title compound (811 mg, 87%).
LC/MS: 385, 387 (M+H)

### Step C: Preparation of tert-butyl 4-(7-bromo-6-methyl-2,3-dioxo-2,3-dihydropyrido[2,3-b]pyrazin-4(1H)-yl)piperidine-1-carboxylate

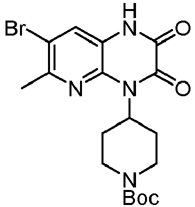

Tert-butyl 4-((3-amino-5-bromo-6-methylpyridin-2-yl)amino)piperidine-1-carboxylate (811 mg, 2.11 mmol) was used in a similar manner to Step C of Preparation Example 17 to obtain the title compound (925 mg, 99%).
LC/MS: 439, 441 (M+H)

### Step D: Preparation of tert-butyl 4-(7-bromo-1,6-dimethyl-2,3-dioxo-2,3-dihydropyrido[2,3-b]pyrazin-4(1H)-yl)piperidine-1-carboxylate

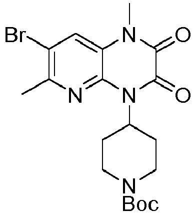

Tert-butyl 4-(7-bromo-6-methyl-2,3-dioxo-2,3-dihydropyrido[2,3-b]pyrazin-4(1H)-yl)piperidine-1-carboxylate (925 mg, 2.11 mmol) was used in a similar manner to Step E of Preparation Example 1 to obtain the title compound (732 mg, 77%).
¹H-NMR (500 MHz, CHLOROFORM-D) δ 7.61 (s, 1H), 5.49 (s, 1H), 4.35∼4.24 (m, 2H), 3.61 (s, 3H), 2.87∼2.85 (m, 4H), 2.65 (s, 3H), 1.64 (br s, 2H), 1.52 (s, 9H)
LC/MS: 475, 477 (M+Na)

### Step E: Preparation of 7-bromo-L6-dimethyl-4-(piperidin-4-yl)-L4-dihydropyrido[2,3-b]pyrazine-2,3-dione dihydrochloride

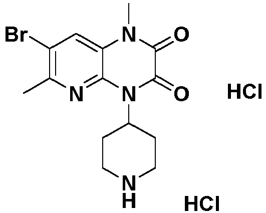

Tert-butyl 4-(7-bromo-1,6-dimethyl-2,3-dioxo-2,3-dihydropyrido[2,3-b]pyrazin-4(1H)-yl)piperidine-1-carboxylate (732 mg, 1.62 mmol) was used in a similar manner to Step F of Preparation Example 1 to obtain the title compound (631 mg, 100%).
LC/MS: 353, 355 (M+H)

### Preparation Example 20: Preparation of 6-acetyl-1-methyl-4-(piperidin-4-yl)-1,4-dihydropyrido[2,3-b]pyrazine-2,3-dione hydrochloride

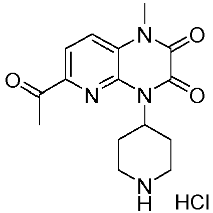

### Step A: Preparation of tert-butyl 4-((3-amino-6-chloropyridin-2-yl)amino)piperidine-1 -carboxylate

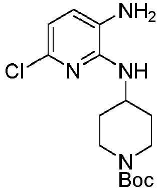

Tert-butyl 4-((6-chloro-3-nitropyridin-2-yl)amino)piperidine-1-carboxylate (7.86 g, 22.03 mmol) obtained in Step A of Preparation Example 4 was used in a similar manner to Step B of Preparation Example 17 to obtain the title compound (4.4 g, 61%).
LC/MS: 271 (M+H-t-Bu), 349 (M+Na)

### Step B: Preparation of tert-butyl 4-(6-chloro-2,3-dioxo-2,3-dihydropyrido[2,3-b]pyrazin-4(1H)-yl)pipendine-1-carboxylates

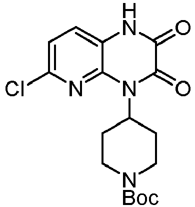

Tert-butyl 4-((3-amino-6-chloropyridin-2-yl)amino)piperidine-1-carboxylate (4.4 g, 13.46 mmol) was used in a similar manner to Step C of Preparation 17 to obtain the title compound (5.13 g), which was directly used in the next reaction.
LC/MS: 281 (M+H-t-Bu)

### Step C: Preparation of tert-butyl 4-(6-chloro-1-methyl-23-dioxo-23-dihydropyrido[2,3-b]pyrazin-4(1H)-yl)piperidine-1-carboxylate

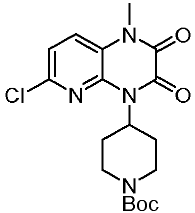

Tert-butyl 4-(6-chloro-2,3-dioxo-2,3-dihydropyrido[2,3-b]pyrazin-4(1H)-yl)piperidine-1-carboxylate (5.13 g, 13.47 mmol) was used in a similar manner to Step E of Preparation Example 1 to obtain the title compound (3.91 g, 74%).
¹H-NMR (500 MHz, CHLOROFORM-D) δ 7.47 (d, J = 8.5 Hz, 1H), 7.23 (d, J = 8.0 Hz, 1H), 5.47∼5.42 (m, 1H), 4.36∼4.26 (m, 2H), 3.64 (s, 3H), 2.91∼2.84 (m, 4H), 1.66∼1.65 (m, 2H), 1.52 (s, 9H)
LCMS: 417 (M+Na)

### Step D: Preparation of tert-butyl 4-(6-(1-ethoxyvinyl)-1-methyl-2,3-dioxo-2,3-dihydropyrido[2,3-b]pyrazine-4(1H)-yl)piperidine-1-carboxylate

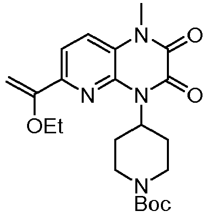

Tert-butyl 4-(6-chloro-1-methyl-2,3-dioxo-2,3-dihydropyrido[2,3-b]pyrazin-4(1H)-yl)piperidin-1-carboxylate (150 mg, 0.38 mmol) was used in the same manner as in Step A of Preparation Example 9 to obtain the title compound (143 mg, 87%).
¹H-NMR (500 MHz, CHLOROFORM-D) δ 7.62 (d, J = 8.0 Hz, 1H), 7.48 (d, J = 8.5 Hz, 1H), 5.52 (br s, 1H), 5.32 (d, J = 1.5 Hz, 1H), 4.41∼4.34 (m, 3H), 4.01 (q, J = 7.0 Hz, 2H), 3.65 (s, 3H), 3.00∼2.93 (m, 4H), 1.71∼1.69 (m, 2H), 1.52 (s, 9H)
LC/MS: 375 (M+H-t-Bu), 453 (M+Na)

### Step E: Preparation of 6-acetyl-1-methyl-4-(piperidin-4-yl)-L4-dihydropyrido[2,3-b]pyrazine-2,3-dione hydrochloride

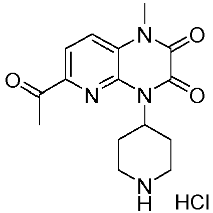

Tert-butyl 4-(6-(1-ethoxyvinyl)-1-methyl-2,3-dioxo-2,3-dihydropyrido[2,3-b]pyrazin-4(1H)-yl)piperidine-1-carboxylate (143 mg, 0.332 mmol) was used in the same manner as in Step F of Preparation Example 1 to obtain the title compound (113 mg), which was used directly in the next reaction without purification.
LC/MS: 303 (M+H)

### Preparation Example 21: Preparation of 6-chloro-1-methyl-4-(piperidin-4-yl)-1,4-dihydropyrido[2,3-b]pyrazine-2,3-dione hydrochloride

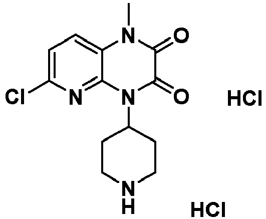

Tert-butyl 4-((6-methyl-3-nitropyridin-2-yl)amino)piperidine-1-carboxylate (500 mg, 1.27 mmol) obtained in Step C of Preparation Example 20 was used in the same manner as in Step F of Preparation Example 1 to obtain the title compound (450 mg, 99%), which was used directly in the next reaction without purification.
LC/MS: 337 (M+H)

### Preparation Example 22: Preparation of 6-methoxy-1-methyl-4-(piperidin-4-yl)-1,4-dihydropyrido[2,3-b]pyrazine-2,3-dione dihydrochloride

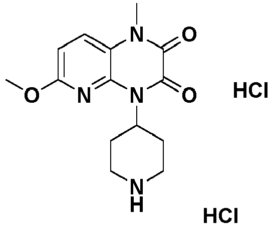

### Step A: Preparation of tert-butyl 4-((6-methoxy-3-nitropyridin-2-yl)amino)piperidine-1-carboxylate

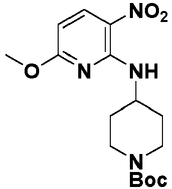

2-Chloro-6-methoxy-3-nitropyridine (1.00 g, 5.30 mmol) was used in the same manner as in Step A of Preparation Example 4 to obtain the title compound (1.80 g, 96%). ¹H-NMR (500 MHz, CHLOROFORM-D) δ 8.66 (d, J = 7.0 Hz, 1H), 8.33 (d, J = 9.2 Hz, 1H), 6.09 (d, J = 9.2 Hz, 1H), 4.33-4.26 (m, 1H), 4.07 (br s, 2H), 3.97 (s, 3H), 3.04 (t, J = 11.3 Hz, 2H), 2.09 (d, J = 10.1 Hz, 2H), 1.62-1.57 (m, 2H), 1.50 (s, 9H) LC/MS: 375 (M+Na)

### Step B: Preparation of tert-butyl 4-((3-amino-6-methoxypyridin-2-yl)amino)piperidine-1-carboxylate

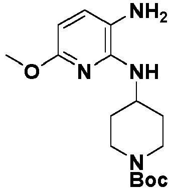

Tert-butyl 4-((6-methoxy-3-nitropyridin-2-yl)amino)piperidine-1-carboxylate (1.80 g, 5.11 mmol) was used in a similar manner to Step B of Preparation Example 17 to obtain the title compound (1.65 g, 73%), which was used directly in the next reaction without purification.
LC/MS: 345 (M+Na)

### Step C: Preparation of tert-butyl 4-(6-methoxy-2,3-dioxo-2,3-dihydropyrido[2,3-b]pyrazin-4(1H)-yl)piperidine-1-carboxylate

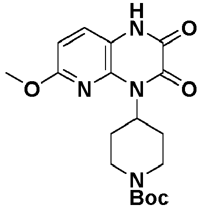

Tert-butyl 4-((3-amino-6-methoxypyridin-2-yl)amino)piperidine-1-carboxylate (1.21 g, 3.72 mmol) was used in a similar manner to Step C of Preparation Example 17 to obtain the title compound (750 mg, 54%), which was used directly in the next reaction.
LC/MS: 399 (M+Na)

### Step D: Preparation of tert-butyl 4-(6-methoxy-1-methyl-2,3-dioxo-2,3-dihydropyrido[2,3-b]pyrazin-4(1H)-yl)piperidine-1-carboxylate

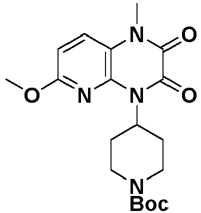

Tert-butyl 4-(6-methoxy-2,3-dioxo-2,3-dihydropyrido[2,3-b]pyrazin-4(1H)-yl)piperidine-1-carboxylate (750 mg, 3.72 mmol) was used in a similar manner to Step E of Preparation Example 1 to obtain the title compound (600 mg, 77%).
¹H-NMR (500 MHz, CHLOROFORM-D) δ 7.50 (d, J = 8.9 Hz, 1H), 6.68 (d, J = 8.9 Hz, 1H), 5.49-5.44 (m, 1H), 4.33 (d, J = 61.0 Hz, 2H), 3.94 (s, 3H), 3.63 (s, 3H), 2.99-2.82 (m, 4H), 1.71 (d, J = 10.1 Hz, 2H), 1.49 (s, 9H)
LC/MS: 335 (M+Na-tBu), 413 (M+Na)

### Step E: Preparation of 6-methoxy-1-methyl-4-(piperidin-4-yl)-L4-dihydropyrido[2,3-b]pyrazine-2,3-dione dihydrochloride

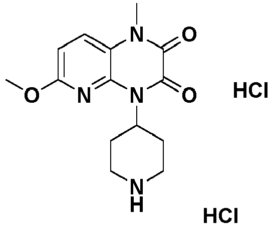

Tert-butyl 4-(6-methoxy-1-methyl-2,3-dioxo-2,3-dihydropyrido[2,3-b]pyrazin-4(1H)-yl)piperidine-1-carboxylate (600 mg, 1.54 mmol) was used in a similar manner to Step F of Preparation Example 1 to obtain the title compound (550 mg, 90%), which was used directly in the next reaction.
LC/MS: 291 (M+H)

### Preparation Example 23: Preparation of 1,8-dimethyl-4-(piperidin-4-yl)-1,4-dihydropyrido[2,3-b]pyrazine-2,3-dione hydrochloride

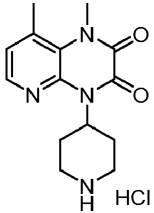

### Step A: Preparation of tert-butyl 4-((4-methyl-3-nitropyridin-2-yl)amino)piperidine-1-carboxylate

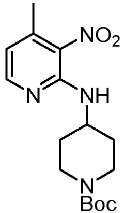

2-Fluoro-4-methyl-3-nitropyridine (1.56 g, 9.99 mmol) was used in the same manner as in Step B of Preparation Example 4 to obtain the title compound (3.32 g, 99%).
¹H-NMR (500 MHz, CHLOROFORM-D) δ 8.14 (d, J = 5.0 Hz, 1H), 7.45 (d, J = 7.0 Hz, 1H), 6.50 (d, J= 5.0 Hz, 1H), 4.31∼4.28 (m, 1H), 4.06 (br s, 2H), 3.04∼2.99 (m, 2H), 2.56 (s, 3H), 2.08~2.06 (m, 2H), 1.52 (s, 9H), 1.51∼1.44 (m, 2H)
LC/MS: 337 (M+H)

### Step B: Preparation of tert-butyl 4-((3-amino-4-methylpyridin-2-yl)amino)piperidine-1-carboxylate

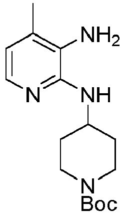

Tert-butyl 4-((4-methyl-3-nitropyridin-2-yl)amino)piperidine-1-carboxylate (1.5 g, 4.46 mmol) was used in a similar manner to Step B of Preparation Example 17 to obtain the title compound (1.35 g, 99%), which was used directly in the next reaction without purification.
LC/MS: 307 (M+H)

### Step C: Preparation of tert-butyl 4-((3-(2-ethoxy-2-oxoacetamido)-4-methylpyridin-2-yl)amino)piperidine-1-carboxylate

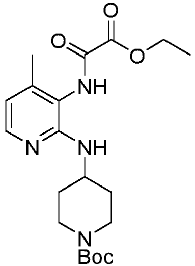

Tert-butyl 4-((3-amino-4-methylpyridin-2-yl)amino)piperidine-1-carboxylate (1.35 g, 4.41 mmol) was used in a similar manner to Step C of Preparation Example 1 to obtain the title compound (1.67 g, 93%).
LC/MS: 407 (M+H)

### Step D: Preparation of tert-butyl 4-(8-methyl-2,3-dioxo-2,3-dihydropyrido[2,3-b]pyrazin-4(1H)-yl)piperidine-1-carboxylate

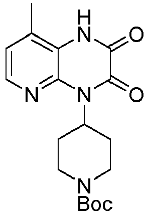

Tert-butyl 4-((3-(2-ethoxy-2-oxoacetamido)-4-methylpyridin-2-yl)amino)piperidine-1-carboxylate (930 mg, 2.29 mmol) and DIPEA(2.0 mL, 11.4 mmol) were dissolved in DCE (15 mL) and stirred at 70°C for 24 hours. The reaction mixture was cooled to room temperature, concentrated under reduced pressure and purified by MPLC to obtain the title compound (34 mg, 4%).
LC/MS: 305 (M+H-t-Bu)

### Step E: Preparation of tert-butyl 4-(1,8-dimethyl-2,3-dioxo-2,3-dihydropyrido[2,3-b]pyrazin-4(1H)-yl)piperidine-1-carboxylate

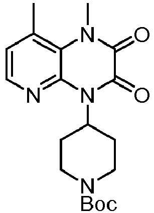

Tert-butyl 4-(8-methyl-2,3-dioxo-2,3-dihydropyrido[2,3-b]pyrazin-4(1H)-yl)piperidine-1-carboxylate (34 mg, 0.094 mmol) was used in a similar manner to Step E of Preparation Example 1 to obtain the title compound (35 mg, 99%).
¹H-NMR (500 MHz, CHLOROFORM-D) δ 8.24 (d, J = 5.0 Hz, 1H), 7.12 (d, J = 5.0 Hz, 1H), 5.74 (br s, 1H), 4.31∼4.22 (m, 2H), 4.13 (s, 3H), 2.96∼2.94 (m, 4H), 2.61 (s, 3H), 1.65∼1.62 (m, 2H), 1.52 (s, 9H)
LC/MS: 397 (M+Na)

### Step F: Preparation of 1,8-dimethyl-4-(piperidin-4-yl)-1,4-dihydropyrido[2,3-b]pyrazine-2,3-dione hydrochloride

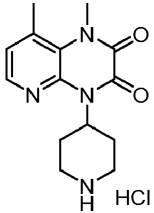

Tert-butyl 4-(1,8-dimethyl-2,3-dioxo-2,3-dihydropyrido[2,3-b]pyrazin-4(1H)-yl)piperidine-1-carboxylate (35 mg, 0.093 mmol) was used in a similar manner to Step F of Preparation Example 1 to obtain the title compound (29.2 mg, 100%).
LC/MS: 275 (M+H)

### Preparation Example 24: Preparation of 4-((1R,3r,5S)-8-azabicyclo[3.2.1]octan-3-yl)-7-chloro-1-methyl-1,4-dihydropyrido[2,3-b]pyrazine-2,3-dione dihydrochloride

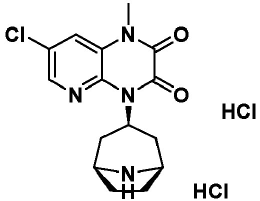

### Step A: Preparation of tert-butyl (1R,3r,5S)-3-((5-chloro-3-nitropyridin-2-yl)amino)-8-azabicyclol3.2.11octane-8-carboxylate

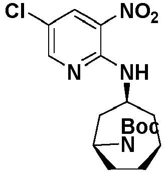

5-Chloro-2-fluoro-3-nitropyridine (1.00 g, 5.66 mmol) and tert-butyl 3-amino-8-azabicyclo[3.2.1]octane-8-carboxylate (1.41 g, 6.23 mmol) were used in the same manner as in Step A of Preparation Example 4 to obtain the title compound (2.11 g, 97%).
¹H-NMR (400 MHz, CHLOROFORM-D) δ 8.38 (d, J = 2.7 Hz, 1H), 8.32 (d, J = 2.3 Hz, 1H), 7.93 (d, J = 7.8 Hz, 1H), 4.74-4.69 (m, 1H), 4.31 (br s, 2H), 2.04-2.01 (m, 4H), 1.80 (q, J = 7.0 Hz, 2H), 1.71-1.60 (m, 2H), 1.48 (s, 9H)
LC/MS: 383 (M+H)

### Step B: Preparation of tert-butyl (1R,3r,5S)-3-((3-amino-5-chloropyridin-2-yl)amino)-8-azabicyclol3.2.11octane-8-carboxylate

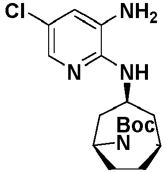

Tert-butyl (1R,3r,5S)-3-((5-chloro-3-nitropyridin-2-yl)amino)-8-azabicyclo[3.2.1]octane-8-carboxylate (2.11 g, 5.49 mmol) was used in a similar manner to Step B of Preparation Example 17 to obtain the title compound (1.88 g, 97%), which was used directly in the next reaction without purification.
LC/MS: 375 (M+Na)

### Step C: Preparation of tert-butyl (1R,3r,5S)-3-(7-chloro-1-methyl-2,3-dioxo-2,3-dihydropyrido[2,3-b]pyrazin-4(1H)-yl)-8-azabicyclo[3.2.1]octane-8-carboxylate

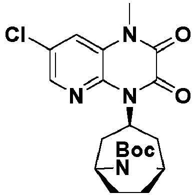

Tert-butyl (1R,3r,5S)-3-((3-amino-5-chloropyridin-2-yl)amino)-8-azabicyclo[3.2.1]octane-8-carboxylate (1.88 g, 5.33 mmol) and DIPEA (5.58 mL, 32.00 mmol) were dissolved in DCM (300 mL), and ethyl 2-chloro-2-oxoacetate (1.79 mL, 15.98 mmol) was slowly added thereto while maintaining at 0°C. After heating to 50°C, the reaction mixture was stirred for 18 hours. After completion of the reaction, the reaction mixture was extracted with a saturated aqueous ammonium chloride solution, and the organic layer was dried over magnesium sulfate and distilled under reduced pressure to obtain a concentrated mixture. The obtained mixture was used in the same manner as in Step E of Preparation Example 1 to obtain the title compound (1.50 g, 67%).
¹H-NMR (500 MHz, DMSO-D6) δ 8.20 (s, 1H), 7.95 (d, J = 1.8 Hz, 1H), 5.83 (br s, 1H), 4.16 (br s, 2H), 3.48 (s, 3H), 2.81-2.72 (m, 2H), 2.01-1.98 (m, 2H), 1.81-1.68 (m, 2H), 1.49 (s, 9H)
LC/MS: 421 (M+H)

### Step D: Preparation of 4-((1R,3r,5S)-8-azabicyclo[3.2.1]octan-3-yl)-7-chloro-1-methyl-1,4-dihydropyrido[2,3-b]pyrazine-2,3-dionehydrochloride

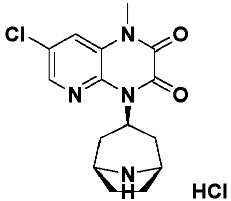

Tert-butyl (1R,3r,5S)-3-(7-chloro-1-methyl-2,3-dioxo-2,3-dihydropyrido[2,3-b]pyrazin-4(1H)-yl)-8-azabicyclo[3.2.1]octane-8-carboxylate (1.50 g, 3.56 mmol) was used in a similar manner to Step F of Preparation Example 1 to obtain the title compound (1.25 g, 98%), which was used directly in the next reaction.
LC/MS: 321 (M+H)

### Preparation Example 25: Preparation of 6-isopropoxy-1-methyl-4- (piperidin-4-yl) -1,4-dihydropyrido[2,3-b]pyrazine-2,3-dione dihydrochloride

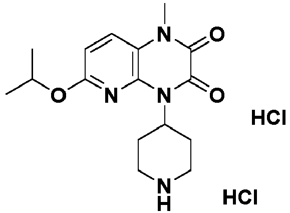

### Step A: Preparation of tert-butyl 4-((6-isopropoxy-3-nitropyridin-2-yl)amino)piperidine-1-carboxylate

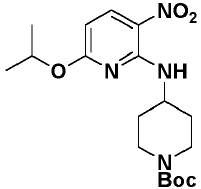

2,6-Difluoro-3-nitropyridine (2 g, 12.49 mmol) and tert-butyl 4-aminopiperidine-1-carboxylate (2.502 g, 12.49 mmol) were dissolved in isopropyl alcohol, and DIPEA (4.36 ml, 24.99 mmol) was added thereto, followed by stirring at 70°C for 4 hours. The reaction mixture was cooled to room temperature, concentrated under reduced pressure and purified by MPLC to obtain the title compound (3.775 g).
¹H-NMR (400 MHz, CHLOROFORM-D) δ 8.28 (d, J = 9.1 Hz, 1H), 6.00 (d, J = 9.1 Hz, 1H), 5.25 (t, J = 6.2 Hz, 1H), 3.85-4.45 (4H), 3.01 (s, 2H), 2.03 (q, J = 4.1 Hz, 2H), 1.46 (s, 9H), 1.35 (d, 6H)
LC/MS: 381 (M+H)

### Step B: Preparation of tert-butyl 4-((3-amino-6-isopropoxypyridin-2-yl)amino)piperidine-1-carboxylate

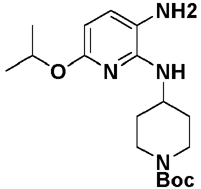

Tert-butyl 4-((6-isopropoxy-3-nitropyridin-2-yl)amino)piperidine-1-carboxylate (3.755 g, 9.87 mmol) was used in a similar manner to Step B of Preparation Example 17 to obtain the title compound (1.07 g).
¹H-NMR (400 MHz, CHLOROFORM-D) δ 6.87 (d, J = 7.8 Hz, 1H), 5.87 (d, J = 8.2 Hz, 1H), 5.07-5.00 (m, 1H), 4.29 (s, 1H), 4.00 (d, J = 10.5 Hz, 3H), 2.94 (t, J= 11.4 Hz, 2H), 2.74 (s, 1H), 2.06 (q, J= 4.1 Hz, 2H), 1.46 (s, 9H), 1.38 (d, J = 9.1 Hz, 2H), 1.34-1.30 (m, 6H)
LC/MS: 351 (M+H)

### Step C: Preparation of tert-butyl 4-(6-isopropoxy-2,3-dioxo-2,3-dihydropyrido[2,3-b]pyrazin-4(1H)-yl)piperidine-1-carboxylate

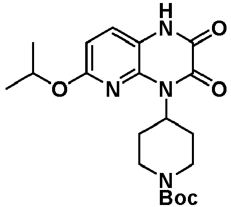

Tert-butyl 4-((3-amino-6-isopropoxypyridin-2-yl)amino)piperidine-1-carboxylate (1.07 g, 3.05 mmol) was used in a similar manner to Step C of Preparation Example 17 to obtain the title compound (1.2 g).
LC/MS: 405 (M+H)

### Step D: Preparation of tert-butyl 6-isopropoxy-1-methyl-2,3-dioxo-2,3-dihydropyrido[2,3-b]pyrazin-4(1H)-yl)piperidine-1-carboxylate

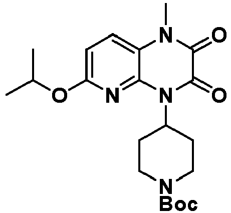

Tert-butyl 4-(6-isopropoxy-2,3-dioxo-2,3-dihydropyrido[2,3-b]pyrazin-4(1H)-yl)piperidine-1-carboxylate (1.256 g, 3.11 mmol) was used in a similar manner to Step D of Preparation Example 17 to obtain the title compound (870 mg).
¹H-NMR (500 MHz, CHLOROFORM-D) δ 7.48 (d, J = 8.9 Hz, 1H), 6.64-6.62 (m, 1H), 5.43 (s, 1H), 5.17-5.13 (m, 1H), 4.29 (d, J = 29.3 Hz, 2H), 3.62 (s, 3H), 2.89 (d, J = 11.0 Hz, 4H), 1.67 (d, J = 20.8 Hz, 2H), 1.51 (s, 9H), 1.40 (d, J = 6.1 Hz, 6H)
LC/MS: 419 (M+H)

### Step E: Preparation of 6-isopropoxy-1-methyl-4-(piperidin-4-yl)-L4-dihydropyrido[2,3-b]pyrazine-2,3-dione dihydrochloride

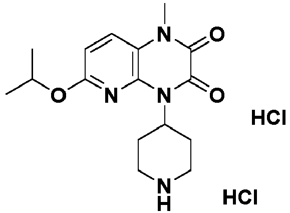

Tert-butyl 4-(6-isopropoxy-1-methyl-2,3-dioxo-2,3-dihydropyrido[2,3-b]pyrazin-4(1H)-yl)piperidine-1-carboxylate (870 mg, 2.079 mmol) was used in a similar manner to Step E of Preparation Example 17 to obtain the title compound (668 mg).
¹H-NMR (400 MHz, CHLOROFORM-D) δ 7.44 (d, J = 8.7 Hz, 1H), 6.59 (dd, J = 8.7, 0.9 Hz, 1H), 5.41 (d, J = 7.8 Hz, 1H), 5.18 (q, J = 6.1 Hz, 1H), 3.69 (d, J = 0.9 Hz, 2H), 3.59 (s, 3H), 3.27 (d, J = 10.1 Hz, 2H), 2.80-2.70 (m, 4H), 1.67 (d, J = 9.6 Hz, 2H), 1.40-1.39 (m, 6H)
LC/MS: 319 (M+H)

### Preparation Example 26: Preparation of 7-fluoro-1-methyl-4-(piperidin-4-yl)-1,4-dihydropyrido[2,3-b]pyrazine-2,3-dione dihydrochloride

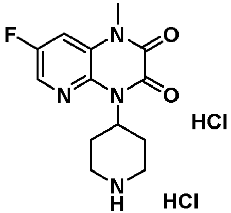

### Step A: Preparation of tert-butyl 4-((5-fluoro-3-nitropyridin-2-yl)amino)piperidine-1-carboxylate

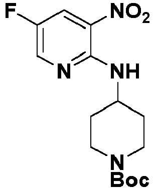

2,6-Difluoro-3-nitropyridine (2 g, 12.49 mmol) and tert-butyl 4-aminopiperidine-1-carboxylate (2.502 g, 12.49 mmol) were used in a similar manner to Step A of Preparation Example 25 to obtain the title compound (4.2 g).
¹H-NMR (400 MHz, CHLOROFORM-D) δ 8.35 (d, J = 3.2 Hz, 1H), 8.18 (dd, J = 8.0, 3.0 Hz, 1H), 8.02 (d, J = 6.9 Hz, 1H), 4.31-4.27 (m, 1H), 4.05 (d, J = 11.0 Hz, 2H), 2.98 (t, J = 11.9 Hz, 2H), 2.04 (d, J = 10.5 Hz, 2H), 1.52-1.49 (m, 2H), 1.46 (s, 9H)
LC/MS: 341 (M+H)

### Step B: Preparation of tert-butyl 4-((3-amino-5-fluoropyridin-2-yl)amino)piperidine-1-carboxylate

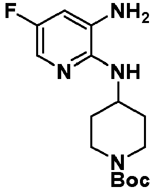

Tert-butyl 4-((5-fluoro-3-nitropyridin-2-yl)amino)piperidine-1-carboxylate (4.2 g, 12.34 mmol) was used in a similar manner to Step B of Preparation Example 17 to obtain the title compound (2.67 g).
¹H-NMR (400 MHz, CHLOROFORM-D) δ 7.56 (d, J = 2.7 Hz, 1H), 6.67 (dd, J = 8.9, 2.5 Hz, 1H), 4.00 (d, J = 11.9 Hz, 2H), 3.70 (s, 1H), 3.32 (s, 2H), 2.94 (t, J = 12.1 Hz, 2H), 2.08-2.03 (m, 2H), 1.45 (s, 9H), 1.35-1.29 (m, 2H)
LC/MS: 311 (M+H)

### Step C: Preparation of tert-butyl 4-(7-fluoro-2,3-dioxo-2,3-dihydropyrido[2,3-b]pyrazin-4(1H)-yl)piperidine-1-carboxylate

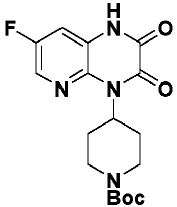

Tert-butyl 4-((3-amino-5-fluoropyridin-2-yl)amino)piperidine-1-carboxylate (2.67 g, 8.60 mmol) was used in a similar manner to Step C of Preparation Example 17 to obtain the title compound (3.0 g).
LC/MS: 365 (M+H)

### Step D: Preparation of tert-butyl 4-(7-fluoro-1-methyl-2,3-dioxo-2,3-dihydropyrido[2,3-b]pyrazin-4(1H)-yl)piperidine-1-carboxylate

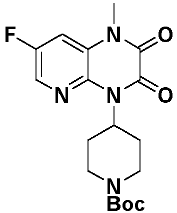

Tert-butyl 4-(7-fluoro-2,3-dioxo-2,3-dihydropyrido[2,3-b]pyrazin-4(1H)-yl)piperidine-1-carboxylate (3 g, 8.23 mmol) was used in a similar manner to Step D of Preparation Example 17 to obtain the title compound (1.92 g).
¹H-NMR (400 MHz, CHLOROFORM-D) δ 8.09 (d, J = 2.7 Hz, 1H), 7.26 (d, J = 2.7 Hz, 1H), 5.46 (s, 1H), 4.28 (s, 2H), 3.59 (s, 3H), 2.88-2.80 (m, 4H), 1.62 (s, 2H), 1.47 (d, J = 15.1 Hz, 9H)
LC/MS: 379 (M+H)

### Step E: Preparation of 7-fluoro-1-methyl-4-(piperidin-4-yl)-L4-dihydropyrido[2,3-b]pyrazine-2,3-dione dihydrochloride

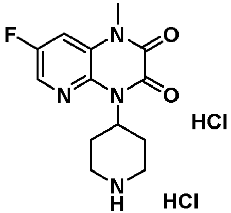

Tert-Butyl 4-(7-fluoro-1-methyl-2,3-dioxo-2,3-dihydropyrido[2,3-b]pyrazin-4(1H)-yl)piperidine-1-carboxylate (1.915 g, 5.06 mmol) was used in a similar manner to Step E of Preparation Example 17 to obtain the title compound (1.27 g).
LC/MS: 279 (M+H)

### Preparation Example 27: Preparation of methyl 1-methyl-2,3-dioxo-4-(piperidin-4-yl)-1,2,3,4-tetrahydropyrido[2,3-b]pyrazine-7-carboxylate dihydrochloride

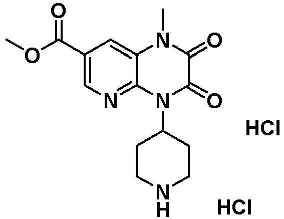

### Step A: Preparation of methyl 6-((1-(tert-butoxycarbonyl)piperidin-4-yl)amino)-5-nitronicotinate

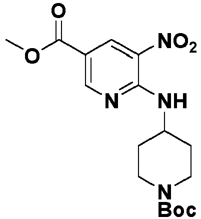

Methyl 6-chloro-5-nicotinate (10.00 g, 46.20 mmol) was used in the same manner as in Step A of Preparation Example 4 to obtain the title compound (17.56 g, 98%).
¹H-NMR (500 MHz, CHLOROFORM-D) δ 9.01 (d, J = 10.1 Hz, 2H), 8.47 (d, J = 7.0 Hz, 1H), 4.47 (br s, 1H), 4.12 (br s, 2H), 3.95 (s, 3H), 3.03 (t, J = 11.1 Hz, 2H), 2.10 (d, J = 12.2 Hz, 2H), 1.61-1.51 (m, 2H), 1.50 (s, 9H)
LC/MS: 325 (M-tBu+2H), 403.2 (M+Na)

### Step B: Preparation of methyl 5-amino-6-((1-(tert-butoxycarbonyl)piperidin-4-yl)amino)nicotinate

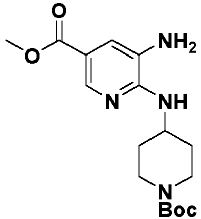

Methyl 6-((1-(tert-butoxycarbonyl)piperidin-4-yl)amino)-5-nitronicotinate (17.20 g, 45.20 mmol) was used in a similar manner to Step B of Preparation Example 17 to obtain the title compound (15.84 g, 99%), which was used directly in the next reaction without purification.
LC/MS: 351 (M+H)

### Step C: Preparation of methyl 4-(1-(tert-butoxycarbonyl)piperidin-4-yl)-23-dioxo-1,2,3,4-tetrahydropyrido[2,3-b]pyrazine-7-carboxylate

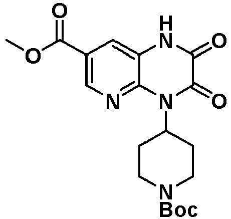

Methyl 5-amino-6-((1-(tert-butoxycarbonyl)piperidin-4-yl)amino)nicotinate (15.84 g, 45.20 mmol) was used in a similar manner to Step C of Preparation Example 17 to obtain the title compound (8.50 g, 46%), which was used directly in the next reaction.
LC/MS: 427 (M+Na)

### Step D: Preparation of methyl 4-(1-(tert-butoxycarbonyl)piperidin-4-yl)-1-methyl-2,3-dioxo-L2,3,4-tetrahydropyridor2,3-b]pyrazine-7-carboxylate

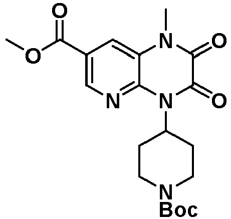

Methyl 4-(1-(tert-butoxycarbonyl)piperidin-4-yl)-2,3-dioxo-1,2,3,4-tetrahydropyrido[2,3-b]pyrazine-7-carboxylate (8.50 g, 21.02 mmol) was used in a similar manner to Step E of Preparation Example 1 to obtain the title compound (4.51 g, 51%).
¹H-NMR (400 MHz, CHLOROFORM-D) δ 8.82 (d, J = 1.8 Hz, 1H), 8.05 (d, J = 1.8 Hz, 1H), 5.55 (br s, 1H), 4.27 (br s, 2H), 3.98 (s, 3H), 3.66 (s, 3H), 2.86-2.81 (m, 4H), 1.67-1.59 (m, 2H), 1.49 (s, 9H)
LC/MS: 431 (M+Na)

### Step E: Preparation of methyl 1-methyl-2,3-dioxo-4-(piperidin-4-yl)-1,2,3,4-tetrahydropyrido[2,3-b]pyrazine-7-carboxylate dihydrochloride

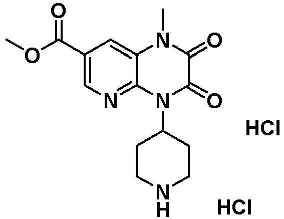

Methyl 4-(1-(tert-butoxycarbonyl)piperidin-4-yl)-1-methyl-2,3-dioxo-1,2,3,4-tetrahydropyrido[2,3-b]pyrazine-7-carboxylate (4.50 g, 10.75 mmol) was used in a similar manner to Step F of Preparation Example 1 to obtain the title compound (35 mg, 99%), which was used directly in the next reaction.
LC/MS: 319 (M+H)

### Preparation Example 28: Preparation of 1,7-dimethyl-4-(piperidin-4-yl)-1,4-dihydropyrido[2,3-b]pyrazine-2,3-dione dihydrochloride

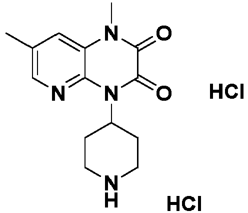

### Step A: Preparation of tert-butyl 4-((5-methyl-3-nitropyridin-2-yl)amino)piperidine-1-carboxylate

2-Chloro-5-methyl-3-nitropyridine (10.00 g, 57.90 mmol) and tert-butyl 4-aminopiperidine-1-carboxylate (12.19 g, 60.80 mmol) were dissolved in DMF (300 mL), and triethylamine (12.12 mL) was added thereto, followed by stirring at 100°C for 20 hours. After the reaction mixture was cooled to room temperature, it was concentrated by distillation under reduced pressure. The concentrated mixture was dissolved in diethyl ether and extracted with a saturated aqueous ammonium chloride solution. Thereafter, the organic layer was dried over anhydrous magnesium sulfate and distilled under reduced pressure. The concentrated mixture was purified by MPLC to obtain the title compound (4.21 g, 22%).
¹H-NMR (500 MHz, CHLOROFORM-D) δ 8.27 (d, J = 8.5 Hz, 2H), 8.05 (d, J = 6.4 Hz, 1H), 4.36-4.35 (m, 1H), 4.08 (br s, 2H), 3.03 (t, J = 11.4 Hz, 2H), 2.29 (s, 3H), 2.12-2.04 (m, 2H), 1.54-1.50 (m, 11H)
LC/MS: 403.2 (M+Na), 325.1 (M-tbu+2H)

### Step B: Preparation of tert-butyl 4-((3-amino-5-methylpyridin-2-yl)amino)piperidine-1-carboxylate

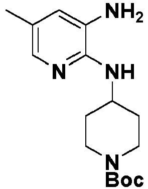

Tert-butyl 4-((5-methyl-3-nitropyridin-2-yl)amino)piperidine-1-carboxylate (4.21 g, 12.52 mmol) was used in a similar manner to Step B of Preparation Example 17 to obtain the title compound (3.64 g, 95%), which was used directly in the next reaction without purification.
LC/MS: 307.2 (M+H)

### Step C: Preparation of tert-butyl 4-(1,7-dimethyl-2,3-dioxo-2,3-dihydropyrido[2,3-b]pyrazin-4(1H)-yl)piperidine-1-carboxylate

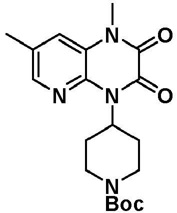

Tert-butyl 4-((3-amino-5-methylpyridin-2-yl)amino)piperidine-1-carboxylate (3.64 g, 11.88 mmol) and triethylamine (6.62 mL, 47.50 mmol) were dissolved in DCE (150 mL), and ethyl 2-chloro-2-oxoacetate (2.65 mL, 23.76 mmol) was slowly added thereto at 0°C, followed by stirring at 60°C for 18 hours. After the reaction mixture was cooled to room temperature, it was extracted with a saturated aqueous ammonium chloride solution. The organic layer was dried over anhydrous magnesium sulfate to obtain a concentrated mixture. The concentrated mixture was used in a similar manner to Step E of Preparation Example 1 to obtain the title compound (1.80 g, 41%). ¹H-NMR (500 MHz, CHLOROFORM-D) δ 8.08 (s, 1H), 7.32 (s, 1H), 5.55 (br s, 1H), 4.43-4.12 (m, 3H), 3.63 (s, 3H), 2.95-2.81 (m, 5H), 2.44 (s, 3H), 1.52 (s, 9H)
LC/MS: 397.2 (M+Na)

### Step D: Preparation of 1,7-dimethyl-4-(piperidin-4-yl)-1,4-dihydropyrido[2,3-b]pyrazine-2,3-dione dihydrochloride

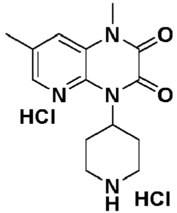

Tert-Butyl 4-(1,7-dimethyl-2,3-dioxo-2,3-dihydropyrido[2,3-b]pyrazin-4(1H)-yl)piperidine-1-carboxylate (1.80 g, 4.81 mmol) was used in a similar manner to Step F of Preparation Example 1 to obtain the title compound (1.65 g, 99%), which was used directly in the next reaction.
¹H-NMR (400 MHz, DMSO-D6) δ 9.14 (d, J= 9.6 Hz, 1H), 8.52 (d, J = 9.6 Hz, 1H), 8.02 (s, 1H), 7.65 (s, 1H), 5.52 (t, J = 11.9 Hz, 1H), 3.45 (s, 3H), 3.34 (d, J = 11.9 Hz, 2H), 3.03 (q, J = 12.3 Hz, 2H), 2.88 (dd, J = 22.4, 12.3 Hz, 2H), 2.34 (s, 3H), 1.76 (d, J = 12.8 Hz, 2H)
LC/MS: 275.1 (M+H)

### Preparation Example 29: Preparation of N,1-dimethyl-2,3-dioxo-4-(piperidin-4-yl)-1,2,3,4-tetrahydropyrido[2,3-b]pyrazine-7-carboxamide dihydrochloride

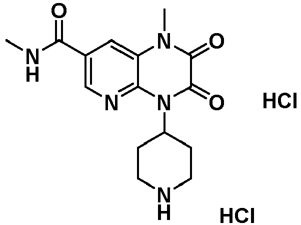

### Step A: Preparation of 4-(1-(tert-butoxycarbonyl)piperidin-4-yl)-1-methyl-2,3-dioxo-1,2,3,4-tetrahydropyrido[2,3-b]pyrazine-7-carboxylic acid

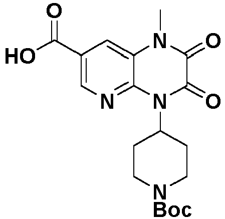

Methyl 4-(1-(tert-butoxycarbonyl)piperidin-4-yl)-1-methyl-2,3-dioxo-1,2,3,4-tetrahydropyrido[2,3-b]pyrazine-7-carboxylate (3.90 g, 9.32 mmol) obtained in Step D of Preparation Example 27 and lithium bromide (4.05 g, 46.60 mmol) were dissolved in THF (90 mL) and distilled water (10 mL), and triethylamine (6.50 mL, 46.60 mmol) was added thereto, followed by stirring at 60°C for 18 hours. The reaction mixture was cooled to room temperature, concentrated by distillation under reduced pressure, and then extracted with EtOAc and distilled water. The aqueous layer was acidified to pH 2-3 using 1 N HCl aqueous solution, and then extracted as an organic layer through DCM solution containing 10% MeOH. The obtained organic layer was dried over anhydrous magnesium sulfate and distilled under reduced pressure to obtain the title compound (2.48 g).
¹H-NMR (500 MHz, DMSO-D6) δ 13.49 (br s, 1H), 8.71 (s, 1H), 8.06 (s, 1H), 5.51 (s, 1H), 4.10 (br s, 2H), 3.55 (s, 3H), 2.88 (br s, 2H), 2.58 (d, J = 12.5 Hz, 2H), 1.63 (d, J = 11.9 Hz, 2H), 1.45 (s, 9H)
LC/MS: 403.1 (M-H)

### Step B: Preparation of tert-butyl 4-(1-methyl-7-(methylcarbamoyl)-23-dioxo-2,3-dihydropyrido[2,3-b]pyrazin-4(1H)-yl)piperidine-1-carboxylate

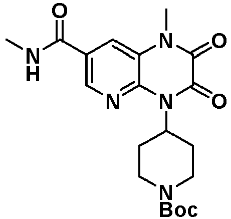

4-(1-(Tert-butoxycarbonyl)piperidin-4-yl)-1-methyl-2,3-dioxo-1,2,3,4-tetrahydropyrido[2,3-b]pyrazine-7-carboxylic acid (100 mg, 0.247 mmol) was dissolved in DCM, and DIPEA (130 µl, 0.742 mmol) and HATU (282 mg, 0.742 mmol) were added thereto, followed by stirring at room temperature for 3 hours. After completion of the reaction, the reaction mixture was extracted with EtOAc and distilled water. The obtained organic layer was dried over anhydrous magnesium sulfate and distilled under reduced pressure to obtain the title compound (100 mg).
¹H-NMR (400 MHz, CHLOROFORM-D) δ 8.52 (d, J = 2.3 Hz, 1H), 8.02 (d, J = 1.8 Hz, 1H), 6.43 (d, J = 4.6 Hz, 1H), 5.44-5.62 (1H), 4.15-4.38 (1H), 3.66 (s, 3H), 3.06 (d, J = 5.0 Hz, 3H), 2.81 (t, J = 10.3 Hz, 4H), 1.64-1.53 (m, 8H), 1.49 (d, J = 3.2 Hz, 9H), 1.44 (d, J = 6.9 Hz, 8H)
LC/MS: 418 (M-H)

### Step C: Preparation of N,1-dimethyl-2,3-dioxo-4-(piperidin-4-yl)-1,2,3,4-tetrahydropyridor23-b lpyrazine-7-carboxamide dihydrochloride

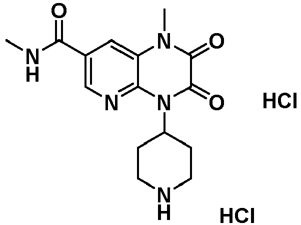

Tert-butyl 4-(1-methyl-7-(methylcarbamoyl)-2,3-dioxo-2,3-dihydropyrido[2,3-b]pyrazin-4(1H)-yl)piperidine-1-carboxylate (100 mg, 0.240 mmol) was used in a similar manner to Step F of Preparation Example 1 to obtain the title compound (75 mg), which was used directly in the next reaction.
LC/MS: 318 (M+H)

### Preparation Example 30: Preparation of 1-methyl-2,3-dioxo-4-(piperidin-4-yl)-1,2,3,4-tetrahydropyrido[2,3-b]pyrazine-7-carboxamide dihydrochloride

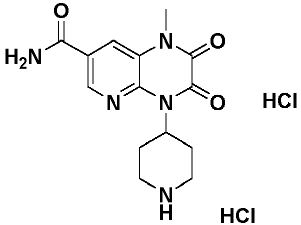

### Step A: Preparation of tert-butyl 4-(7-carbamoyl-1-methyl-23-dioxo-23-dihydropyrido[2,3-b]pyrazin-4(1H)-yl)piperidine-1-carboxylate

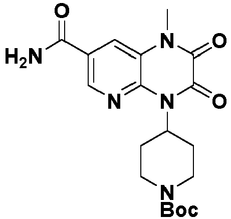

4-(1-(Tert-butoxycarbonyl)piperidin-4-yl)-1-methyl-2,3-dioxo-1,2,3,4-tetrahydropyrido[2,3-b]pyrazine-7-carboxylic acid (200 mg, 0.495 mmol) obtained in Step A of Preparation Example 29 was dissolved in DCM, and thionyl chloride (54.1 µl, 0.742 mmol) was added thereto, followed by stirring at 75°C for 5 hours. After completion of the reaction, the reaction mixture was distilled under reduced pressure and dissolved in anhydrous THF. Ammonium hydroxide (553 µl, 14.19 mmol) was slowly added thereto at 0°C, followed by stirring at room temperature for 1 hour. After completion of the reaction, the reaction mixture was extracted with EtOAc and distilled water. The obtained organic layer was dried over anhydrous magnesium sulfate and distilled under reduced pressure to obtain the title compound (75 mg).
¹H-NMR (400 MHz, CHLOROFORM-D) δ 8.90 (d, J = 1.8 Hz, 1H), 8.43-8.40 (m, 1H), 5.97-6.95 (2H), 5.86 (s, 1H), 4.63 (s, 2H), 4.00 (s, 3H), 3.18 (d, J = 8.7 Hz, 4H), 1.96 (d, J = 10.1 Hz, 2H), 1.81 (s, 9H)
LC/MS: 404 (M-H)

### Step B: Preparation of 1-methyl-2,3-dioxo-4-(piperidin-4-yl)-1,2,3,4-tetrahydropyridor23-b lpyrazine-7-carboxamide dihydrochloride

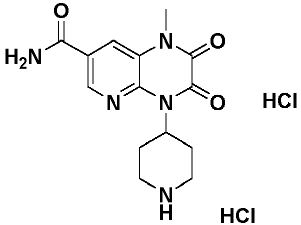

Tert-butyl 4-(7-carbamoyl-1-methyl-2,3-dioxo-2,3-dihydropyrido[2,3-b]pyrazin-4(1H)-yl)piperidine-1-carboxylate (75 mg, 0.186 mmol) was used in a similar manner to Step F of Preparation Example 1 to obtain the title compound (56 mg), which was used directly in the next reaction.
LC/MS: 304 (M-H)

### Example 1: Preparation of methyl-4-(1-(4-(trifluoromethoxy)benzyl)piperidin-4-yl)-1,4-dihydropyrido[2,3-b]pyrazine-2,3-dione

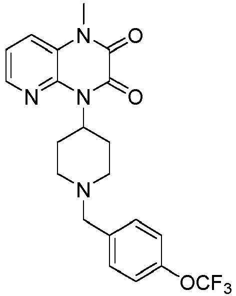

1-Methyl-4-(piperidin-4-yl)-1,4-dihydropyrido[2,3-b]pyrazine-2,3-dione hydrochloride (18 mg, 0.054 mmol) obtained in Preparation Example 1 and diisopropyl (0.019 mL, 0.11 mmol) and 4-(trifluoromethoxy)benzaldehyde (0.015 mL, 0.11) were dissolved in dichloromethane (5 mL), and sodium triacetoxyborohydride (57 mg, 0.27 mmol) was added thereto, followed by stirring at room temperature for 18 hours. The reactant was diluted with an aqueous sodium bicarbonate solution and extracted three times with dichloromethane. The organic layer was dried over anhydrous magnesium sulfate and concentrated under reduced pressure, and the residue was purified by preparatory TLC to obtain the title compound (22 mg).
¹H NMR (500 MHz, CHLOROFORM-D) δ 8.30-8.29 (m, 1H), 7.51 (d, 1H), 7.43 (d, 2H), 7.24∼7.19 (m, 3H), 5.47∼5.43 (m, 1H), 3.65 (s, 3H), 3.62 (s, 2H), 3.10∼3.03 (m, 4H), 2.28∼2.24 (m, 2H), 1.65∼1.63 (m, 2H)
LC/MS: 435 (M+H), 457 (M+Na)

### Example 2: Preparation of 1,6-dimethyl-4-(1-(4-(trifluoromethoxy)benzyl)piperidin-4-yl)-1,4-dihydropvrido[2,3-b]pyrazine-2,3-dione

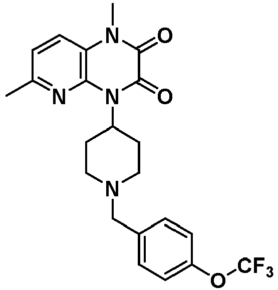

1,6-Dimethyl-4-(piperidin-4-yl)-1,4-dihydropyrido[2,3-b]pyrazine-2,3-dione dihydrochloride (34 mg, 0.109 mmol) obtained in Preparation Example 3 and 4-(trifluoromethoxy)benzaldehyde (41.6 mg, 0.19 mmol) were used in the same manner as in Example 1 to obtain the title compound (42 mg).
¹H NMR (500 MHz, CHLOROFORM-D) δ 7.44 (d, J = 8.5 Hz, 2H), 7.40 (d, J = 8.0 Hz, 1H), 7.20 (d, J = 8.0 Hz, 2H), 7.06 (d, J = 8.5 Hz, 1H), 5.49∼5.45 (m, 1H), 3.62 (s, 3H), 3.62 (s, 2H), 3.11∼3.02 (m, 4H), 2.60 (s, 3H), 2.30∼2.25 (m, 2H), 1.63∼1.61 (m, 2H)
LC/MS: 449 (M+H)

### Example 3: Preparation of ethyl-4-(1-(4-(trifluoromethoxy)benzyl)piperidin-4-yl)-1,4-dihydropyrido[2,3-b]pyrazine-2,3-dione

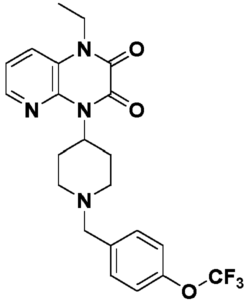

1-Ethyl-4-(piperidin-4-yl)-1,4-dihydropyrido[2,3-b]pyrazine-2,3-dione dihydrochloride (36 mg , 0.104 mmol) obtained in Preparation Example 12 was used in a similar manner to Example 1 to obtain the title compound (22 mg).
¹H-NMR (400 MHz, CHLOROFORM-D) δ 8.24 (dd, J = 4.6, 1.4 Hz, 1H), 7.57-7.47 (m, 1H), 7.41 (d, J = 8.7 Hz, 2H), 7.22-7.14 (m, 3H), 5.44 (t, J = 12.1 Hz, 1H), 4.21 (q, J = 7.2 Hz, 2H), 3.64 (s, 2H), 3.25-3.00 (m, 4H), 2.29 (t, J = 13.3 Hz, 2H), 1.62 (d, J = 8.7 Hz, 2H), 1.35 (t, J = 7.3 Hz, 3H)
LC/MS: 449 (M+H)

### Example 4: Preparation of isobutyl-4-(1-(4-(trifluoromethoxy)benzyl)piperidin-4-yl)-1,4-dihydropyrido[2,3-b]pyrazine-2,3-dione

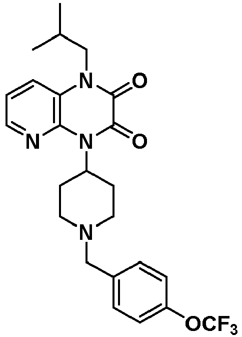

1-Isobutyl-4-(piperidin-4-yl)-1,4-dihydropyrido[2,3-b]pyrazine-2,3-dione dihydrochloride (19 mg, 0.051 mmol) obtained in Preparation Example 13 was used in a similar manner to Example 1 to obtain the title compound (22 mg).
¹H-NMR (400 MHz, CHLOROFORM-D) δ 8.24 (dd, J = 4.6, 1.4 Hz, 1H), 7.45 (dd, J = 8.2, 0.9 Hz, 1H), 7.40 (d, J = 8.7 Hz, 2H), 7.16 (q, J = 4.3 Hz, 3H), 5.50-5.35 (1H), 4.02 (d, J = 7.3 Hz, 2H), 3.58 (s, 2H), 3.13-2.91 (m, 4H), 2.32-2.12 (m, 3H), 1.70-1.53 (m, 2H), 1.02 (q, J = 6.9 Hz, 6H)
LC/MS: 477 (M+H)

### Example 5: Preparation of 1,6-dimethyl-4-(1-(naphthalen-1-ylmethyl)piperidin-4-yl)-1,4-dihydropyrido[2,3-b]pyrazine-2,3-dione

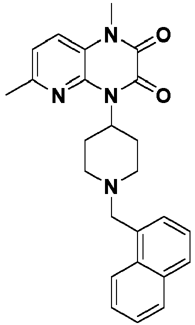

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and 1-naphthylaldehyde (0.012 mL, 0.086 mmol) were used in a similar manner to Example 1 to obtain the title compound (9.7 mg).
¹H-NMR (400 MHz, CHLOROFORM-D) δ 8.49-8.35 (1H), 7.89-7.81 (1H), 7.81-7.70 (1H), 7.49 (d, J = 7.8 Hz, 3H), 7.45-7.37 (1H), 7.34 (d, J = 8.2 Hz, 1H), 7.02 (s, 1H), 5.55-5.37 (1H), 4.01 (s, 2H), 3.58 (s, 3H), 3.07 (s, 4H), 2.55 (s, 3H), 2.31 (s, 2H), 1.66-1.48 (2H)
LC/MS: 415 (M+H)

### Example 6: Preparation of 4-(1-((2-chloroquinolin-3-yl)methyl)piperidin-4-yl)-1,6-dimethyl-1,4-dihydropyrido[2,3-b]pyrazine-2,3-dione

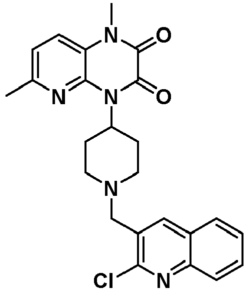

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and 2-chloroquinoline-3-carbaldehyde (16.55 mg, 0.086 mmol) were used in a similar manner to Example 1 to obtain the title compound (12.2 mg).
¹H-NMR (400 MHz, CHLOROFORM-D) δ 8.38 (s, 1H), 8.00 (d, J = 8.2 Hz, 1H), 7.86 (d, J = 8.2 Hz, 1H), 7.78-7.62 (m, 1H), 7.55 (t, J = 7.5 Hz, 1H), 7.37 (d, J = 8.2 Hz, 1H), 7.04 (d, J = 8.2 Hz, 1H), 5.63-5.42 (m, 1H), 3.82 (s, 2H), 3.70-3.52 (m, 3H), 3.24-3.00 (m, 4H), 2.58 (s, 3H), 2.52-2.34 (2H), 1.74-1.55 (m, 2H)
LC/MS: 450 (M+H)

### Example 7: Preparation of 4-(1-((1,2-dimethyl-1H-indol-3-yl)methyl)piperidin-4-yl)-1,6-dimethyl-1,4-dihydropyrido[2,3-b]pyrazine-2,3-dione

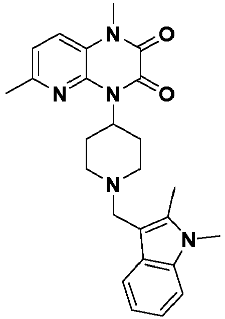

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and 1,2-dimethyl-1H-indole-3-carbaldehyde (14.97 mg, 0.086 mmol) obtained in Preparation Example 14 were used in a similar manner to Example 1 to obtain the title compound (1.5 mg).
¹H-NMR (400 MHz, CHLOROFORM-D) δ 7.63 (d, J = 7.3 Hz, 1H), 7.35 (d, J = 8.2 Hz, 1H), 7.28 (d, J = 7.8 Hz, 2H), 7.18 (t, J = 7.3 Hz, 1H), 7.12 (t, J = 7.3 Hz, 1H), 7.02 (d, J = 8.2 Hz, 1H), 5.32 (d, J = 30.2 Hz, 1H), 4.11 (q, J = 7.2 Hz, 2H), 3.73 (d, J = 23.8 Hz, 3H), 3.60 (d, J = 15.6 Hz, 3H), 3.45-3.18 (m, 4H), 1.66 (d, J = 8.7 Hz, 2H)
LC/MS: 432 (M+H)

### Example 8: Preparation of 1,6-dimethyl-4-(1-((1-methyl-1H-indol-5-yl)methyl)piperidin-4-yl)-1,4-dihydropyrido[2,3-b]pyrazine-2,3-dione

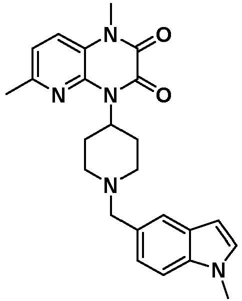

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and 1-methyl-1H-indole-5-carbaldehyde (13.75 mg, 0.086 mmol) obtained in Preparation Example 15 were used in a similar manner to Example 1 to obtain the title compound (23.6 mg).
¹H-NMR (400 MHz, CHLOROFORM-D) δ 7.58 (s, 1H), 7.34 (d, J = 8.2 Hz, 1H), 7.29 (d, J = 9.1 Hz, 2H), 7.10-6.95 (m, 2H), 6.45 (d, J = 3.2 Hz, 1H), 5.52-5.31 (m, 1H), 3.89-3.77 (m, 3H), 3.76 (s, 2H), 3.59 (t, J = 6.6 Hz, 3H), 3.19-2.94 (m, 4H), 2.56 (t, J = 12.8 Hz, 3H), 2.41-2.10 (m, 2H), 1.57 (d, J = 10.5 Hz, 2H)
LC/MS: 418 (M+H)

### Example 9: Preparation of 1,6-dimethyl-4-(1-(quinolin-4-ylmethyl)piperidin-4-yl)-1,4-dihydropyrido[2,3-b]pyrazine-2,3-dione

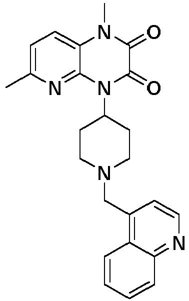

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and quinoline-4-carbaldehyde (0.011 mL, 0.086 mmol) were used in a similar manner to Example 1 to obtain the title compound (20 mg).
¹H-NMR (400 MHz, CHLOROFORM-D) δ 8.87 (d, J = 4.1 Hz, 1H), 8.33 (d, J = 8.2 Hz, 1H), 8.12 (d, J = 8.7 Hz, 1H), 7.80-7.62 (m, 1H), 7.62-7.53 (m, 1H), 7.51 (d, J = 4.1 Hz, 1H), 7.36 (d, J= 8.2 Hz, 1H), 7.10-6.95 (1H), 5.47 (t, J= 11.9 Hz, 1H), 4.02 (s, 2H), 3.59 (s, 3H), 3.21-2.96 (m, 4H), 2.55 (d, J = 14.2 Hz, 3H), 2.47-2.26 (m, 2H), 1.64-1.48 (2H)
LC/MS: 416 (M+H)

### Example 10: Preparation of 4-(1-([1,1'-biphenyl]-2-ylmethyl)piperidin-4-yl)-1,6-dimethyl-1,4-dihydropyrido[2,3-b]pyrazine-2,3-dione

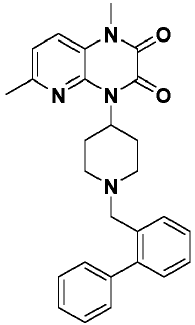

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and [1,1'-biphenyl]-2-carbaldehyde (0.014 mL, 0.086 mmol) were used in a similar manner to Example 1 to obtain the title compound (23 mg).
¹H-NMR (400 MHz, CHLOROFORM-D) δ 7.59 (d, J = 7.3 Hz, 1H), 7.52-7.37 (4H), 7.37-7.32 (m, 3H), 7.29 (d, J = 7.3 Hz, 1H), 7.25 (t, J = 3.4 Hz, 2H), 7.00 (d, J = 8.2 Hz, 1H), 5.46-5.25 (m, 1H), 3.58 (s, 3H), 3.43 (s, 2H), 3.09-2.84 (m, 4H), 2.61-2.46 (3H), 2.08 (t, J = 11.4 Hz, 2H), 1.80-1.59 (m, 2H), 1.54 (d, J = 11.0 Hz, 2H)
LC/MS: 441 (M+H)

### Example 11: Preparation of 1-methyl-2,3-dioxo-4-(1-(4-(trifluoromethoxy)benzyl)piperidin-4-yl)-1,2,3,4-tetrahydropyrido[2,3-b]pyrazine-6-carbonitrile

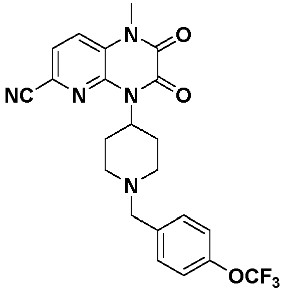

1-Methyl-2,3-dioxo-4-(piperidin-4-yl)-1,2,3,4-tetrahydropyrido[2,3-b]pyrazine-6-carbonitrile dihydrochloride (36 mg, 0.100 mmol) obtained in Preparation Example 4 and 4-(trifluoromethoxy)benzaldehyde (41.6 mg, 0.19 mmol) were used in a similar manner to Example 1 to obtain the title compound (26 mg).
¹H NMR (500 MHz, CHLOROFORM-D) δ 7.64 (d, J = 8.5 Hz, 1H), 7.57 (d, J = 8.5 Hz, 1H), 7.44 (d, J = 8.5 Hz, 2H), 7.20 (d, J = 8.0 Hz, 2H), 7.06 (d, J = 8.5 Hz, 1H), 5.38-5.31 (m, 1H), 3.67 (s, 3H), 3.61 (s, 2H), 3.05-2.93 (m, 4H), 2.28-2.24 (m, 2H), 1.64-1.62 (m, 2H)
LC/MS: 460 (M+H)

### Example 12: Preparation of 4-(1-(2-chlorobenzyl)piperidin-4-yl)-1,6-dimethyl-1,4-dihydropyrido[2,3-b]pyrazine-2,3-dione

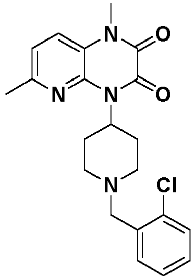

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and 2-chlorobenzaldehyde (18 mg, 0.13 mmol) were used in a similar manner to Example 1 to obtain the title compound (21 mg).
¹H-NMR (500 MHz, CHLOROFORM-D) δ 7.63 (d, J = 7.3 Hz, 1H), 7.38 (q, J = 8.3 Hz, 2H), 7.30-7.27 (m, 1H), 7.20 (t, J = 7.5 Hz, 1H), 7.06 (d, J = 8.2 Hz, 1H), 5.51-5.47 (m, 1H), 3.73 (s, 2H), 3.62 (s, 3H), 3.12-3.06 (m, 4H), 2.60 (s, 3H), 2.40-2.34 (m, 2H), 1.92 (s, 1H), 1.62 (d, J = 11.9 Hz, 2H)
LC/MS: 399 (M+H)

### Example 13: Preparation of 4-(1-(3-chlorobenzyl)piperidin-4-yl)-1,6-dimethyl-1,4-dihydropyrido[2,3-b]pyrazine-2,3-dione

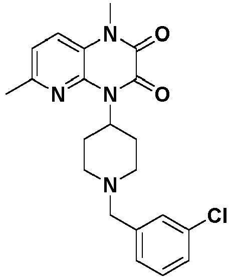

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and 3-chlorobenzaldehyde (18 mg, 0.13 mmol) were used in a similar manner to Example 1 to obtain the title compound (18 mg).
¹H-NMR (500 MHz, CHLOROFORM-D) δ 7.41-7.38 (m, 2H), 7.29-7.24 (m, 3H), 7.06 (d, J = 8.2 Hz, 1H), 5.48-5.43 (m, 1H), 3.62 (s, 3H), 3.59 (s, 2H), 3.06-3.01 (m, 3H), 2.62 (d, J = 14.6 Hz, 3H), 2.28-2.06 (m, 2H), 1.94 (s, 1H), 1.70-1.61 (m, 2H)
LC/MS: 399 (M+H)

### Example 14: Preparation of 4-(1-(4-chlorobenzyl)piperidin-4-yl)-1,6-dimethyl-1,4-dihydropyrido[2,3-b]pyrazine-2,3-dione

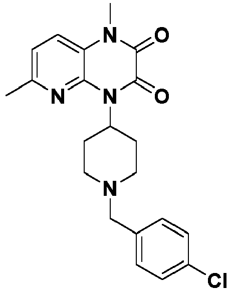

1,6-Dimethyl-4-(piperidin-4-yl)-1,4-dihydropyrido[2,3-b]pyrazine-2,3-dione hydrochloride (30 mg, 0.086 mmol) obtained in Preparation Example 3, 1-(bromomethyl)-4-chlorobenzene (0.018 mg, 0.86 mmol) and DIPEA (0.02 mL, 0.11 mmol) were dissolved in dichloromethane (5 mL) and stirred at room temperature for 6 hours. The reaction mixture was diluted with EtOAc and washed with brine, and the organic layer was dried over anhydrous magnesium sulfate and concentrated under reduced pressure. The residue was purified by MPLC to obtain the title compound (31 mg).
¹H-NMR (500 MHz, CHLOROFORM-D) δ 7.34-7.28 (m, 5H), 7.05 (d, J = 7.9 Hz, 1H), 5.45 (d, J= 11.3 Hz, 1H), 3.62 (s, 3H), 3.58 (s, 2H), 3.04 (q, J= 12.3 Hz, 4H), 2.58 (d, J = 17.1 Hz, 3H), 2.26-2.22 (m, 2H), 1.92 (s, 1H), 1.70-1.60 (m, 2H)
LC/MS: 399 (M+H)

### Example 15: Preparation of 4-(1-((2-methoxypyridin-3-yl)methyl)piperidin-4-yl)-1,6-dimethyl-1,4-dihydropyrido[2,3-b]pyrazine-2,3-dione

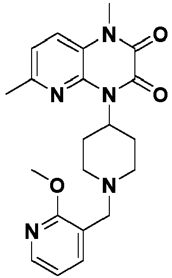

1,6-Dimethyl-4-(piperidin-4-yl)-1,4-dihydropyrido[2,3-b]pyrazine-2,3-dione hydrochloride (30 mg, 0.086 mmol) obtained in Preparation Example 3 and 2-methoxynicotinaldehyde (26 mg, 0.19 mmol) were used in a similar manner to Example 1 to obtain the title compound (15 mg).
¹H-NMR (500 MHz, CHLOROFORM-D) δ 8.09 (d, J = 3.4 Hz, 1H), 7.80 (d, J = 6.7 Hz, 1H), 7.40 (d, J = 7.9 Hz, 1H), 7.06 (d, J = 8.2 Hz, 1H), 6.93 (dd, J = 7.2, 5.0 Hz, 1H), 5.48 (t, J = 11.7 Hz, 1H), 3.99 (s, 3H), 3.62 (s, 5H), 3.12-3.06 (m, 4H), 2.61 (d, J = 11.6 Hz, 3H), 2.37-2.33 (m, 2H), 1.63 (d, J = 10.4 Hz, 2H)
LC/MS: 396 (M+H)

### Example 16: Preparation of 4-(1-((6-fluoropyridin-2-yl)methyl)piperidin-4-yl)-1,6-dimethyl-1,4-dihydropyrido[2,3-b]pyrazine-2,3-dione

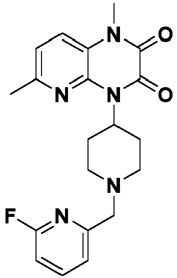

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and 6-fluoropicolinaldehyde (36 mg, 0.29 mmol) were used in a similar manner to Example 1 to obtain the title compound (21 mg).
¹H-NMR (500 MHz, CHLOROFORM-D) δ 8.18 (s, 1H), 7.93-7.89 (m, 1H), 7.41-7.39 (m, 1H), 7.06 (d, J = 4.0 Hz, 1H), 6.95 (dd, J = 8.2, 2.7 Hz, 1H), 5.50-5.45 (m, 1H), 3.62 (s, 4H), 3.61 (s, 2H), 3.06-3.00 (m, 5H), 2.59 (s, 3H), 2.30-2.25 (m, 2H)
LC/MS: 384 (M+H), 406 (M+Na)

### Example 17: Preparation of 4-(1-((6-fluoropyridin-3-yl)methyl)piperidin-4-yl)-1,6-dimethyl-1,4-dihydropyrido[2,3-b]pyrazine-2,3-dione

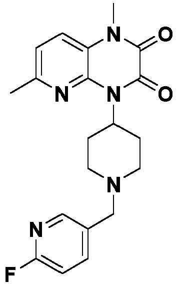

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and 6-fluoronicotinaldehyde (36 mg, 0.29 mmol) were used in a similar manner to Example 1 to obtain the title compound (11 mg).
¹H-NMR (500 MHz, CHLOROFORM-D) δ 8.19-8.11 (m, 1H), 7.49-7.39 (m, 1H), 7.10-7.00 (m, 3H), 5.54-5.43 (m, 1H), 3.64 (s, 2H), 3.63-3.48 (m, 3H), 3.10 (qd, J = 12.3, 3.7 Hz, 2H), 3.00 (d, J = 11.6 Hz, 2H), 2.63-2.58 (m, 3H), 2.34-2.19 (m, 2H), 1.65-1.45 (m, 2H)
LC/MS: 384 (M+H)

### Example 18: Preparation of 1,6-dimethyl-4-(1-(4-phenoxybenzyl)piperidin-4-yl)-1,4-dihydropyrido[2,3-b]pyrazine-2,3-dione

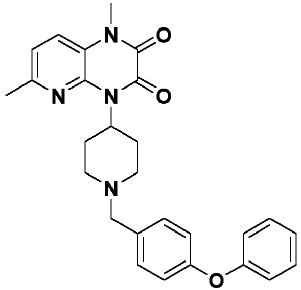

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and 4-phenoxybenzaldehyde (17.13 mg, 0.086 mmol) were used in a similar manner to Example 1 to obtain the title compound (28 mg).
¹H-NMR (400 MHz, CHLOROFORM-D) δ 7.37-7.29 (m, 5H), 7.10-7.06 (m, 1H), 7.03-6.99 (m, 3H), 6.96 (dt, J = 9.1, 2.4 Hz, 2H), 5.43 (t, J = 11.9 Hz, 1H), 3.59 (s, 2H), 3.58 (s, 3H), 3.10-3.00 (m, 4H), 2.60-2.54 (m, 3H), 2.27-2.21 (m, 2H), 1.60-1.57 (m, 2H)
LC/MS: 457 (M+H)

### Example 19: Preparation of 1,6-dimethyl-4-(1-(naphthalen-2-ylmethyl)piperidin-4-yl)-1,4-dihydropyrido[2,3-b]pyrazine-2,3-dione

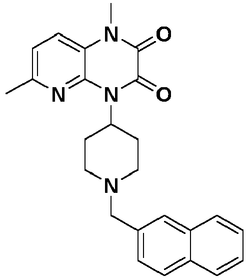

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and 2-naphthaldehyde (13.49 mg, 0.086 mmol) were used in a similar manner to Example 1 to obtain the title compound (15 mg).
¹H-NMR (400 MHz, CHLOROFORM-D) δ 7.83-7.78 (m, 4H), 7.56 (dd, J = 8.7, 1.4 Hz, 1H), 7.51-7.42 (m, 2H), 7.35 (d, J = 8.2 Hz, 1H), 7.01 (t, J = 8.9 Hz, 1H), 5.48-5.42 (m, 1H), 3.76 (s, 2H), 3.62 (d, J = 28.8 Hz, 3H), 3.10-3.02 (m, 4H), 2.58 (d, J = 9.6 Hz, 3H), 2.30-2.24 (m, 2H), 1.59 (d, J = 11.4 Hz, 2H)
LC/MS: 415 (M+H)

### Example 20: Preparation of 4-(1-(isoquinolin-5-ylmethyl)piperidin-4-yl)-1,6-dimethyl-1,4-dihydropyrido[2,3-b]pyrazine-2,3-dione

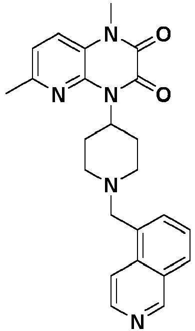

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and isoquinoline-5-carbaldehyde (46 mg, 0.29 mmol) were used in a similar manner to Example 1 to obtain the title compound (35 mg).
¹H-NMR (500 MHz, CHLOROFORM-D) δ 9.27 (s, 1H), 8.59 (d, J = 5.8 Hz, 1H), 8.23 (d, J = 5.8 Hz, 1H), 7.92 (d, J = 8.2 Hz, 1H), 7.74 (d, J = 7.0 Hz, 1H), 7.58 (t, J = 7.5 Hz, 1H), 7.05 (d, J = 8.2 Hz, 1H), 5.48 (t, J = 11.9 Hz, 1H), 4.00 (s, 2H), 3.61 (s, 3H), 3.10-3.06 (m, 3H), 2.58 (s, 3H), 2.37-2.32 (m, 2H), 1.84 (s, 2H), 1.63-1.60 (m, 2H)
LC/MS: 438 (M+Na)

### Example 21: Preparation of 1,6-dimethyl-4-(1-(3-phenoxybenzyl)piperidin-4-yl)-1,4-dihydropyrido[2,3-b]pyrazine-2,3-dione

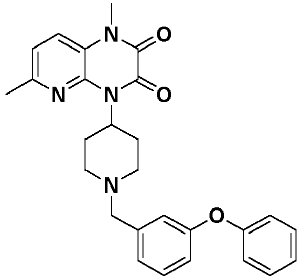

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and 3-phenoxybenzaldehyde (0.015 ml, 0.086 mmol) were used in a similar manner to Example 1 to obtain the title compound (28 mg).
¹H-NMR (400 MHz, CHLOROFORM-D) δ 7.37-7.26 (m, 4H), 7.14-7.05 (m, 3H), 7.01 (dt, J = 8.7, 1.3 Hz, 3H), 6.89 (dd, J = 7.8, 1.8 Hz, 1H), 5.43-5.36 (m, 1H), 3.59 (s, 2H), 3.58 (s, 3H), 3.08-3.02 (m, 4H), 2.56 (d, J = 23.3 Hz, 3H), 2.23 (t, J = 11.7 Hz, 2H), 1.58 (d, J = 8.7 Hz, 2H)
LC/MS: 457 (M+H)

### Example 22: Preparation of 4-(1-(3-chlorobenzyl)piperidin-4-yl)-1-methyl-1,4-dihydropyrido[2,3-b]pyrazine-2,3-dione

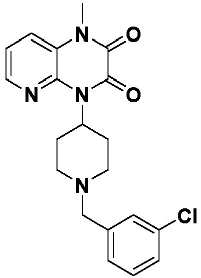

1-Methyl-4-(piperidin-4-yl)-1,4-dihydropyrido[2,3-b]pyrazine-2,3-dione hydrochloride (30 mg, 0.10 mmol) obtained in Preparation Example 1 and 3-chlorobenzaldehyde (43 mg, 0.30 mmol) were used in a similar manner to Example 1 to obtain the title compound (20 mg).
¹H-NMR (500 MHz, CHLOROFORM-D) δ 8.30 (d, J = 4.3 Hz, 1H), 7.51 (d, J = 7.9 Hz, 1H), 7.40 (s, 1H), 7.28-7.22 (m, 4H), 5.43 (t, J = 12.1 Hz, 1H), 3.64 (s, 3H), 3.59 (s, 2H), 3.06 (q, J = 12.3 Hz, 4H), 2.27-2.23 (m, 2H), 1.63 (d, J = 11.0 Hz, 2H)
LC/MS: 385 (M+H)

### Example 23: Preparation of 4-(1-benzylpiperidin-4-yl)-1,6-dimethyl-1,4-dihydropyrido[2,3-b]pyrazine-2,3-dione

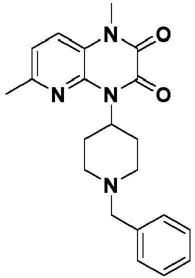

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and benzaldehyde (31 mg, 0.29 mmol) were used in a similar manner to Example 1 to obtain the title compound (8 mg).
¹H-NMR (500 MHz, CHLOROFORM-D) δ 7.41-7.27 (m, 6H), 7.05 (d, J = 8.2 Hz, 1H), 5.45 (t, J = 12.1 Hz, 1H), 3.63 (d, J= 14.0 Hz, 5H), 3.11-3.05 (m, 3H), 2.60 (s, 3H), 2.28-2.24 (m, 3H), 1.61 (d, J = 11.0 Hz, 2H)
LC/MS: 365 (M+H)

### Example 24: Preparation of 1,6-dimethyl-4-(1-(1-(quinoxalin-6-yl)ethyl)piperidin-4-yl)-1,4-dihydropyrido[2,3-b]pyrazine-2,3-dione

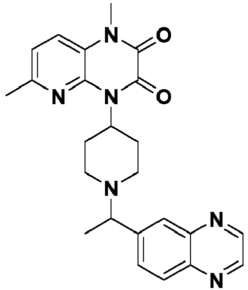

1,6-Dimethyl-4-(piperidin-4-yl)-1,4-dihydropyrido[2,3-b]pyrazine-2,3-dione dihydrochloride (30 mg, 0.086 mmol) obtained in Preparation Example 3 and 6-(1-chloroethyl)quinoxaline (23.17 mg, 0.086 mmol) obtained in Preparation Example 16 were dissolved in DMF (2 mL), and potassium carbonate (45 mg, 0.33 mmol) was added thereto, followed by stirring at 90°C for 16 hours. The reaction mixture was diluted with EtOAc and washed with brine, and the organic layer was dried over anhydrous magnesium sulfate and concentrated under reduced pressure. The residue was purified by MPLC to obtain the title compound (22 mg).
¹H-NMR (500 MHz, CHLOROFORM-D) δ 8.85 (d, J = 4.3 Hz, 2H), 8.22-8.12 (m, 1H), 8.04-8.02 (m, 2H), 7.39 (d, J = 8.2 Hz, 1H), 7.05 (d, J = 7.9 Hz, 1H), 5.44 (t, J = 12.2 Hz, 1H), 3.91-3.87 (m, 1H), 3.69-3.59 (m, 4H), 3.18 (d, J= 10.7 Hz, 1H), 3.12-2.90 (m, 3H), 2.64-2.60 (m, 3H), 2.34-2.29 (m, 2H), 1.53 (d, J = 6.7 Hz, 3H)
LC/MS: 431 (M+H)

### Example 25: Preparation of 4-(1-(3-chlorobenzyl)piperidin-4-yl)-1-methyl-2,3-dioxo-1,2,3,4-tetrahydropyrido[2,3-b]pyrazine-6-carbonitrile

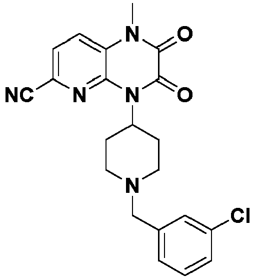

1-Methyl-2,3-dioxo-4-(piperidin-4-yl)-1,2,3,4-tetrahydropyrido[2,3-b]pyrazine-6-carbonitrile dihydrochloride (50 mg, 0.155 mmol) obtained in Preparation Example 4 and 3-chlorobenzaldehyde (33 mg, 0.27 mmol) were used in a similar manner to Example 1 to obtain the title compound (31 mg).
¹H NMR (500 MHz, CHLOROFORM-D) δ 8.64 (d, J = 8.5 Hz, 1H), 7.56 (d, J = 8.0 Hz, 1H), 7.41 (s, 1H), 7.32-7.28 (m, 3H), 5.39-5.33 (m, 1H), 3.67 (s, 3H), 3.67 (s, 2H), 3.12-2.98 (m, 4H), 2.37-2.32 (m, 2H), 1.66-1.64 (m, 2H)
LC/MS: 410 (M+H)

### Example 26: Preparation of 1-methyl-4-(1-(naphthalen-1-ylmethyl)piperidin-4-yl)-2,3-dioxo-1,2,3,4-tetrahydropyrido[2,3-b]pyrazine-6-carbonitrile

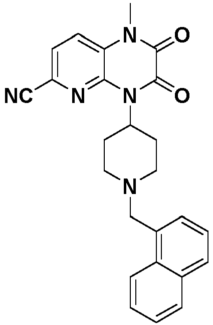

1-Methyl-2,3-dioxo-4-(piperidin-4-yl)-1,2,3,4-tetrahydropyrido[2,3-b]pyrazine-6-carbonitrile dihydrochloride (40 mg, 0.124 mmol) obtained in Preparation Example 4 and 1-naphthaldehyde (19 mg, 0.12 mmol) were used in a similar manner to Example 1 to obtain the title compound (33 mg).
¹H NMR (500 MHz, CHLOROFORM-D) δ 8.43 (d, J = 8.5 Hz, 1H), 7.88 (d, J = 8.0 Hz, 1H), 7.81 (d, J= 8.0 Hz, 1H), 7.63~7.51 (m, 5H), 7.46~7.43 (m, 1H), 5.40-5.35 (m, 1H), 4.06 (s, 2H), 3.66 (s, 3H), 3.15-3.13 (m, 2H), 3.032~2.95 (m, 2H), 2.38-2.34 (m, 2H), 1.64~1.62 (m, 2H)
LC/MS: 426 (M+H)

### Example 27: Preparation of 7-chloro-4-(1-(3-chlorobenzyl)piperidin-4-yl)-1-methyl-1,4-dihydropyrido[2,3-b]pyrazine-2,3-dione

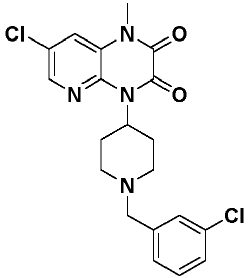

7-Chloro-1-methyl-4-(piperidin-4-yl)-1,4-dihydropyrido[2,3-b]pyrazine-2,3-dione dihydrochloride (40 mg, 0.109 mmol) obtained in Preparation Example 2 and 3-chlorobenzaldehyde (0.012 mL, 0.109 mmol) were used in a similar manner to Example 1 to obtain the title compound (15 mg).
¹H-NMR (400 MHz, CHLOROFORM-D) δ 8.21 (d, J = 1.8 Hz, 1H), 7.45 (d, J = 1.8 Hz, 1H), 7.36 (s, 1H), 7.25-7.20 (m, 3H), 5.30 (qd, J= 7.9, 4.1 Hz, 1H), 3.59 (s, 3H), 3.54 (s, 2H), 3.01-2.92 (m, 4H), 2.22-2.16 (m, 2H), 1.61-1.58 (m, 2H)
LC/MS: 420 (M+H)

### Example 28: Preparation of 7-chloro-1-methyl-4-(1-(4-(trifluoromethoxy)benzyl)piperidin-4-yl)-1,4-dihydropyrido[2,3-b]pyrazine-2,3-dione

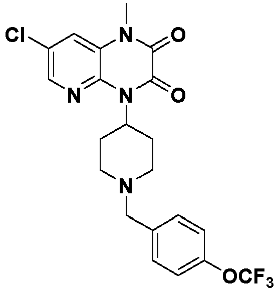

7-Chloro-1-methyl-4-(piperidin-4-yl)-1,4-dihydropyrido[2,3-b]pyrazine-2,3-dione dihydrochloride (40 mg, 0.109 mmol) obtained in Preparation Example 2 and 4-(trifluoromethoxy)benzaldehyde (0.016 mL, 0.109 mmol) were used in a similar manner to Example 1 to obtain the title compound (22 mg).
¹H-NMR (400 MHz, CHLOROFORM-D) δ 8.20 (d, J = 1.8 Hz, 1H), 7.45 (d, J = 2.3 Hz, 1H), 7.39 (d, J = 8.2 Hz, 2H), 7.16 (d, J = 7.8 Hz, 2H), 5.34-5.29 (m, 1H), 3.59 (s, 3H), 3.56 (s, 2H), 3.01-2.92 (m, 4H), 2.22-2.16 (m, 2H), 1.58 (s, 2H)
LC/MS: 469 (M+H)

### Example 29: Preparation of 7-chloro-1-methyl-4-(1-(naphthalen-1-ylmethyl)piperidin-4-yl)-1,4-dihydropyrido[2,3-b]pyrazine-2,3-dione

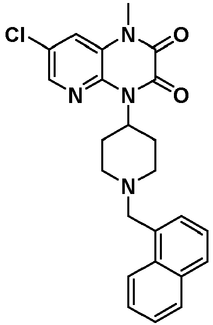

7-Chloro-1-methyl-4-(piperidin-4-yl)-1,4-dihydropyrido[2,3-b]pyrazine-2,3-dione dihydrochloride (50 mg, 0.170 mmol) obtained in Preparation Example 2 and 1-naphthaldehyde (0.023 mL, 0.170 mmol) were used in a similar manner to Example 1 to obtain the title compound (48 mg).
¹H-NMR (400 MHz, CHLOROFORM-D) δ 8.34 (d, J = 8.2 Hz, 1H), 8.19 (d, J = 1.8 Hz, 1H), 7.84 (d, J = 7.8 Hz, 1H), 7.77 (d, J = 8.2 Hz, 1H), 7.55-7.39 (m, 5H), 5.34 (tt, J = 12.1, 3.9 Hz, 1H), 3.98 (s, 2H), 3.59 (d, J = 12.8 Hz, 3H), 3.09 (d, J = 11.4 Hz, 2H), 2.97 (qd, J= 12.3, 3.8 Hz, 2H), 2.28 (dd, J = 11.9, 10.1 Hz, 2H), 1.58 (d, J = 10.1 Hz, 2H)
LC/MS: 435 (M+H)

### Example 30: Preparation of 7-chloro-1-methyl-4-(1-(3-(trifluoromethyl)benzyl)piperidin-4-yl)-1,4-dihydropyrido[2,3-b]pyrazine-2,3-dione

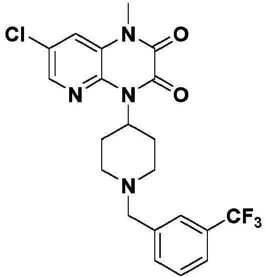

7-Chloro-1-methyl-4-(piperidin-4-yl)-1,4-dihydropyrido[2,3-b]pyrazine-2,3-dione dihydrochloride (50 mg, 0.170 mmol) obtained in Preparation Example 2 and 3-(trifluoromethyl)benzaldehyde (0.018 mL, 0.136 mmol) were used in a similar manner to Example 1 to obtain the title compound (56.7 mg).
¹H-NMR (400 MHz, CHLOROFORM-D) δ 8.21 (d, J = 1.8 Hz, 1H), 7.60-7.42 (m, 5H), 5.31 (d, J = 3.7 Hz, 1H), 3.62 (s, 2H), 3.59 (s, 3H), 3.02-2.94 (m, 4H), 2.21 (dd, J = 13.0, 11.2 Hz, 2H), 1.61-1.57 (m, 2H)
LC/MS: 453 (M+H)

### Example 31: Preparation of 7-chloro-4-(3,3-dimethyl-1-(4-(trifluoromethoxy) benzyl)piperidin-4-yl)-1-methyl-1,4-dihydropyrido[2,3-b]pyrazine-2,3-dione

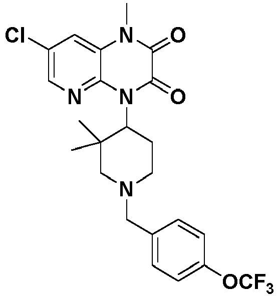

7-Chloro-4-(3,3-dimethylpiperidin-4-yl)-1-methyl-1,4-dihydropyrido[2,3-b]pyrazine-2,3-dione dihydrochloride (47 mg, 0.119 mmol) obtained in Preparation Example 5 and 4-(trifluoromethoxy)benzaldehyde (68 mg, 0.36 mmol) were used in a similar manner to Example 1 to obtain the title compound (49 mg).
¹H NMR (500 MHz, CHLOROFORM-D) δ 8.20 (d, J = 2.0 Hz, 1H), 7.47 (d, J = 2.0 Hz, 1H), 7.43 (d, J = 8.5 Hz, 2H), 7.20 (d, J = 8.0 Hz, 2H), 5.55-5.20 (m, 1H), 3.64 (s, 3H), 3.62~3.44 (m, 3H), 3.10-3.03 (m, 1H), 2.51-2.49 (m, 1H), 2.21-2.04 (m, 2H), 1.58-1.52 (m, 2H), 1.24-1.17 (2 s, 3H), 0.86~0.70 (2 s, 3H)
LC/MS: 497 (M+H)

### Example 32: Preparation of 7-chloro-1-methyl-4-((1R,3s,5S)-8-(4-(trifluoromethoxy)benzyl)-8-azabicyclo[3.2.1]octan-3-yl)-1,4-dihydropyrido[2,3-b]pyrazine-2,3-dione

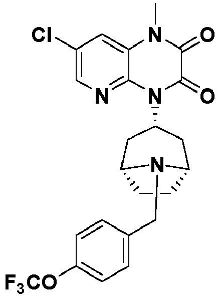

4-(8-Azabicyclo[3.2.1]octan-3-yl)-7-chloro-1-methyl-1,4-dihydropyrido[2,3-b]pyrazine-2,3-dione dihydrochloride (40 mg, 0.10 mmol) obtained in Preparation Example 6 and 4-(trifluoromethoxy)benzaldehyde (58 mg, 0.31 mmol) were used in a similar manner to Example 1 to obtain the title compound (32 mg).
¹H-NMR (500 MHz, CHLOROFORM-D) δ 8.26 (d, J = 4.6 Hz, 1H), 7.50-7.46 (m, 4H), 7.19 (d, J = 6.1 Hz, 1H), 5.94 (t, J = 9.6 Hz, 1H), 3.62 (s, 3H), 3.51 (s, 2H), 3.35 (s, 2H), 2.33 (dd, J = 21.4, 9.5 Hz, 2H), 2.18-2.06 (m, 4H), 1.94 (d, J = 7.6 Hz, 2H)
LC/MS: 495 (M+H)

### Example 33: Preparation of 7-chloro-1-methyl-4-(1-(quinoxalin-5-ylmethyl)piperidin-4-yl)-1,4-dihydropyrido[2,3-b]pyrazine-2,3-dione

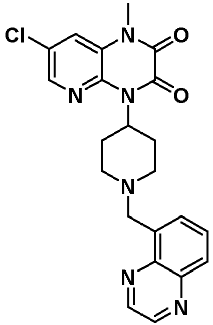

7-Chloro-1-methyl-4-(piperidin-4-yl)-1,4-dihydropyrido[2,3-b]pyrazine-2,3-dione dihydrochloride (50 mg, 0.170 mmol) obtained in Preparation Example 2 and quinoxaline-5-carbaldehyde (23.66 mg, 0.150 mmol) were used in a similar manner to Example 1 to obtain the title compound (20 mg).
¹H-NMR (400 MHz, CHLOROFORM-D) δ 8.84 (s, 2H), 8.21 (d, J = 1.8 Hz, 1H), 8.01 (q, J = 3.8 Hz, 2H), 7.80 (t, J = 7.8 Hz, 1H), 7.45 (d, J = 1.8 Hz, 1H), 5.34 (tt, J = 12.2, 4.0 Hz, 1H), 4.32 (s, 2H), 3.60 (d, J = 9.6 Hz, 3H), 3.14 (d, J = 11.4 Hz, 2H), 3.05 (qd, J = 12.2, 3.9 Hz, 2H), 2.40-2.31 (m, 2H), 1.62 (d, J = 9.1 Hz, 2H)
LC/MS: 437 (M+H)

### Example 34: Preparation of 7-bromo-1-methyl-4-(1-(4-(trifluoromethoxy)benzyl)piperidin-4-yl)-1,4-dihydropyrido[2,3-b]pyrazine-2,3-dione

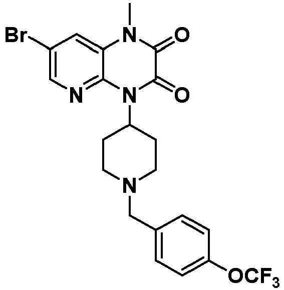

7-Bromo-1-methyl-4-(piperidin-4-yl)-1,4-dihydropyrido[2,3-b]pyrazine-2,3-dione dihydrochloride (314 mg, 0.762 mmol) obtained in Preparation Example 7 and 4-(trifluoromethoxy)benzaldehyde (290 mg, 1.524 mmol) were used in a similar manner to Example 1 to obtain the title compound (296 mg).
¹H NMR (500 MHz, CHLOROFORM-D) δ 8.33 (d, J = 2.0 Hz, 1H), 7.61 (d, J = 2.0 Hz, 1H), 7.42 (d, J = 8.5 Hz, 2H), 7.20 (d, J = 8.0 Hz, 2H), 5.36-5.32 (m, 1H), 3.63 (s, 3H), 3.60 (s, 2H), 3.04-2.97 (m, 4H), 2.25-2.20 (m, 2H), 1.64-1.61 (m, 2H)
LC/MS: 512, 514 (M+H)

### Example 35: Preparation of 7-chloro-4-(1-((5-chloronaphthalen-1-yl)methyl)piperidin-4-yl)-1-methyl-1,4-dihydropyrido[2,3-b]pyrazine-2,3-dione

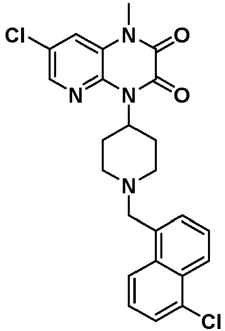

7-Chloro-1-methyl-4-(piperidin-4-yl)-1,4-dihydropyrido[2,3-b]pyrazine-2,3-dione dihydrochloride (50 mg, 0.170 mmol) obtained in Preparation Example 2 and 5-chloro-1-naphthaldehyde (32.3 mg, 0.170 mmol) were used in a similar manner to Example 1 to obtain the title compound (48 mg).
¹H-NMR (400 MHz, CHLOROFORM-D) δ 8.31 (d, J = 8.7 Hz, 1H), 8.26-8.22 (m, 1H), 8.19 (d, J = 2.3 Hz, 1H), 7.59 (dd, J = 7.3, 0.9 Hz, 1H), 7.53 (dd, J = 14.0, 6.2 Hz, 2H), 7.44 (dd, J = 8.9, 7.1 Hz, 2H), 5.38-5.32 (m, 1H), 3.98 (s, 2H), 3.58 (s, 3H), 3.06 (d, J = 11.4 Hz, 2H), 2.96 (qd, J = 12.3, 4.2 Hz, 2H), 2.32-2.27 (m, 2H), 1.58 (d, J = 11.4 Hz, 2H)
LC/MS: 470 (M+H)

### Example 36: Preparation of 1-methyl-7-(1-methyl-1H-pyrazol-4-yl)-4-(1-(4-(trifluoromethoxy)benzyl)piperidin-4-yl)-1,4-dihydropyrido[2,3-b]pyrazine-2,3-dione

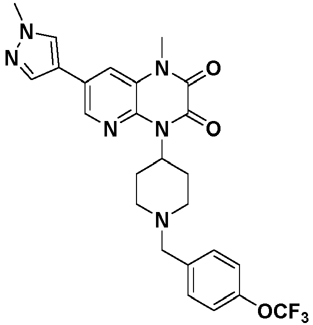

7-Bromo-1-methyl-4-(1-(4-(trifluoromethoxy)benzyl)piperidin-4-yl)-1,4-dihydropyrido[2, 3-b]pyrazine-2,3-dione (30 mg, 0.058 mmol) and 1-methyl-4-(3,3,4,4-tetramethylborolan-1-yl)-1H-pyrazole (11.93 mg, 0.058 mmol) were dissolved in DME, and sodium carbonate aqueous solution (88 µl, 0.175 mmol) and tetrakis (6.75 mg, 5.84 µmol) were added thereto, followed by stirring at 110°C under nitrogen gas for 12 hours. After completion of the reaction, the reaction mixture was filtered with Celite and extracted three times with EtOAc. The organic layer was dried over anhydrous magnesium sulfate and concentrated under reduced pressure. The residue was purified by MPLC to obtain the title compound (2 mg).
¹H-NMR (400 MHz, METHANOL-D4) δ 8.46 (d, J = 2.1 Hz, 1H), 8.12 (d, J = 2.3 Hz, 1H), 7.95 (d, J = 2.3 Hz, 1H), 7.86 (s, 1H), 7.70 (d, J = 8.7 Hz, 1H), 7.50 (d, J = 8.2 Hz, 1H), 7.33 (d, J = 7.8 Hz, 1H), 7.26 (d, J = 7.8 Hz, 1H), 4.54 (s, 1H), 4.48 (s, 1H), 3.93 (s, 3H), 3.72 (d, J = 21.5 Hz, 2H), 3.65 (s, 3H), 3.63 (s, 1H), 3.12 (d, J = 13.7 Hz, 3H), 2.40 (s, 1H), 1.71 (t, J = 10.3 Hz, 2H)
LC/MS: 515 (M+H)

### Example 37: Preparation of 7-chloro-1-methyl-4-(1-((5,6,7,8-tetrahydronaphthalen-1-yl)methyl)piperidin-4-yl)-1,4-dihydropyrido[2,3-b]pyrazine-2,3-dione

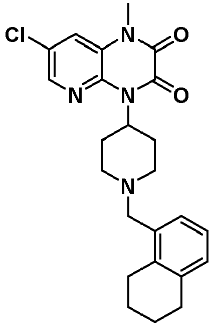

7-Chloro-1-methyl-4-(piperidin-4-yl)-1,4-dihydropyrido[2,3-b]pyrazine-2,3-dione dihydrochloride (50 mg, 0.170 mmol) obtained in Preparation Example 2 and 5,6,7,8-tetrahydronaphthalene-1-carbaldehyde (0.025 ml, 0.170 mmol) were used in a similar manner to Example 1 to obtain the title compound (41 mg).
¹H-NMR (400 MHz, CHLOROFORM-D) δ 8.20 (d, J = 2.3 Hz, 1H), 7.44 (d, J = 1.8 Hz, 1H), 7.13 (d, J = 7.3 Hz, 1H), 7.05 (t, J = 7.3 Hz, 1H), 6.98 (d, J = 7.3 Hz, 1H), 5.32 (tt, J = 12.0, 4.0 Hz, 1H), 3.59 (s, 3H), 3.45 (s, 2H), 3.02-2.88 (m, 4H), 2.81 (dt, J = 15.6, 6.3 Hz, 4H), 2.21-2.12 (m, 2H), 1.86-1.74 (m, 4H), 1.57 (d, J = 15.6 Hz, 2H)
LC/MS: 439 (M+H)

### Example 38: Preparation of 7-bromo-1-methyl-2,3-dioxo-4-(1-(4-(trifluoromethoxy)benzyl)piperidin-4-yl)-1,2,3,4-tetrahydropyrido[2,3-b]pyrazine-6-carbonitrile

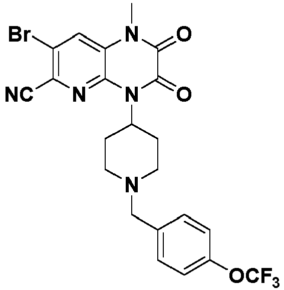

7-Bromo-1-methyl-2,3-dioxo-4-(piperidin-4-yl)-1,2,3,4-tetrahydropyrido[2,3-b]pyrazine-6-carbonitrile dihydrochloride (55 mg, 0.126 mmol) obtained in Preparation Example 10 and 4-(trifluoromethoxy)benzaldehyde (68 mg, 0.36 mmol) were used in a similar manner to Example 1 to obtain the title compound (57 mg).
¹H NMR (500 MHz, CHLOROFORM-D) δ 7.70 (s, 1H), 7.43 (d, J = 8.5 Hz, 2H), 7.20 (d, J = 8.5 Hz, 2H), 5.29~5.23 (m, 1H), 3.65 (s, 3H), 3.61(s, 2H), 3.05~2.89 (m, 4H), 2.28-2.23 (m, 2H), 1.63-1.61 (m, 2H)
LC/MS: 538, 540 (M+H)

### Example 39: Preparation of 7-(2-fluorophenyl)-1-methyl-4-(1-(4-(trifluoromethoxy)benzyl)piperidin-4-yl)-1,4-dihydropyrido[2,3-b]pyrazine-2,3-dione

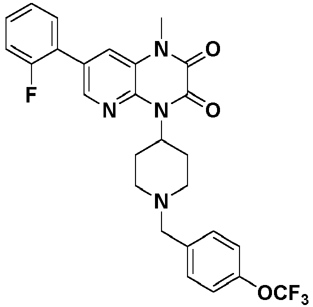

7-Bromo-1-methyl-4-(1-(4-(trifluoromethoxy)benzyl)piperidin-4-yl)-1,4-dihydropyrido[2,3-b]pyrazine-2,3-dione (30 mg, 0.058 mmol) obtained in Example 35 and (2-fluorophenyl)boronic acid (9.81 mg, 0.070 mmol) were used in a similar manner to Example 38 to obtain the title compound (21 mg).
¹H-NMR (400 MHz, METHANOL-D4) δ 8.43 (t, J = 1.6 Hz, 1H), 7.90 (d, J = 0.9 Hz, 1H), 7.65-7.58 (m, 4H), 7.55-7.51 (m, 2H), 7.48 (d, J = 8.7 Hz, 2H), 7.45-7.41 (m, 1H), 7.33-7.27 (m, 2H), 7.23 (d, J = 8.2 Hz, 3H), 5.52 (s, 1H), 3.28 (m, 3H), 3.06-2.97 (m, 4H), 2.27 (t, J = 11.7 Hz, 2H), 1.70 (d, J = 10.5 Hz, 2H)
LC/MS: 529 (M+H)

### Example 40: Preparation of 7-cyclopropyl-1-methyl-4-(1-(4-(trifluoromethoxy)benzyl)piperidin-4-yl)-1,4-dihydropyrido[2,3-b]pyrazine-2,3-dione

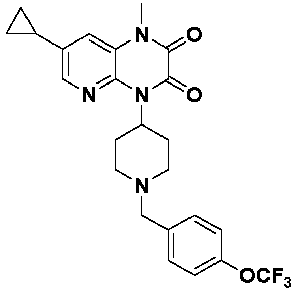

7-Bromo-1-methyl-4-(1-(4-(trifluoromethoxy)benzyl)piperidin-4-yl)-1,4-dihydropyrido[2,3-b]pyrazine-2,3-dione (30 mg, 0.058 mmol) obtained in Example 35 and cyclopropylboronic acid (6.02 mg, 0.070 mmol) were dissolved in toluene, and potassium phosphate tribasic (37.2 mg, 0.175 mmol), tricyclohexylphosphine (5.84 µl, 3.51 µmol) and Pd(OAc)₂ (0.787 mg, 3.51 µmol) were added thereto, followed by stirring at 100°C under nitrogen gas for 12 hours. After completion of the reaction, the reaction mixture was filtered with Celite and extracted three times with EtOAc. The organic layer was dried over anhydrous magnesium sulfate and concentrated under reduced pressure. The residue was purified by MPLC to obtain the title compound (4.2 mg).
¹H-NMR (400 MHz, CHLOROFORM-D) δ 8.04 (d, J = 1.8 Hz, 1H), 7.39 (d, J = 8.2 Hz, 2H), 7.16 (d, J = 8.2 Hz, 2H), 7.12 (d, J = 1.8 Hz, 1H), 5.36 (s, 1H), 3.59 (s, 3H), 3.56 (s, 2H), 3.05-2.98 (m, 4H), 2.23-2.16 (m, 2H), 1.99-1.93 (m, 1H), 1.10-1.05 (m, 2H), 0.74 (td, J = 5.7, 4.6 Hz, 2H)
LC/MS: 475 (M+H)

### Example 41: Preparation of 7-chloro-6-(2-fluorophenyl)-1-methyl-4-(1-(4-(trifluoromethoxy)benzyl)piperidin-4-yl)-1,4-dihydropyrido[2,3-b]pyrazine-2,3-dione

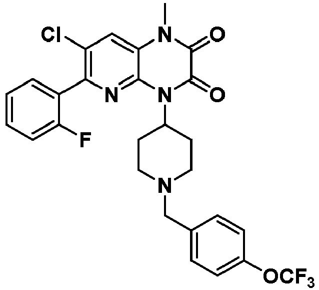

7-Chloro-6-(2-fluorophenyl)-1 -methyl-4-(piperidin-4-yl)-1,4-dihydropyrido [2,3-b]pyrazine-2,3-dione hydrochloride (45 mg, 0.106 mmol) obtained in Preparation Example 11 and 4-(trifluoromethoxy)benzaldehyde (68 mg, 0.36 mmol) were used in a similar manner to Example 1 to obtain the title compound (49 mg ).
¹H NMR (500 MHz, CHLOROFORM-D) δ 7.62 (s, 1H), 7.56-7.52 (m, 2H), 736~7.33(m, 3H), 7.28-7.24 (m, 1H), 7.15 (d, J= 8.5 Hz, 2H), 5.36-5.32 (m, 1H), 3.67 (s, 3H), 3.54(s, 2H), 3.04-2.95 (m, 4H), 2.24-2.19 (m, 2H), 1.68-1.61 (m, 2H)
LC/MS: 563 (M+H)

### Example 42: Preparation of 7-chloro-4-(1-(3-chloro-4-fluorobenzyl)piperidin-4-yl)-1-methyl-1,4-dihydropyrido[2,3-b]pyrazine-2,3-dione

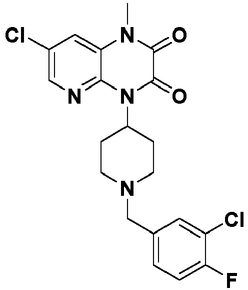

7-Chloro-1-methyl-4-(piperidin-4-yl)-1,4-dihydropyrido[2,3-b]pyrazine-2,3-dione dihydrochloride (40 mg, 0.109 mmol) obtained in Preparation Example 2 and 3-chloro-4-fluorobenzaldehyde (0.013 mL, 0.109 mmol) were used in a similar manner to Example 1 to obtain the title compound (34 mg).
¹H-NMR (400 MHz, CHLOROFORM-D) δ 8.35 (s, 1H), 8.20 (d, J = 1.8 Hz, 1H), 7.47 (d, J= 1.8 Hz, 1H), 7.44 (dd, J = 7.1, 2.1 Hz, 1H), 7.28 (qd, J = 4.4, 2.3 Hz, 1H), 7.11 (t, J = 8.5 Hz, 1H), 5.41-5.34 (m, 1H), 3.72 (s, 2H), 3.59 (s, 3H), 3.15-3.03 (m, 4H), 2.45-2.39 (m, 2H), 1.64 (d, J = 11.4 Hz, 2H)
LC/MS: 438 (M+H)

### Example 43: Preparation of 7-chloro-4-(1-(3-chloro-4-(trifluoromethoxv)benzvl) piperidin-4-yl)-1-methyl-1,4-dihydropyrido[2,3-b]pyrazine-2,3-dione

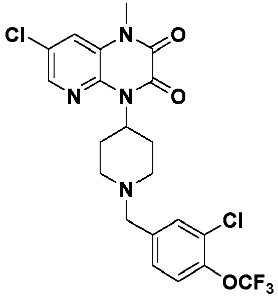

7-Chloro-1-methyl-4-(piperidin-4-yl)-1,4-dihydropyrido[2,3-b]pyrazine-2,3-dione dihydrochloride (40 mg, 0.109 mmol) obtained in Preparation Example 2 and 3-chloro-4-(trifluoromethoxy)benzaldehyde (0.017 mL, 0.109 mmol) were used in a similar manner to Example 1 to obtain the title compound (45 mg).
¹H-NMR (400 MHz, CHLOROFORM-D) δ 8.21 (d, J = 2.3 Hz, 1H), 7.50 (d, J = 1.8 Hz, 1H), 7.46 (d, J = 1.8 Hz, 1H), 7.29 (qd, J = 8.8, 1.6 Hz, 2H), 5.37-5.31 (m, 1H), 3.59 (d, J = 1.8 Hz, 5H), 3.06-2.96 (m, 4H), 2.30-2.24 (m, 2H), 1.63-1.59 (m, 2H)
LC/MS: 504 (M+H)

### Example 44: Preparation of 7-cyclopropyl-1-methyl-2,3-dioxo-4-(1-(4-(trifluoromethoxy)benzyl)piperidin-4-yl)-1,2,3,4-tetrahydropyrido[2,3-b]pyrazine-6-carbonitrile

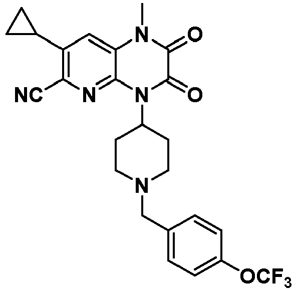

7-Bromo-1-methyl-2,3-dioxo-4-(1-(4-(trifluoromethoxy)benzyl)piperidin-4-yl)-1,2,3,4-tetrahydropyrido[2,3-b]pyrazine-6-carbonitrile (30 mg, 0.058 mmol) obtained in Example 40 was used in a similar manner to Example 1 to obtain the title compound (14 mg).
¹H-NMR (400 MHz, METHANOL-D4) δ 7.47 (d, J = 8.7 Hz, 2H), 7.22 (d, J = 7.3 Hz, 3H), 5.30 (s, 1H), 3.62 (d, J= 5.0 Hz, 2H), 3.58 (d, J = 5.5 Hz, 3H), 3.02 (d, J= 11.4 Hz, 2H), 2.93-2.90 (m, 2H), 2.29-2.23 (m, 3H), 1.66 (d, J= 11.9 Hz, 2H), 1.24-1.19 (m, 2H), 0.96 (t, J = 5.0 Hz, 2H)
LC/MS: 500 (M+H)

### Example 45: Preparation of 4-(1-(bis(3-chlorophenyl)methyl)piperidin-4-yl)-7-chloro-1-methyl-1,4-dihydropyrido[2,3-b]pyrazine-2,3-dione

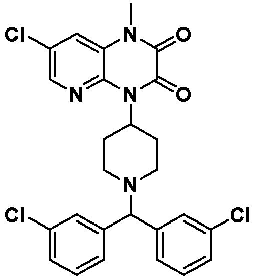

7-Chloro-1-methyl-4-(piperidin-4-yl)-1,4-dihydropyrido[2,3-b]pyrazine-2,3-dione dihydrochloride (40 mg, 0.11 mmol) obtained in Preparation Example 2, 3,3'-(chloromethylene)bis(chlorobenzene) (44 mg, 0.16 mmol) and potassium carbonate (45 mg, 0.33 mmol) were dissolved in acetonitrile (2 mL), refluxed at 85°C and stirred for 4 hours. The reaction mixture was diluted with EtOAc and washed with brine, and the organic layer was dried over anhydrous magnesium sulfate and concentrated under reduced pressure. The residue was purified by MPLC to obtain the title compound (30 mg).
¹H-NMR (500 MHz, CHLOROFORM-D) δ 8.24 (s, 1H), 7.49-7.28 (m, 8H), 5.36 (t, J = 11.4 Hz, 1H), 4.42 (s, 1H), 3.62 (s, 3H), 3.02-2.96 (m, 4H), 2.14-2.07 (m, 3H), 1.56 (d, J = 9.8 Hz, 2H)
LC/MS: 529 (M+H)

### Example 46: Preparation of 7-chloro-4-(1-((2,3-dihydrobenzo[b][1,4]dioxin-6-yl)methyl)piperidin-4-yl)-1-methyl-1,4-dihydropyrido[2,3-b]pyrazine-2,3-dione

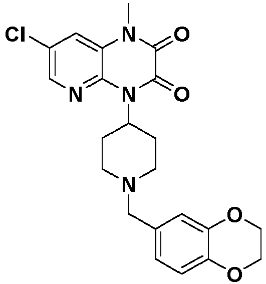

7-Chloro-1-methyl-4-(piperidin-4-yl)-1,4-dihydropyrido[2,3-b]pyrazine-2,3-dione dihydrochloride (30 mg, 0.08 mmol) obtained in Preparation Example 2 and 2,3-dihydrobenzo[b][1,4]dioxine-6-carbaldehyde (80 mg, 0.49 mmol) were used in a similar manner to Example 1 to obtain the title compound (28 mg).
¹H-NMR (500 MHz, CHLOROFORM-D) δ 8.21 (d, J = 1.5 Hz, 1H), 7.48 (d, J = 1.8 Hz, 1H), 6.98 (d, J = 7.0 Hz, 1H), 6.85-6.79 (m, 2H), 5.32-5.27 (m, 1H), 4.28 (d, J = 3.1 Hz, 4H), 3.67 (s, 2H), 3.62 (d, J = 10.7 Hz, 3H), 3.50 (s, 2H), 3.12-3.00 (m, 4H), 2.31 (t, J = 11.4 Hz, 2H), 1.61 (d, J = 11.6 Hz, 2H)
LC/MS: 443 (M+H)

### Example 47: Preparation of 1-methyl-2,3-dioxo-4-1-(4-(trifluoromethoxy)benzyl)piperidin-4-yl)-1,2,3,4-tetrahydropyrido[2,3-b]pyrazine-7-carbonitrile

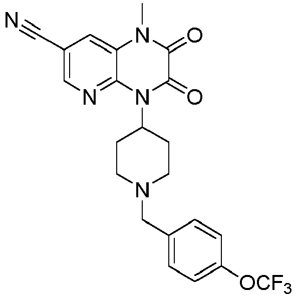

1-Methyl-2,3-dioxo-4-(piperidin-4-yl)-1,2,3,4-tetrahydropyrido[2,3-b]pyrazine-7-carbonitrile dihydrochloride (40 mg, 0.112 mmol) obtained in Preparation Example 8 and 4-(trifluoromethoxy)benzaldehyde (42.5 mg, 0.223 mmol) were used in a similar manner to Example 1 to obtain the title compound (32 mg).
¹H NMR (500 MHz, CHLOROFORM-D) δ 8.56 (d, J = 1.5 Hz, 2H), 7.68 (d, J = 1.5 Hz, 1H), 7.42 (d, J = 8.5 Hz, 2H), 7.20 (d, J = 8.0 Hz, 1H), 5.39-5.35 (m, 1H), 3.66 (s, 3H), 3.60 (s, 2H), 3.05-2.97 (m, 4H), 2.25-2.20 (m, 2H), 1.65-1.62 (m, 2H)
LC/MS: 460 (M+H)

### Example 48: Preparation of 7-acetyl-1-methyl-4-(1-(4-(trifluoromethoxy)benzyl)piperidin-4-yl)-1,4-dihydropyrido[2,3-b]pyrazine-2,3-dione

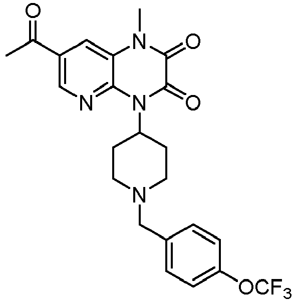

7-Acetyl-1-methyl-4-(piperidin-4-yl)-1,4-dihydropyrido[2,3-b]pyrazine-2,3-dione dihydrochloride (65 mg, 0.173 mmol) obtained in Preparation Example 9 and 4-(trifluoromethoxy)benzaldehyde (42.5 mg, 0.223 mmol) were used in a similar manner to Example 1 to obtain the title compound (62 mg).
¹H NMR (500 MHz, CHLOROFORM-D) δ 8.85 (d, J = 1.5 Hz, 2H), 8.06 (d, J = 1.5 Hz, 1H), 7.44 (d, J = 8.5 Hz, 2H), 7.21 (d, J = 8.0 Hz, 1H), 5.47-5.42 (m, 1H), 3.70 (s, 3H), 3.62 (s, 2H), 3.06-3.04(m, 4H), 2.71 (s, 3H), 2.28-2.23 (m, 2H), 1.66-1.65 (m, 2H)
LC/MS: 477 (M+H)

### Example 49: Preparation of 7-chloro-4-((2R,5S)-2,5-dimethyl-1-(4-trifluoromethoxy)benzyl)piperidin-4-yl)-1-methyl-1,4-dihydropyrido[2,3-b] pyrazine-2,3-dione

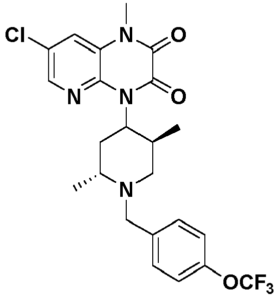

7-Chloro-4-((2R,5S)-2,5-dimethylpiperidin-4-yl)-1-methyl-1,4-dihydropyrido[2,3-b]pyrazine-2,3-dione dihydrochloride (34 mg, 0.095 mmol) obtained in Preparation Example 17 and 4-(trifluoromethoxy)benzaldehyde (42.5 mg, 0.223 mmol) were used in a similar manner to Example 1 to obtain the title compound (34 mg, 72%).
¹H-NMR (500 MHz, CHLOROFORM-D) δ 8.20 (d, J = 2.5 Hz, 1H), 7.49 (d, J = 2.0 Hz, 1H), 7.42 (d, J 8.5 Hz, 2H), 7.18 (d, J= 8.0 Hz, 2H), 5.35-5.31 (m, 1H), 4.19 (d, J = 14.0 Hz, 1H), 3.62 (s, 3H), 3.48-3.41 (m, 1H), 3.07-3.04 (m, 1H), 2.66-2.64 (m, 1H), 2.41-2.34 (m, 2H), 2.28-2.26 (m, 1H), 1.69-1.66 (m, 1H), 1.27 (d, J = 6.0 Hz, 3H), 1.12 (d, J = 6.4 Hz, 3H)
LC/MS: 497 (M+H)

### Example 50: Preparation of 7-chloro-1,6-dimethyl-4-(1-(4-(trifluoromethoxy)benzyl)piperidin-4-yl)-1,4-dihydropyrido[2,3-b]pyrazine-2,3-dione

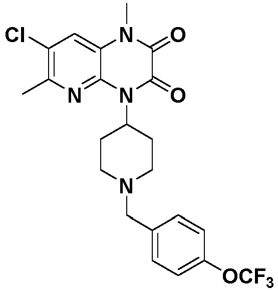

7-Chloro-1,6-dimethyl-4-(piperidin-4-yl)-1,4-dihydropyrido[2,3-b]pyrazine-2,3-dione hydrochloride (50 mg, 0.145 mmol) obtained in Preparation Example 18 and 4-(trifluoromethoxy)benzaldehyde (83 mg, 0.435 mmol) were used in a similar manner to Example 1 to obtain the title compound (58 mg, 83%).
¹H-NMR (500 MHz, CHLOROFORM-D) δ 7.45 (s, 1H), 7.43 (d, J 8.5 Hz, 2H), 7.20 (d, J = 8.0 Hz, 2H), 5.39-5.35 (m, 1H), 3.61 (s, 3H), 3.60 (s, 2H), 3.05-2.99 (m, 4H), 2.65 (s, 3H), 2.26-2.21 (m, 2H), 1.62-1.60 (m, 2H)
LC/MS: 483 (M+H)

### Example 51: Preparation of 7-bromo-1,6-dimethyl-4-(1-(4-(trifluoromethoxy)benzyl)piperidin-4-yl)-1,4-dihydropyrido[2,3-b]pyrazine-2,3-dione

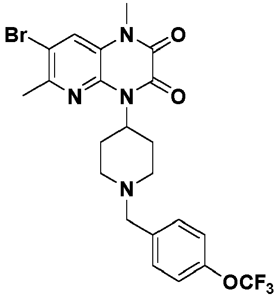

7-Bromo-1,6-dimethyl-4-(piperidin-4-yl)-1,4-dihydropyrido[2,3-b]pyrazine-2,3-dione hydrochloride (49 mg, 0.126 mmol) obtained in Preparation Example 19 and 4-(trifluoromethoxy)benzaldehyde (72 mg, 0.377 mmol) were used in a similar manner to Example 1 to obtain the title compound (54 mg, 81%).
¹H-NMR (500 MHz, CHLOROFORM-D) δ 7.60 (s, 1H), 7.43 (d, J = 8.5 Hz, 2H), 7.20 (d, J = 8.0 Hz, 2H), 5.39-5.34 (m, 1H), 3.61 (s, 3H), 3.60 (s, 2H), 3.06-3.01 (m, 4H), 2.69 (s, 3H), 2.25-2.21 (m, 2H), 1.65-1.60 (m, 2H)
LC/MS: 527, 529 (M+H)

### Example 52: Preparation of 7-chloro-4-(1-((1-isopropyl-1H-pyrazol-4-yl)methyl)piperidin-4-yl)-1-methyl-1,4-dihydropyrido[2,3-b]pyrazine-2,3-dione

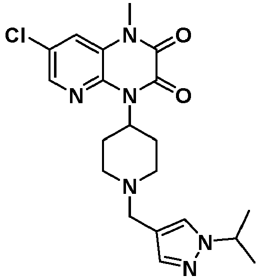

7-Chloro-1-methyl-4-(piperidin-4-yl)-1,4-dihydropyrido[2,3-b]pyrazine-2,3-dione hydrochloride obtained in Preparation Example 2 (49 mg, 0.148 mmol) and 1-isopropyl-1H-pyrazole-4-carbaldehyde (51 mg, 0.370 mmol) were used in a similar manner to Example 1 to obtain the title compound (42 mg, 68%).
¹H-NMR (500 MHz, CHLOROFORM-D) δ 8.21 (d, J = 2.0 Hz, 1H), 7.520-7.41 (m, 4H), 5.31~5.26 (m, 1H), 4.52~4.47 (m, 1H), 3.62 (s, 3H), 3.53 (s, 2H), 3.09-2.97 (m, 4H), 2.20-2.15 (m, 2H), 1.64-1.62 (m, 2H), 1.52 (t, J = 5.0 Hz, 6H)
LC/MS: 417 (M+H)

### Example 53: Preparation of 6-acetyl-1-methyl-4-(1-(4-(trifluoromethoxy)benzyl)piperidin-4-yl)-1,4-dihydropyrido[2,3-b]pyrazine-2,3-dione

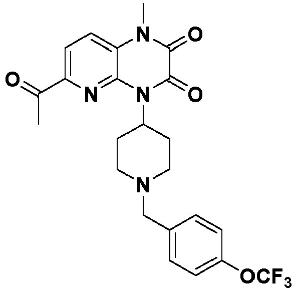

6-Acetyl-1-methyl-4-(piperidin-4-yl)-1,4-dihydropyrido[2,3-b]pyrazine-2,3-dione hydrochloride (48 mg, 0.142 mmol) obtained in Preparation Example 20 and 4-(trifluoromethoxy)benzaldehyde (67 mg, 0.354 mmol) were used in a similar manner to Example 1 to obtain the title compound (19 mg, 28%).
¹H-NMR (500 MHz, CHLOROFORM-D) δ 8.01 (d, J = 8.5 Hz, 1H), 7.62 (d, J = 8.5 Hz, 1H), 7.41 (d, J = 8.5 Hz, 2H), 7.19 (d, J = 8.0 Hz, 2H), 5.43-5.37 (m, 1H), 3.69 (s, 3H), 3.58 (s, 2H), 3.14-3.06 (m, 4H), 2.83 (s, 3H), 2.25-2.21 (m, 2H), 1.73-1.71 (m, 2H)
LC/MS: 477 (M+H)

### Example 54: Preparation of 6-chloro-1-methyl-4-(1-(4-(trifluoromethoxy)benzyl)piperidin-4-yl)-1,4-dihydropyrido[2,3-b]pyrazine-2,3-dione

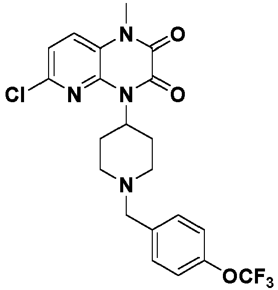

6-Chloro-1-methyl-4-(piperidin-4-yl)-1,4-dihydropyrido[2,3-b]pyrazine-2,3-dione hydrochloride (30 mg, 0.10 mmol) obtained in Preparation Example 21 and 4-(trifluoromethoxy)benzaldehyde (39 mg, 0.21 mmol) were used in a similar manner to Example 1 to obtain the title compound (15 mg, 31%).
¹H-NMR (400 MHz, CHLOROFORM-D) δ 7.42 (m, J = 8.4 Hz, 3H), 7.25-7.15 (m, 3H), 5.30-5.24 (m, 1H), 3.60 (s, 3H), 3.58 (s, 2H), 3.00-2.92 (m, 4H), 2.27-2.08 (m, 2H), 1.60-1.57 (m, 2H)
LC/MS: 469.1 (M+H), 470.1 (M+2H), 471.1 (M+3H)

### Example 55: Preparation of 6-methoxy-1-methyl-4-(1-(4-(trifluoromethoxy)benzyl)piperidin-4-yl)-1,4-dihydropyrido[2,3-b]pyrazine-2,3-dione

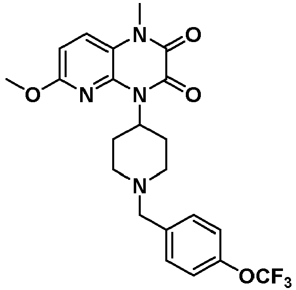

6-Methoxy-1-methyl-4-(piperidin-4-yl)-1,4-dihydropyrido[2,3-b]pyrazine-2,3-dione dihydrochloride (30 mg, 0.10 mmol) obtained in Preparation Example 22 and 4-(trifluoromethoxy)benzaldehyde (39 mg, 0.21 mmol) were used in a similar manner to Example 1 to obtain the title compound (21 mg, 44%).
¹H-NMR (400 MHz, CHLOROFORM-D) δ 7.45 (d, J = 8.7 Hz, 1H), 7.35 (d, J = 8.2 Hz, 2H), 7.15 (d, J = 8.2 Hz, 2H), 6.65 (d, J = 8.7 Hz, 1H), 5.32-5.26 (m, 1H), 4.02 (s, 3H), 3.58 (d, J = 5.9 Hz, 4H), 3.53 (s, 2H), 3.10-2.97 (m, 3H), 2.16 (t, J = 11.9 Hz, 2H), 1.64 (d, J= 11.4 Hz, 2H)
LC/MS: 465.1 (M+H)

### Example 56: Preparation of 1,8-dimethyl-4-(1-(4-(trifluoromethoxy)benzyl)piperidin-4-yl)-1,4-dihydropyrido[2,3-b]pyrazine-2,3-dione

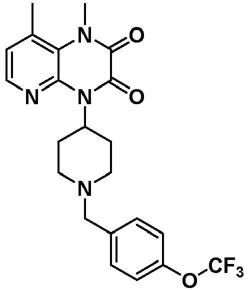

1,8-Dimethyl-4-(piperidin-4-yl)-1,4-dihydropyrido[2,3-b]pyrazine-2,3-dione hydrochloride obtained in Preparation 23 (15 mg, 0.048 mmol) and 4-(trifluoromethoxy)benzaldehyde (18 mg, 0.097 mmol) were used in a similar manner to Example 1 to obtain the title compound (18 mg, 83%).
¹H-NMR (500 MHz, CHLOROFORM-D) δ 8.29 (d, J = 5.0 Hz, 1H), 7.44 (d, J = 8.5 Hz, 2H), 7.20 (d, J = 8.5 Hz, 2H), 7.12 (d, J = 4.5 Hz, 1H), 5.60 (br s, 1H), 4.11 (s, 3H), 3.63 (s, 2H), 3.15-3.13 (m, 2H), 3.05-3.03 (m, 2H), 2.61 (s, 3H), 2.30-2.26 (m, 2H), 1.64~1.61 (m, 2H),
LC/MS: 449 (M+H)

### Example 57: Preparation of 7-chloro-1-methyl-4-((1R,3r,5S)-8-(4-(trifluoromethoxy)benzyl)-8-azabicyclo[3.2.1]octan-3-yl)-1,4-dihydropyrido[2,3-b]pyrazine-2,3-dione

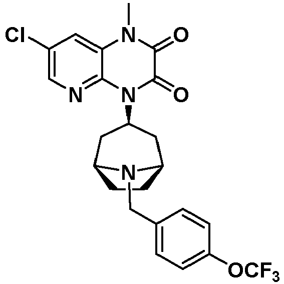

4-((1R,3r,5S)-8-azabicyclo[3.2.1] octan-3-yl)-7-chloro-1-methyl-1,4-dihydropyrido[2,3-b]pyrazine-2,3-dione hydrochloride (30 mg, 0.084 mmol) obtained in Preparation Example 24 and 4-(trifluoromethoxy)benzaldehyde (48 mg, 0.25 mmol) were used in a similar manner to Example 1 to obtain the title compound (42 mg, 84%).
¹H-NMR (500 MHz, CHLOROFORM-D) δ 8.27 (s, 1H), 7.54 (d, J = 7.0 Hz, 2H), 7.51 (s, 1H), 7.18 (d, J = 8.2 Hz, 2H), 5.94-5.90 (m, 1H), 4.10 (s, 2H), 3.63 (d, J = 12.8 Hz, 3H), 3.37 (s, 2H), 3.01-2.96 (m, 2H), 2.12 (s, 2H), 1.83 (d, J= 7.6 Hz, 2H), 1.34-1.29 (m, 2H)
LC/MS: 495.1 (M+H), 517.1 (M+Na)

### Example 58: Preparation of 6-isopropoxy-1-methyl-4-(1-(4-(trifluoromethoxy)benzyl)piperidin-4-yl)-1,4-dihydropyrido[2,3-b]pyrazine-2,3-dione

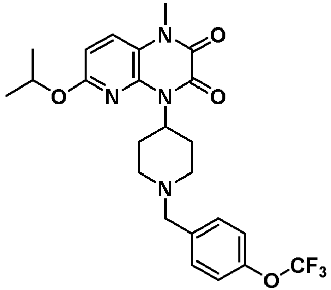

6-Isopropoxy-1-methyl-4-(piperidin-4-yl)-1,4-dihydropyrido[2,3-b]pyrazine-2,3-dione dihydrochloride (30 mg, 0.094 mmol) obtained in Preparation Example 25 and 4-(trifluoromethoxy)benzaldehyde (19.71 mg, 0.104 mmol) were used in a similar manner to Example 1 to obtain the title compound (28 mg).
¹H-NMR (400 MHz, CHLOROFORM-D) δ 7.43 (d, J = 8.7 Hz, 1H), 7.37 (d, J = 8.7 Hz, 2H), 7.15 (d, J = 7.8 Hz, 2H), 6.58 (d, J = 8.7 Hz, 1H), 5.29 (dd, J = 11.4, 5.0 Hz, 2H), 3.58 (s, 3H), 3.53 (s, 2H), 3.04-2.98 (m, 4H), 2.18-2.12 (m, 2H), 1.64-1.61 (m, 2H), 1.40 (d, J = 5.9 Hz, 6H)
LC/MS: 493 (M+H)

### Example 59: Preparation of 7-fluoro-1-methyl-4-(1-(4-(trifluoromethoxy)benzyl)piperidin-4-yl)-1,4-dihydropyrido[2,3-b]pyrazine-2,3-dione

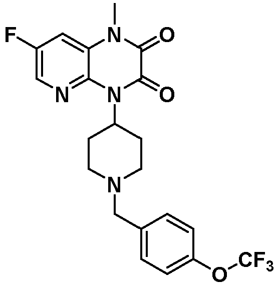

7-Fluoro-1-methyl-4-(piperidin-4-yl)-1,4-dihydropyrido[2,3-b]pyrazine-2,3-dione (30 mg, 0.108 mmol) obtained in Preparation Example 26 and 4-(trifluoromethoxy)benzaldehyde (22.55 mg, 0.119 mmol) were used in a similar manner to Example 1 to obtain the title compound (38 mg).
¹H-NMR (400 MHz, CHLOROFORM-D) δ 8.13 (d, J = 2.3 Hz, 1H), 7.39 (d, J = 8.2 Hz, 2H), 7.24 (dd, J = 9.1, 2.3 Hz, 1H), 7.16 (d, J = 7.8 Hz, 2H), 5.35-5.29 (m, 1H), 3.58 (s, 3H), 3.56 (s, 2H), 3.03-2.93 (m, 4H), 2.22-2.16 (m, 2H), 1.58 (s, 2H)
LC/MS: 453 (M+H)

### Example 60: Preparation of methyl 1-methyl-2,3-dioxo-4-(1-(4-(trifluoromethoxy)benzyl)piperidin-4-yl)-1,2,3,4-tetrahydropyrido[2,3-b]pyrazine-7-carboxylate

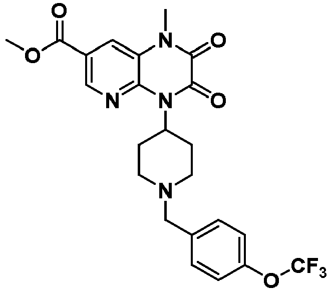

Methyl 1-methyl-2,3-dioxo-4- (piperidin-4-yl)-1,2,3,4-tetrahydropyrido[2,3-b] pyrazine-7-carboxylate dihydrochloride (300 mg, 0.77 mmol) obtained in Preparation Example 27 and 4-(trifluoromethoxy)benzaldehyde (292 mg, 1.53 mmol) were used in a similar manner to Example 1 to obtain the title compound (261 mg, 69%).
¹H-NMR (500 MHz, CHLOROFORM-D) δ 8.89 (d, J = 5.5 Hz, 1H), 8.07 (d, J = 6.1 Hz, 1H), 7.43 (d, J = 7.6 Hz, 2H), 7.20 (d, J = 7.9 Hz, 2H), 5.44 (t, J = 11.4 Hz, 1H), 4.01 (s, 3H), 3.69 (s, 3H), 3.60 (s, 2H), 3.04-2.99 (m, 4H), 2.24 (t, J = 11.9 Hz, 2H), 1.64 (d, J = 9.2 Hz, 2H)
LC/MS: 493.2 (M+H)

### Example 61: Preparation of 1,7-dimethyl-4-(1-(4-(trifluoromethoxy)benzyl)piperidin-4-yl)-1,4-dihydropyrido[2,3-b]pyrazine-2,3-dione

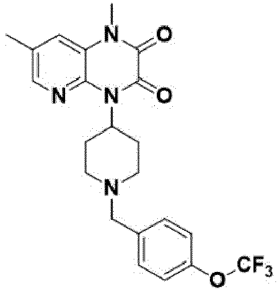

1,7-Dimethyl-4-(piperidin-4-yl)-1,4-dihydropyrido[2,3-b]pyrazine-2,3-dione dihydrochloride (50 mg, 0.14 mmol) obtained in Preparation Example 28 and 4-(trifluoromethoxy)benzaldehyde (82 mg, 0.43 mmol) were used in a similar manner to Example 1 to obtain the title compound (58 mg, 90%).
¹H-NMR (500 MHz, CHLOROFORM-D) δ 8.08 (s, 1H), 7.45 (d, J = 7.9 Hz, 2H), 7.30 (s, 1H), 7.20 (d, J = 7.9 Hz, 2H), 5.43 (t, J= 11.6 Hz, 1H), 3.68 (s, 2H), 3.63 (s, 3H), 3.13-3.07 (m, 4H), 2.43 (s, 3H), 2.33 (t, J = 12.1 Hz, 2H), 1.64 (d, J = 11.3 Hz, 2H)
LC/MS: 449.2 (M+H), 450.2 (M+2H)

### Example 62: Preparation of 1-methyl-2,3-dioxo-4-(1-(4-(trifluoromethoxy)benzyl)piperidin-4-yl)-1,2,3,4-tetrahydropyrido[2,3-b]pyrazine-7-carboxylic acid

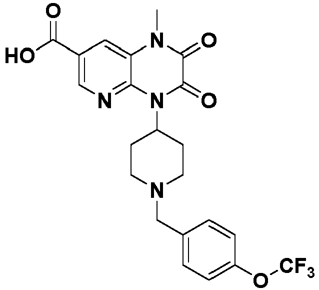

Methyl 1-methyl-2,3-dioxo-4-(1-(4-(trifluoromethoxy)benzyl)piperidin-4-yl)-1,2,3,4-tetrahydropyrido[2,3-b]pyrazine-7-carboxylate (230 mg, 0.47 mmol) obtained in Example 60 was used in a similar manner to Step A of Preparation Example 29 to obtain the title compound (65 mg, 29%).
¹H-NMR (500 MHz, DMSO-D6) δ 8.73 (s, 1H), 8.05 (s, 1H), 7.48 (d, J = 8.2 Hz, 2H), 7.35 (d, J = 7.9 Hz, 2H), 5.33 (s, 1H), 3.58 (s, 2H), 3.54 (s, 3H), 2.95 (d, J = 10.1 Hz, 2H), 2.77 (d, J = 11.9 Hz, 2H), 2.13 (t, J= 11.9 Hz, 2H), 1.60 (d, J = 11.0 Hz, 2H)
LC/MS: 479.1 (M+H), 480.2 (M+2H)

### Example 63: Preparation of N-(2-(dimethylamino)ethyl)-1-methyl-2,3-dioxo-4-(1-(4-(trifluoromethoxy)benzyl)piperidin-4-yl)-1,2,3,4-tetrahydropyrido[2,3-blpyrazine-7-carboxamide

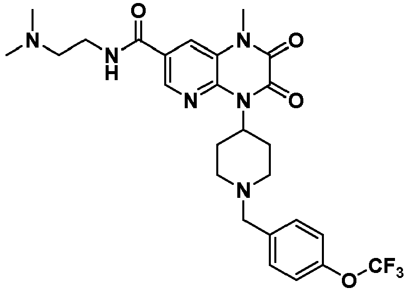

1-Methyl-2,3-dioxo-4-(1-(4-(trifluoromethoxy)benzyl)piperidin-4-yl)-1,2,3,4-tetrahydropyrido[2,3-b]pyrazine-7-carboxylic acid (15 mg, 0.031 mmol) obtained in Example 62, HATU (18 mg, 0.047 mmol) and N1,N1-dimethylethane-1,2-diamine (8 mg, 0.094 mmol) were dissolved in DCM (1 mL), and DIPEA (12 mg, 0.094 mmol) was added thereto, followed by stirring at room temperature for 3 hours. After extraction using DCM and distilled water, the organic layer was dried over anhydrous magnesium sulfate and distilled under reduced pressure. The residue was purified by MPLC to obtain the title compound (10 mg, 58%).
¹H-NMR (400 MHz, CHLOROFORM-D) δ 8.62 (d, J = 1.4 Hz, 1H), 8.07 (d, J = 1.4 Hz, 1H), 7.39 (d, J = 8.2 Hz, 2H), 7.35 (br s, 1H), 7.16 (d, J = 8.2 Hz, 2H), 5.38 (t, J = 11.7 Hz, 1H), 3.66 (s, 3H), 3.61-3.56 (m, 4H), 3.04-2.99 (m, 4H), 2.65-2.52 (m, 2H), 2.35 (s, 6H), 2.19 (t, J = 11.9 Hz, 2H), 1.60 (d, J = 8.7 Hz, 2H)
LC/MS: 549.3 (M+H), 550.3 (M+2H)

### Example 64: Preparation of N,N,1-trimethyl-2,3-dioxo-4-(1-(4-(trifluoromethoxy)benzyl)piperidin-4-yl)-1,2,3,4-tetrahydropyrido[2,3-b]pyrazine-7-carboxamide

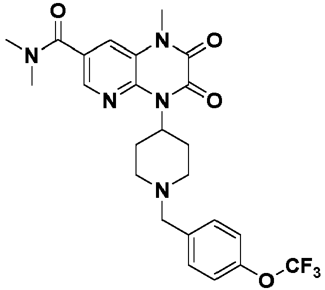

1-Methyl-2,3-dioxo-4-(1-(4-(trifluoromethoxy)benzyl)piperidin-4-yl)-1,2,3,4-tetrahydropyrido[2,3-b]pyrazine-7-carboxylic acid (35 mg, 0.073 mmol) obtained in Example 62 was used in a similar manner to Example 63 to obtain the title compound (32 mg, 87%).
¹H-NMR (400 MHz, CHLOROFORM-D) d 8.30 (d, J = 1.8 Hz, 1H), 7.62 (d, J = 1.8 Hz, 1H), 7.38 (d, J = 8.2 Hz, 2H), 7.16 (d, J = 7.8 Hz, 2H), 5.42-5.36 (m, 1H), 3.61 (s, 3H), 3.57 (s, 2H), 3.12 (d, J = 11.4 Hz, 6H), 3.00-2.94 (m, 4H), 2.21 (t, J = 12.1 Hz, 2H), 1.62 (d, J= 11.0 Hz, 2H)
LC/MS: 506.2 (M+H), 507.2 (M+2H)

### Example 65: Preparation of N,1-dimethyl-2,3-dioxo-4-(1-(4-(trifluoromethoxy)benzyl)piperidin-4-yl)-1,2,3,4-tetrahydropyrido[2,3-b]pyrazine-7-carboxamide

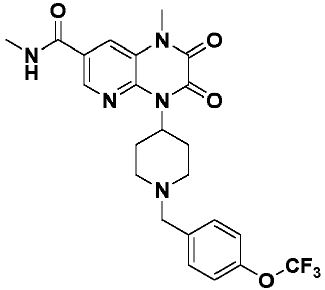

N,1-dimethyl-2,3-dioxo-4-(piperidin-4-yl)-1,2,3,4-tetrahydropyrido[2,3-b]pyrazine-7-carboxamide dihydrochloride (30 mg, 0.095 mmol) obtained in Preparation Example 29 and 4-(trifluoromethoxy)benzaldehyde (0.016 ml, 0.104 mmol) were used in a similar manner to Example 1 to obtain the title compound (19 mg, 41%).
¹H-NMR (400 MHz, CHLOROFORM-D) δ 8.51 (d, J= 1.8 Hz, 1H), 8.01 (d, J = 1.8 Hz, 1H), 7.39 (d, J = 8.7 Hz, 2H), 7.17 (d, J = 8.7 Hz, 2H), 6.23 (s, 1H), 5.39 (d, J = 12.3 Hz, 1H), 3.65 (s, 3H), 3.58 (s, 2H), 3.07 (d, J = 4.6 Hz, 3H), 3.02-2.95 (m, 4H), 2.24-2.19 (m, 2H), 1.61 (d, J = 8.2 Hz, 2H)
LC/MS: 492 (M+H)

### Example 66: Preparation of 1-methyl-2,3-dioxo-4-(1-(4-(trifluoromethoxy)benzyl)piperidin-4-yl)-1,2,3,4-tetrahydropyrido[2,3-b]pyrazine-7-carboxamide

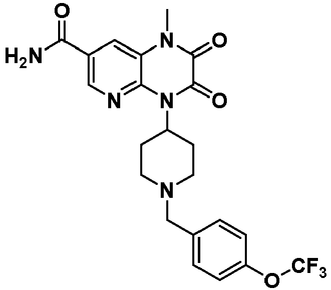

1-Methyl-2,3-dioxo-4-(piperidin-4-yl)-1,2,3,4-tetrahydropyrido[2,3-b]pyrazine-7-carboxamide dihydrochloride (30 mg, 0.099 mmol) obtained in Preparation 30 and 4-(trifluoromethoxy)benzaldehyde (0.016 ml, 0.109 mmol) were used in a similar manner to Example 1 to obtain the title compound (20 mg, 42%).
¹H-NMR (400 MHz, CHLOROFORM-D) δ 8.57 (d, J = 1.8 Hz, 1H), 8.05 (d, J = 1.8 Hz, 1H), 7.39 (d, J = 8.2 Hz, 2H), 7.17 (d, J = 8.2 Hz, 2H), 5.38 (s, 1H), 3.66 (s, 3H), 3.57 (s, 2H), 3.00 (d, J = 11.0 Hz, 4H), 2.23-2.16 (m, 2H), 1.44 (dd, J = 27.0, 6.4 Hz, 2H)
LC/MS: 478 (M+H)

### Example 67: Preparation of 4-(1-(3-chlorobenzyl)piperidin-4-yl)-1,7-dimethyl-1,4-dihydropyrido[2,3-b]pyrazine-2,3-dione

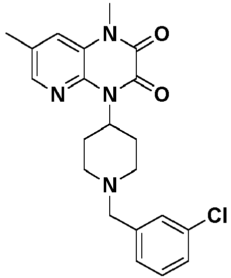

1,7-Dimethyl-4-(piperidin-4-yl)-1,4-dihydropyrido[2,3-b]pyrazine-2,3-dione dihydrochloride (50 mg, 0.14 mmol) obtained in Preparation Example 28 and 3-chlorobenzaldehyde (61 mg, 0.43 mmol) were used in a similar manner to Example 1 to obtain the title compound (43 mg, 75%).
¹H-NMR (500 MHz, CHLOROFORM-D) δ 8.11 (s, 1H), 7.41 (s, 1H), 7.31-7.24 (m, 4H), 5.40 (t, J= 11.6 Hz, 1H), 3.63 (s, 3H), 3.60 (s, 2H), 3.12-3.01 (m, 4H), 2.44 (s, 3H), 2.26 (t, J = 11.4 Hz, 2H), 1.62 (d, J = 11.3 Hz, 2H)
LC/MS: 399.2 (M+H)

### Experimental Example: Measurement of inhibitory effect against DGKα enzyme

First, 3X OAG (3 mM)/ATP (0.45 mM) substrate solution was prepared from 1X substrate assay buffer (40 mM MOPS (pH 7.2), 20 mM MgCl₂, 1 mM DTT, 0.4 mM CaCl₂, 3 mM sodium deoxycholate, 100 mMNaCl, 0.1 mg/mL BSA, 0.12% NP-40), and vortexed thoroughly for 3 minutes to induce detergent-lipid micelle formation. Then, 3X DGKα (7.5 nM) enzyme solution was prepared from 2X enzyme assay buffer (80 mM MOPS (pH 7.2), 2 mM DTT, 200 mM NaCl, 0.2 mg/mL BSA) and vortexed for a short time.

After preparing the above two solutions, a half-area opaque 96-well assay plate was prepared, and 10 µL of 3X diluted compound solution (30 µM to 0 µM) was transferred to each well. Next, 10 µL of 3X DGKα enzyme solution was transferred to the same plate, mixed by pipetting, and then 10 µL of 3X OAG/ATP substrate solution was added to the assay plate and mixed well. The plate was incubated at room temperature for 20 minutes for the enzyme reaction. Next, 15 µL of ADP-Glo reagent was added to each well and mixed by pipetting, followed by incubating the plate at room temperature for 40 minutes to deplete the remaining ATPs. After this step, 30 µL of kinase detection reagent was added and mixed, and the plate was incubated at room temperature for an additional 20 minutes and luminescence was measured by Envision to calculate the IC₅₀ value of each compound.

The measurement results are represented in Table 1 (+: IC₅₀ >5 µM, ++: 5 µM> IC₅₀ >300 nM, +++: IC₅₀ <300 nM).

**[Table 1]**

| **Example** | **IC₅₀** | **Example** | **IC₅₀** |
|---|---|---|---|
| 1 | +++ | 35 | +++ |
| 2 | +++ | 36 | +++ |
| 3 | ++ | 37 | +++ |
| 4 | + | 38 | ++ |
| 5 | +++ | 39 | +++ |
| 6 | ++ | 40 | + |
| 7 | + | 41 | +++ |
| 8 | ++ | 42 | +++ |
| 9 | ++ | 43 | +++ |
| 10 | ++ | 44 | ++ |
| 11 | ++ | 45 | ++ |
| 12 | ++ | 46 | ++ |
| 13 | +++ | 47 | ++ |
| 14 | ++ | 48 | +++ |
| 15 | + | 49 | ++ |
| 16 | + | 50 | +++ |
| 17 | ++ | 51 | +++ |
| 18 | +++ | 52 | + |
| 19 | ++ | 53 | ++ |
| 20 | ++ | 54 | +++ |
| 21 | ++ | 55 | ++ |
| 22 | +++ | 56 | + |
| 23 | ++ | 57 | ++ |
| 24 | + | 58 | + |
| 25 | ++ | 59 | ++ |
| 26 | ++ | 60 | +++ |
| 27 | +++ | 61 | +++ |
| 28 | +++ | 62 | ++ |
| 29 | +++ | 63 | + |
| 30 | +++ | 64 | + |
| 31 | ++ | 65 | ++ |
| 32 | +++ | 66 | +++ |
| 33 | ++ | 67 | +++ |
| 34 | +++ | | |

## Claims

1. A compound of the following Formula 1, or a pharmaceutically acceptable salt or stereoisomer thereof: 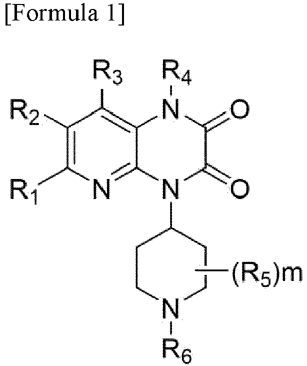 wherein
m represents an integer of 0, 1 or 2;
R₁ represents hydrogen, halo, cyano (-CN), alkyl, alkoxy, alkylcarbonyl or aryl;
R₂ represents hydrogen, halo, cyano, carboxy (-COOH), alkyl, alkylcarbonyl, alkoxycarbonyl, aminocarbonyl, alkylaminocarbonyl, dialkylaminocarbonyl, dialkylaminoalkylaminocarbonyl, cycloalkyl, aryl or heteroaryl;
R₃ represents hydrogen or alkyl;
R₄ represents alkyl;
R₅ represents alkyl, or may combine with each other to form a ring when m is 2;
R₆ represents 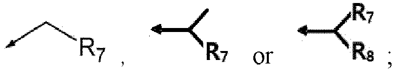 wherein R₇ and R₈ independently of one another represent carbocyclyl or heterocyclyl;
wherein the heteroaryl and heterocyclyl have one or more heteroatoms selected from nitrogen (N), oxygen (O) and sulfur (S); and
the aryl, heteroaryl, carbocyclyl and heterocyclyl may be optionally substituted with one or more substituents selected from the group consisting of halo, alkyl, alkoxy, haloalkyl, haloalkoxy, aryl and aryloxy.

2. The compound, or a pharmaceutically acceptable salt or stereoisomer thereof according to Claim 1, wherein
m represents an integer of 0, 1 or 2;
R₁ represents hydrogen, halo, cyano, C₁-C₇ alkyl, C₁-C₇ alkoxy, C₁-C₇ alkylcarbonyl or C₆-C₁₀ aryl;
R₂ represents hydrogen, halo, cyano, carboxy, C₁-C₇ alkyl, C₁-C₇ alkylcarbonyl, C₁-C₇ alkoxycarbonyl, aminocarbonyl, C₁-C₇ alkylaminocarbonyl, di(C₁-C₇ alkyl)aminocarbonyl, di(C₁-C₇ alkyl)amino-C₁-C₇ alkylaminocarbonyl, C₃-C₇ cycloalkyl, C₆-C₁₀ aryl, or 5- to 10-membered heteroaryl having 1 to 3 heteroatoms selected from N and O;
R₃ represents hydrogen or C₁-C₇ alkyl;
R₄ represents C₁-C₇ alkyl;
R₅ represents C₁-C₇ alkyl, or may combine with each other to form a C₂-C₄ ring when m is 2;
R₆ represents 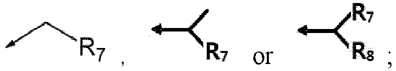 wherein R₇ and R₈ independently of one another represent C₅-C₁₀ carbocyclyl, or 5- to 12-membered heterocyclyl having 1 to 3 heteroatoms selected from N, O and S;
wherein the aryl, heteroaryl, carbocyclyl and heterocyclyl may be unsubstituted or substituted with 1 to 3 substituents selected from the group consisting of halo, C₁-C₇ alkyl, C₁-C₇ alkoxy, halo-C₁-C₇ alkyl, halo-C₁-C₇ alkoxy, C₆-C₁₀ aryl and C₆-C₁₀ aryloxy.

3. The compound, or a pharmaceutically acceptable salt or stereoisomer thereof according to Claim 1, wherein the compound of Formula 1 is selected from the following group:
methyl-4-(1-(4-(trifluoromethoxy)benzyl)piperidin-4-yl)-1,4-dihydropyrido[2,3-b]pyrazine-2,3-dione ;
1,6-dimethyl-4-(1-(4-(trifluoromethoxy)benzyl)piperidin-4-yl)-1,4-dihydropyrido[2,3-b]pyrazine-2,3-dione;
ethyl-4-(1-(4-(trifluoromethoxy)benzyl)piperidin-4-yl)-1,4-dihydropyrido[2,3-b]pyrazine-2,3-dione;
isobutyl-4-(1 -(4-(trifluoromethoxy)benzyl)piperidin-4-yl)-1,4-dihydropyrido[2,3-b]pyrazine-2,3-dione;
1,6-dimethyl-4-(1-(naphthalen-1-ylmethyl)piperidin-4-yl)-1,4-dihydropyrido[2,3-b]pyrazine-2,3-dione;
4-(1-((2-chloroquinolin-3-yl)methyl)piperidin-4-yl)-1,6-dimethyl-1,4-dihydropyrido[2,3-b]pyrazine -2,3-dione;
4-(1-((1,2-dimethyl-1H-indol-3-yl)methyl)piperidin-4-yl)-1,6-dimethyl-1,4-dihydropyrido[2,3-b]pyrazine-2,3-dione;
1,6-dimethyl-4-(1-((1-methyl-1H-indol-5-yl)methyl)piperidin-4-yl)-1,4-dihydropyrido[2,3-b]pyrazine-2,3-dione;
1,6-dimethyl-4-(1-(quinolin-4-ylmethyl)piperidin-4-yl)-1,4-dihydropyrido[2,3-b]pyrazine-2,3-dione;
4-(1-([1,1'-biphenyl]-2-ylmethyl)piperidin-4-yl)-1,6-dimethyl-1,4-dihydropyrido[2,3-b]pyrazine-2,3-dione;
1-methyl-2,3-dioxo-4-(1-(4-(trifluoromethoxy)benzyl)piperidin-4-yl)-1,2,3,4-tetrahydropyrido[2,3-b]pyrazine-6-carbonitrile;
4-(1-(2-chlorobenzyl)piperidin-4-yl)-1,6-dimethyl-1,4-dihydropyrido[2,3-b]pyrazine-2, 3-dione;
4-(1-(3-chlorobenzyl)piperidin-4-yl)-1,6-dimethyl-1,4-dihydropyrido[2,3-b]pyrazine-2,3 -dione;
4-(1-(4-chlorobenzyl)piperidin-4-yl)-1,6-dimethyl-1,4-dihydropyrido[2,3-b]pyrazine-2,3 -dione;
4-(1-((2-methoxypyridin-3-yl)methyl)piperidin-4-yl)-1,6-dimethyl-1,4-dihydropyrido[2,3-b]pyrazine-2,3-dione;
4-(1-((6-fluoropyridin-2-yl)methyl)piperidin-4-yl)-1,6-dimethyl-1,4-dihydropyrido[2,3-b]pyrazine-2,3-dione;
4-(1-((6-fluoropyridin-3-yl)methyl)piperidin-4-yl)-1,6-dimethyl-1,4-dihydropyrido[2,3-b]pyrazine-2,3-dione;
1,6-dimethyl-4-(1-(4-phenoxybenzyl)piperidin-4-yl)-1,4-dihydropyrido[2,3-b]pyrazine-2,3 -dione;
1,6-dimethyl-4-(1-(naphthalen-2-ylmethyl)piperidin-4-yl)-1,4-dihydropyrido[2,3-b]pyrazine-2,3-dione;
4-(1-(isoquinolin-5-ylmethyl)piperidin-4-yl)-1,6-dimethyl-1,4-dihydropyrido[2,3-b]pyrazine-2,3-dione;
1,6-dimethyl-4-(1-(3-phenoxybenzyl)piperidin-4-yl)-1,4-dihydropyrido[2,3-b]pyrazine-2,3 -dione;
4-(1-(3-chlorobenzyl)piperidin-4-yl)-1-methyl-1,4-dihydropyrido[2,3-b]pyrazine-2,3 -dione;
4-(1-benzylpiperidin-4-yl)-1, 6-dimethyl-1,4-dihydropyri do[2,3-b]pyrazine-2,3-dione;
1,6-dimethyl-4-(1-(1-(quinoxalin-6-yl)ethyl)piperidin-4-yl)-1,4-dihydropyrido[2,3-b]pyrazine-2,3-dione;
4-(1-(3-chlorobenzyl)piperidin-4-yl)-1-methyl-2,3-dioxo-1,2,3,4-tetrahydropyrido[2,3-b]pyrazine-6-carbonitrile;
1-methyl-4-(1-(naphthalen-1-ylmethyl)piperidin-4-yl)-2,3-dioxo-1,2,3,4-tetrahydropyrido[2,3-b]pyrazine-6-carbonitrile;
7-chloro-4-(1-(3-chlorobenzyl)piperidin-4-yl)-1-methyl-1,4-dihydropyrido[2,3-b]pyrazine-2,3 -dione;
7-chloro-1-methyl-4-(1-(4-(trifluoromethoxy)benzyl)piperidin-4-yl)-1,4-dihydropyrido[2,3-b]pyrazine-2,3-dione;
7-chloro-1-methyl-4-(1-(naphthalen-1-ylmethyl)piperidin-4-yl)-1,4-dihydropyrido[2,3-b]pyrazine-2,3-dione;
7-chloro-1-methyl-4-(1-(3-(trifluoromethyl)benzyl)piperidin-4-yl)-1,4-dihydropyrido[2,3-b]pyrazine-2,3-dione;
7-chloro-4-(3,3-dimethyl-1-(4-(trifluoromethoxy)benzyl)piperidin-4-yl)-1-methyl-1,4-dihydropyrido[2,3-b]pyrazine-2,3-dione;
7-chloro-1-methyl-4-((1R,3s,5S)-8-(4-(trifluoromethoxy)benzyl)-8-azabicyclo[3.2.1]octan-3-yl)-1,4-dihydropyrido[2,3-b]pyrazine-2,3-dione;
7-chloro-1-methyl-4-(1-(quinoxalin-5-ylmethyl)piperidin-4-yl)-1,4-dihydropyrido[2,3-b]pyrazine-2,3-dione;
7-bromo-1-methyl-4-(1-(4-(trifluoromethoxy)benzyl)piperidin-4-yl)-1,4-dihydropyrido[2,3-b]pyrazine-2,3-dione;
7-chloro-4-(1-((5-chloronaphthalen-1-yl)methyl)piperidin-4-yl)-1-methyl-1,4-dihydropyrido[2,3-b]pyrazine-2,3-dione;
1-methyl-7-(1-methyl-1H-pyrazol-4-yl)-4-(1-(4-(trifluoromethoxy)benzyl)piperidin-4-yl)-1,4-dihydropyrido[2,3-b]pyrazine-2,3-dione;
7-chloro-1-methyl-4-(1-((5,6,7,8-tetrahydronaphthalen-1-yl)methyl)piperidin-4-yl)-1,4-dihydropyrido[2,3-b]pyrazine-2,3-dione;
7-bromo-1-methyl-2,3-dioxo-4-(1-(4-(trifluoromethoxy)benzyl)piperidin-4-yl)-1,2,3,4-tetra hydropyrido[2,3-b]pyrazine-6-carbonitrile;
7-(2-fluorophenyl)-1-methyl-4-(1-(4-(trifluoromethoxy)benzyl)piperidin-4-yl)-1,4-dihydropyrido[2,3-b]pyrazine-2,3-dione;
7-cyclopropyl-1-methyl-4-(1-(4-(trifluoromethoxy)benzyl)piperi din-4-yl)-1,4-dihydropyrido[2,3-b]pyrazine-2,3-dione;
7-chloro-6-(2-fluorophenyl)-1-methyl-4-(1-(4-(trifluoromethoxy)benzyl)piperidin-4-yl)-1,4-dihydropyrido[2,3-b]pyrazine-2,3-dione;
7-chloro-4-(1-(3-chloro-4-fluorobenzyl)piperidin-4-yl)-1-methyl-1,4-dihydropyrido[2,3-b]pyrazine-2,3-dione;
7-chloro-4-(1-(3-chloro-4-(trifluoromethoxy)benzyl)piperidin-4-yl)-1-methyl-1,4-dihydropyrido[2,3-b]pyrazine-2,3-dione;
7-cyclopropyl-1-methyl-2,3-dioxo-4-(1-(4-(trifluoromethoxy)benzyl)piperidin-4-yl)-1,2,3,4-tetrahydropyrido[2,3-b]pyrazine-6-carbonitrile;
4-(1-(bis(3-chlorophenyl)methyl)piperidin-4-yl)-7-chloro-1-methyl-1,4-dihydropyrido[2,3-b]pyrazine-2,3-dione;
7-chloro-4-(1-((2,3-dihydrobenzo[b][1,4]dioxin-6-yl)methyl)piperidin-4-yl)-1-methyl-1,4-dihydropyrido[2,3-b]pyrazine-2,3-dione;
1-methyl-2,3-dioxo-4-(1-(4-(trifluoromethoxy)benzyl)piperidin-4-yl)-1,2,3,4-tetrahydropyrido[2,3-b]pyrazine-7-carbonitrile;
7-acetyl-1-methyl-4-(1-(4-(trifluoromethoxy)benzyl)piperidin-4-yl)-1,4-dihydropyrido[2,3-b]pyrazine-2,3-dione;
7-chloro-4-((2R,5S)-2,5-dimethyl-1-(4-trifluoromethoxy)benzyl)piperidin-4-yl)-1-methyl-1,4-dihydropyrido[2,3 -b]pyrazine-2,3-dione;
7-chloro-1,6-dimethyl-4-(1-(4-(trifluoromethoxy)benzyl)piperidin-4-yl)-1,4-dihydropyrido[2,3-b]pyrazine-2,3-dione;
7-bromo-1,6-dimethyl-4-(1-(4-(trifluoromethoxy)benzyl)piperidin-4-yl)-1,4-dihydropyrido[2,3-b]pyrazine-2,3-dione;
7-chloro-4-(1-((1-isopropyl-1H-pyrazol-4-yl)methyl)piperidin-4-yl)-1-methyl-1,4-dihydropyrido[2,3-b]pyrazine-2,3-dione;
6-acetyl-1-methyl-4-(1-(4-(trifluoromethoxy)benzyl)piperidin-4-yl)-1,4-dihydropyrido[2,3-b]pyrazine-2,3-dione;
6-chloro-1-methyl-4-(1-(4-(trifluoromethoxy)benzyl)piperidin-4-yl)-1,4-dihydropyrido[2,3-b]pyrazine-2,3-dione;
6-methoxy-1-methyl-4-(1-(4-(trifluoromethoxy)benzyl)piperidin-4-yl)-1,4-dihydropyrido[2,3-b]pyrazine-2,3-dione;
1,8-dimethyl-4-(1-(4-(trifluoromethoxy)benzyl)piperidin-4-yl)-1,4-dihydropyrido[2,3-b]pyrazine-2,3-dione;
7-chloro-1-methyl-4-((1R,3r,5S)-8-(4-(trifluoromethoxy)benzyl)-8-azabicyclo[3.2.1]octan-3-yl)-1,4-dihydropyrido[2,3-b]pyrazine-2,3-dione;
6-isopropoxy-1-methyl-4-(1-(4-(trifluoromethoxy)benzyl)piperidin-4-yl)-1,4-dihydropyrido[2,3-b]pyrazine-2,3-dione;
7-fluoro-1-methyl-4-(1-(4-(trifluoromethoxy)benzyl)piperidin-4-yl)-1,4-dihydropyrido[2,3-b]pyrazine -2,3-dione;
methyl 1-methyl-2,3-dioxo-4-(1-(4-(trifluoromethoxy)benzyl)piperidin-4-yl)-1,2,3,4-tetrahydropyrido[2,3-b]pyrazine-7-carboxylate;
1,7-dimethyl-4-(1-(4-(trifluoromethoxy)benzyl)piperidin-4-yl)-1,4-dihydropyrido[2,3-b]pyrazine-2,3-dione;
1-methyl-2,3-dioxo-4-(1-(4-(trifluoromethoxy)benzyl)piperidin-4-yl)-1,2,3,4-tetrahydropyrido[2,3-b]pyrazine-7-carboxylic acid;
N-(2-(dimethylamino)ethyl)-1-methyl-2,3-dioxo-4-(1-(4-(trifluoromethoxy)benzyl)piperidin-4-yl)-1,2,3,4-tetrahydropyrido[2,3-b]pyrazine-7-carboxamide;
N,N,1-trimethyl-2,3-dioxo-4-(1-(4-(trifluoromethoxy)benzyl)piperidin-4-yl)-1,2,3,4-tetrahydropyrido[2,3-b]pyrazine-7-carboxamide;
N,1-dimethyl-2,3-dioxo-4-(1-(4-(trifluoromethoxy)benzyl)piperidin-4-yl)-1,2,3,4-tetrahydropyrido[2,3-b]pyrazine-7-carboxamide;
1-methyl-2,3-dioxo-4-(1-(4-(trifluoromethoxy)benzyl)piperidin-4-yl)-1,2,3,4-tetrahydropyrido[2,3-b]pyrazine-7-carboxamide; and
4-(1-(3-chlorobenzyl)piperidin-4-yl)-1,7-dimethyl-1,4-dihydropyrido[2,3-b]pyrazine-2,3-dione.

4. A pharmaceutical composition for the prevention or treatment of diseases associated with diacylglycerol kinases (DGKs) comprising the compound of Formula 1, or a pharmaceutically acceptable salt or stereoisomer thereof as defined in any one of Claims 1 to 3 as an active ingredient, together with a pharmaceutically acceptable carrier.

5. The pharmaceutical composition according to Claim 4, wherein the disease associated with diacylglycerol kinases (DGKs) is cancer.

6. The pharmaceutical composition according to Claim 5, wherein the cancer is selected from the group consisting of gastrointestinal cancer, pancreatic cancer, breast cancer, colon cancer, retinoblastoma, liver cancer, lung cancer, ovarian cancer, cervical cancer, endometrial cancer, brain tumor, testicular cancer, laryngeal cancer, prostate cancer, neuroblastoma, kidney cancer, thyroid cancer, esophageal cancer, skin cancer, osteosarcoma and bladder cancer.
